# EUROPEAN PATENT SPECIFICATION

(11) **EP 2 111 452 B1**
(45) Date of publication and mention of the grant of the patent: **11.04.2012**
(21) Application number: 08708671.6
(22) Date of filing: 05.02.2008
(51) Int. Cl.: C12N 15/11, C12N 15/82, A01H 5/00

(54) **COMPOSITIONS AND METHODS USING RNA INTERFERENCE OF OPR3-LIKE GENE FOR CONTROL OF NEMATODES**
ZUSAMMENSETZUNGEN UND VERFAHREN MIT RNA-INTERFERENZ OPR3-ÄHNLICHEM GEN ZUR KONTROLLE VON NEMATODEN
COMPOSITIONS ET MÉTHODES UTILISANT L'ARN INTERFÉRENCE D'UN GÈNE DU TYPE OPR3 POUR LA LUTTE CONTRE LES NÉMATODES

(30) Priority: 08.02.2007 US 900146 P
(43) Date of publication of application: 28.10.2009
(73) Proprietor: BASF Plant Science GmbH, 67056 Ludwigshafen (DE)
(72) Inventor: WIIG, Aaron, Durham, NC 27713 (US)
(74) Representative: Oswald, Oliver
(86) International application number: PCT/EP2008/051370
(87) International publication number: WO 2008/095910

(56) References cited:
- WO-A-03/048319
- WO-A-2004/005485
- US-A1- 2004 031 072

## Description

The invention relates to the control of nematodes. Disclosed herein are methods of producing transgenic plants with increased nematode resistance, expression vectors comprising polynucleotide conferring nematode resistance, and transgenic plants and seeds generated thereof.

Nematodes are microscopic roundworms that feed on the roots, leaves and stems of more than 2,000 row crops, vegetables, fruits, and ornamental plants, causing an estimated $100 billion crop loss worldwide. A variety of parasitic nematode species infect crop plants, including root-knot nematodes (RKN), cyst- and lesion-forming nematodes. Root-knot nematodes, which are characterized by causing root gall formation at feeding sites, have a relatively broad host range and are therefore pathogenic on a large number of crop species. The cyst- and lesion-forming nematode species have a more limited host range, but still cause considerable losses in susceptible crops.

Pathogenic nematodes are present throughout the United States, with the greatest concentrations occurring in the warm, humid regions of the South and West and in sandy soils. Soybean cyst nematode (Heterodera glycines), the most serious pest of soybean plants, was first discovered in the United States in North Carolina in 1954. Some areas are so heavily infested by soybean cyst nematode (SCN) that soybean production is no longer economically possible without control measures. Although soybean is the major economic crop attacked by SCN, SCN parasitizes some fifty hosts in total, including field crops, vegetables, ornamentals, and weeds.

Signs of nematode damage include stunting and yellowing of leaves, and wilting of the plants during hot periods. However, nematode infestation can cause significant yield losses without any obvious above-ground disease symptoms. The primary causes of yield reduction are due to root damage underground. Roots infected by SCN are dwarfed or stunted. Nematode infestation also can decrease the number of nitrogen-fixing nodules on the roots, and may make the roots more susceptible to attacks by other soil-bome plant pathogens.

The nematode life cycle has three major stages: egg, juvenile, and adult. The life cycle varies between species of nematodes. For example, the SCN life cycle can usually be completed in 24 to 30 days under optimum conditions whereas other species can take as long as a year, or longer, to complete the life cycle. When temperature and moisture levels become favorable in the spring, worm-shaped juveniles hatch from eggs in the soil. Only nematodes in the juvenile developmental stage are capable of infecting soybean roots.

The life cycle of SCN has been the subject of many studies, and as such are a useful example for understanding the nematode life cycle. After penetrating soybean roots, SCN juveniles move through the root until they contact vascular tissue, at which time they stop migrating and begin to feed. With a stylet, the nematode injects secretions that modify certain root cells and transform them into specialized feeding sites. The root cells are morphologically transformed into large multinucleate syncytia (or giant cells in the case of RKN), which are used as a source of nutrients for the nematodes. The actively feeding nematodes thus steal essential nutrients from the plant resulting in yield loss. As female nematodes feed, they swell and eventually become so large that their bodies break through the root tissue and are exposed on the surface of the root.

After a period of feeding, male SCN nematodes, which are not swollen as adults, migrate out of the root into the soil and fertilize the enlarged adult females. The males then die, while the females remain attached to the root system and continue to feed. The eggs in the swollen females begin developing, initially in a mass or egg sac outside the body, and then later within the nematode body cavity. Eventually the entire adult female body cavity is filled with eggs, and the nematode dies. It is the egg-filled body of the dead female that is referred to as the cyst. Cysts eventually dislodge and are found free in the soil. The walls of the cyst become very tough, providing excellent protection for the approximately 200 to 400 eggs contained within. SCN eggs survive within the cyst until proper hatching conditions occur. Although many of the eggs may hatch within the first year, many also will survive within the protective cysts for several years.

A nematode can move through the soil only a few inches per year on its own power. However, nematode infestation can be spread substantial distances in a variety of ways. Anything that can move infested soil is capable of spreading the infestation, including farm machinery, vehicles and tools, wind, water, animals, and farm workers. Seed sized particles of soil often contaminate harvested seed. Consequently, nematode infestation can be spread when contaminated seed from infested fields is planted in non-infested fields. There is even evidence that certain nematode species can be spread by birds. Only some of these causes can be prevented.

Traditional practices for managing nematode infestation include: maintaining proper soil nutrients and soil pH levels in nematode-infested land; controlling other plant diseases, as well as insect and weed pests; using sanitation practices such as plowing, planting, and cultivating of nematode-infested fields only after working non-infested fields; cleaning equipment thoroughly with high pressure water or steam after working in infested fields; not using seed grown on infested land for planting non-infested fields unless the seed has been properly cleaned; rotating infested fields and alternating host crops with non-host crops; using nematicides; and planting resistant plant varieties.

Methods have been proposed for the genetic transformation of plants in order to confer increased resistance to plant parasitic nematodes. U.S. Patent Nos. 5,589,622 and 5,824,876 are directed to the identification of plant genes expressed specifically in or adjacent to the feeding site of the plant after attachment by the nematode. The promoters of these plant target genes can then be used to direct the specific expression of detrimental proteins or enzymes, or the expression of antisense RNA to the target gene or to general cellular genes. The plant promoters may also be used to confer nematode resistance specifically at the feeding site by transforming the plant with a construct comprising the promoter of the plant target gene linked to a gene whose product induces lethality in the nematode after ingestion.

Recently, RNA interference (RNAi), also referred to as gene silencing, has been proposed as a method for controlling nematodes. When double-stranded RNA (dsRNA) corresponding essentially to the sequence of a target gene or mRNA is introduced into a cell, expression from the target gene is inhibited (See e.g., U.S. Patent No. 6,506,559). U.S. Patent No. 6,506,559 demonstrates the effectiveness of RNAi against known genes in Caenorhabditis elegans, but does not demonstrate the usefulness of RNAi for controlling plant parasitic nematodes.

Use of RNAi to target essential nematode genes has been proposed, for example, in WO 01/96584, WO 01/37654, US 2004/0098761, US 2005/0091713, US 2005/0188438, US 2006/0037101, US 2006/0080749, US 2007/0199100, and US 2007/0250947.

A number of models have been proposed for the action of RNAi. In mammalian systems, dsRNAs larger than 30 nucleotides trigger induction of interferon synthesis and a global shut-down of protein syntheses, in a non-sequence-specific manner. However, U.S. Patent No. 6,506,559 discloses that in nematodes, the length of the dsRNA corresponding to the target gene sequence may be at least 25, 50, 100, 200, 300, or 400 bases, and that even larger dsRNAs were also effective at inducing RNAi in C. elegans. It is known that when hairpin RNA constructs comprising double stranded regions ranging from 98 to 854 nucleotides were transformed into a number of plant species, the target plant genes were efficiently silenced. There is general agreement that in many organisms, including nematodes and plants, large pieces of dsRNA are cleaved into about 19-24 nucleotide fragments (siRNA) within cells, and that these siRNAs are the actual mediators of the RNAi phenomenon.

The OPR3 enzyme (12-oxyphytodienoate reductase) is involved in jasmonic acid (JA) biosynthesis. The OPR3 enzyme converts 12-oxo-cis-10,15-phytodienoate (OPDA) to 3-oxo-2-cis(cis-2-pentenyl)-cyclopentane-1-octanoate, which undergoes 3 rounds of beta oxidation to generate (+)-7-isojasmonate (JA). Arabidopsis opr3 mutant plants are unable to accumulate JA and are male sterile (Stintzi and Browse, 2000, PNAS. 97:10625-10630). Treating Arabidopsis opr3 plants with exogenous OPDA up-regulated several genes and disclosed two distinct signal pathways, one through COI1 and one through an electrophile effect of the cyclopentones (Stintzi et al., 2001, PNAS 98:12317-12319). OPDA in concert with JA finetunes the expression of defense genes. Resistance to certain insects and fungi can occur in the absence of JA (Stintzi et al., 2001, PNAS 98:12837-42).

Although there have been numerous efforts to use RNAi to control plant parasitic nematodes, to date no transgenic nematode-resistant plant has been deregulated in any country. Accordingly, there continues to be a need to identify safe and effective compositions and methods for the controlling plant parasitic nematodes using RNAi, and for the production of plants having increased resistance to plant parasitic nematodes.

The present inventors have discovered that the soybean target gene, 12-oxyphytodienate reductase-like (OPR3-like) also designated as 45174942 (SEQ ID NO: 1), is up-regulated in SCN-induced syncytia compared to uninfected root tissue. The present inventors have demonstrated that inhibition of OPR3 levels using RNAi affects the ability of the plant to resist nematode infestation.
The present invention provides the embodiments characterized in any one of claims 1 to 11.

The present invention may be understood more readily by reference to the following detailed description of the preferred embodiments of the invention and the examples included herein. Unless otherwise noted, the terms used herein are to be understood according to conventional usage by those of ordinary skill in the relevant art. In addition to the definitions of terms provided below, definitions of common terms in molecular biology may also be found in Rieger et al., 1991 Glossary of genetics: classical and molecular, 5th Ed., Berlin: Springer-Verlag; and in Current Protocols in Molecular Biology, F.M. Ausubel et al., Eds., Current Protocols, a joint venture between Greene Publishing Associates, Inc. and John Wiley & Sons, Inc., (1998 Supplement). It is to be understood that as used in the specification and in the claims, "a" or "an" can mean one or more, depending upon the context in which it is used. Thus, for example, reference to "a cell" can mean that at least one cell can be utilized. It is to be understood that the terminology used herein is for the purpose of describing specific embodiments only and is not intended to be limiting.

Throughout this application, various patent and literature publications are referenced. The disclosures of all of these publications and those references cited within those publications in their entireties are hereby incorporated by reference into this application in order to more fully describe the state of the art to which this invention pertains.

A plant "OPR3-like gene" is defined herein as a gene having at least 60% sequence identity to a the 45174942 polynucleotide having the sequence as set forth in SEQ ID NO:1, which is the G. max OPR3-like gene. OPR3-like genes include genes having sequences such as those set forth in SEQ ID NOs:2, 7, 9, 11, 13, 15, 17, 19, 21, 23, 25, 27, and 29, which are homologs of the G. max OPR3-like gene of SEQ ID NO:1. The OPR3-like genes defined herein encode polypeptides having at least 60% sequence identity to the G. max OPR3-like polypeptide having the sequence as set forth in SEQ ID NO:30. Such polypeptides include OPR3-like polypeptides having sequences as set forth in SEQ ID NOs: 8, 10, 12, 14, 16, 18, 20, 22, 24, 26, and 28

Additional OPR3-like genes (OPR3-like gene homologs) may be isolated from plants other than soybean using the information provided herein and techniques known to those of skill in the art of biotechnology. For example, a nucleic acid molecule from a plant that hybridizes under stringent conditions to the nucleic acid of SEQ ID NO:1 can be isolated from plant tissue cDNA libraries. Alternatively, mRNA can be isolated from plant cells (e.g., by the guanidinium-thiocyanate extraction procedure of Chirgwin et al., 1979, Biochemistry 18:5294-5299), and cDNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon the nucleotide sequence shown in SEQ ID NO:1. Additional oligonucleotide primers may be designed that are based on the sequences of the OPR3-like genes having the sequences as set forth in SEQ ID NOs: 2, 7, 9, 11, 13, 15, 17, 19, 21, 23, 25, 27, and 29. Nucleic acid molecules corresponding to the OPR3-like target genes defined herein can be amplified using cDNA or, alternatively, genomic DNA, as a template and appropriate oligonucleotide primers according to standard PCR amplification techniques. The nucleic acid molecules so amplified can be cloned into appropriate vectors and characterized by DNA sequence analysis.

As used herein, "RNAi" or "RNA interference" refers to the process of sequence-specific post-transcriptional gene silencing in plants, mediated by double-stranded RNA (dsRNA). As used herein, "dsRNA" refers to RNA that is partially or completely double stranded. Double stranded RNA is also referred to as small or short interfering RNA (siRNA), short interfering nucleic acid (siNA), short interfering RNA, micro-RNA (miRNA), and the like. In the RNAi process, dsRNA comprising a first strand that is substantially identical to a portion of a target gene, e.g. an OPR3-like gene, and a second strand that is complementary to the first strand is introduced into a plant. After introduction into the plant, the target gene-specific dsRNA is processed into relatively small fragments (siRNAs) and can subsequently become distributed throughout the plant, leading to a loss-of-function mutation having a phenotype that, over the period of a generation, may come to closely resemble the phenotype arising from a complete or partial deletion of the target gene. Alternatively, the target gene-specific dsRNA is operably associated with a regulatory element or promoter that results in expression -of the dsRNA in a tissue, temporal, spatial or inducible manner and may further be processed into relatively small fragments by a plant cell containing the RNAi processing machinery, and the loss-of-function phenotype is obtained. Also, the regulatory element or promoter may direct expression preferentially to the roots or syncytia or giant cell where the dsRNA may be expressed either constitutively in those tissues or upon induction by the feeding of the nematode or juvenile nematode, such as J2 nematodes.

As used herein, taking into consideration the substitution of uracil for thymine when comparing RNA and DNA sequences, the term "substantially identical" as applied to dsRNA means that the nucleotide sequence of one strand of the dsRNA is at least about 80%-90% identical to 20 or more contiguous nucleotides of the target gene, more preferably, at least about 90-95% identical to 20 or more contiguous nucleotides of the target gene, and most preferably at least about 95%, 96%, 97%. 98% or 99% identical or absolutely identical to 20 or more contiguous nucleotides of the target gene. 20 or more nucleotides means a portion, being at least about 20, 21, 22, 23, 24, 25, 50, 100, 200, 300, 400, 500, 1000, 1500, or 2000 consecutive bases or up to the full length of the target gene.

As used herein, "complementary" polynucleotides are those that are capable of base pairing according to the standard Watson-Crick complementarity rules. Specifically, purines will base pair with pyrimidines to form a combination of guanine paired with cytosine (G:C) and adenine paired with either thymine (A:T) in the case of DNA, or adenine paired with uracil (A:U) in the case of RNA. It is understood that two polynucleotides may hybridize to each other even if they are not completely complementary to each other, provided that each has at least one region that is substantially complementary to the other. As used herein, the term "substantially complementary" means that two nucleic acid sequences are complementary over at least 80% of their nucleotides. Preferably, the two nucleic acid sequences are complementary over at least 85%, 90%, 95%, 96%, 97%, 98%, 99% or more or all of their nucleotides. Alternatively, substantially complementary" means that two nucleic acid sequences can hybridize under high stringency conditions. As used herein, the term "substantially identical" or "corresponding to" means that two nucleic acid sequences have at least 80% sequence identity. Preferably, the two nucleic acid sequences have at least 85%, 90%, 95%, 96%, 97%, 98%, 99% or 100% of sequence identity.

Also as used herein, the terms "nucleic acid" and "polynucleotide" refer to RNA or DNA that is linear or branched, single or double stranded, or a hybrid thereof. The term also encompasses RNA/DNA hybrids. When dsRNA is produced synthetically, less common bases, such as inosine, 5-methylcytosine, 6-methyladenine, hypoxanthine and others can also be used for antisense, dsRNA, and ribozyme pairing. For example, polynucleotides that contain C-5 propyne analogues of uridine and cytidine have been shown to bind RNA with high affinity and to be potent antisense inhibitors of gene expression. Other modifications, such as modification to the phosphodiester backbone, or the 2'-hydroxy in the ribose sugar group of the RNA can also be made.

As used herein, the term "control," when used in the context of an infection, refers to the reduction or prevention of an infection. Reducing or preventing an infection by a nematode will cause a plant to have increased resistance to the nematode; however, such increased resistance does not imply that the plant necessarily has 100% resistance to infection. In preferred embodiments, the resistance to infection by a nematode in a resistant plant is greater than 10%, 20%, 30%, 40%, 50%, 60%, 70%, 80%, 90%, or 95% in comparison to a wild type plant that is not resistant to nematodes. Preferably the wild type plant is a plant of a similar, more preferably identical genotype as the plant having increased resistance to the nematode, but does not comprise a dsRNA directed to the target gene. The plant's resistance to infection by the nematode may be due to the death, sterility, arrest in development, or impaired mobility of the nematode upon exposure to the plant comprising dsRNA specific to a gene essential for development or maintenance of a functional feeding site, syncytia, or giant cell. The term "resistant to nematode infection" or "a plant having nematode resistance" as used herein refers to the ability of a plant, as compared to a wild type plant, to avoid infection by nematodes, to kill nematodes or to hamper, reduce or stop the development, growth or multiplication of nematodes. This might be achieved by an active process, e.g. by producing a substance detrimental to the nematode, or by a passive process, like having a reduced nutritional value for the nematode or not developing structures induced by the nematode feeding site like syncytia or giant cells. The level of nematode resistance of a plant can be determined in various ways, e.g. by counting the nematodes being able to establish parasitism on that plant, or measuring development times of nematodes, proportion of male and female nematodes or, for cyst nematodes, counting the number of cysts or nematode eggs produced on roots of an infected plant or plant assay system.

The term "plant" is intended to encompass plants at any stage of maturity or development, as well as any tissues or organs (plant parts) taken or derived from any such plant Unless otherwise clearly indicated by context. Plant parts include, but are not limited to, stems, roots, flowers, ovules, stamens, seeds, leaves, embryos, meristematic regions, callus tissue, anther cultures, gametophytes, sporophytes, pollen, microspores, protoplasts, hairy root cultures, and the like. The present invention also includes seeds produced by the plants of the present invention. In one embodiment, the seeds are true breeding for an increased resistance to nematode infection as compared to a wild-type variety of the plant seed. As used herein, a "plant cell" includes, but is not limited to, a protoplast, gamete producing cell, and a cell that regenerates into a whole plant. Tissue culture of various tissues of plants and regeneration of plants therefrom is well known in the art and is widely published.

As used herein, the term "transgenic" refers to any plant, plant cell, callus, plant tissue, or plant part that contains all or part of at least one recombinant polynucleotide. In many cases, all or part of the recombinant polynucleotide is stably integrated into a chromosome or stable extra-chromosomal element, so that it is passed on to successive generations. For the purposes of the invention, the term "recombinant polynucleotide" refers to a polynucleotide that has been altered, rearranged, or modified by genetic engineering. Examples include any cloned polynucleotide, or polynucleotides, that are linked or joined to heterologous sequences. The term "recombinant" does not refer to alterations of polynucleotides that result from naturally occurring events, such as spontaneous mutations, or from non-spontaneous mutagenesis followed by selective breeding.

As used herein, the term "amount sufficient to inhibit expression" refers to a concentration or amount of the dsRNA that is sufficient to reduce levels or stability of mRNA or protein produced from a target gene in a plant. As used herein, "inhibiting expression" refers to the absence or observable decrease in the level of protein and/or mRNA product from a target gene. Inhibition of target gene expression may be lethal to the parasitic nematode either directly or indirectly through modification or eradication of the feeding site, syncytia, or giant cell, or such inhibition may delay or prevent entry into a particular developmental step (e.g., metamorphosis), if access to a fully functional feeding site, syncytia, or giant cell is associated with a particular stage of the parasitic nematode's life cycle. The consequences of inhibition can be confirmed by examination of the plant root for reduction or elimination of cysts or other properties of the nematode or nematode infestation (as presented below in Example 3).

In accordance with the uses of the invention, a plant is transformed with a nucleic acid or a dsRNA, which specifically inhibits expression of OPR3-like gene in the plant that is essential for the development or maintenance of a feeding site, syncytia, or giant cell; ultimately affecting the survival, metamorphosis, or reproduction of the nematode. In one embodiment, the dsRNA is encoded by a vector that has been transformed into an ancestor of the infected plant. Preferably, the nucleic acid sequence expressing said dsRNA is under the transcriptional control of a root specific promoter or a parasitic nematode feeding cell-specific promoter or a nematode inducible promoter.

Accordingly, the dsRNA referred to in accordance with the invention comprises a first strand that is substantially identical to a portion of the OPR3-like target gene of a plant genome, and a second strand that is substantially complementary to the first strand. The target gene is selected from the group consisting of: a) a polynucleotide comprising a sequence as set forth in SEQ ID NO:1, 2, or 29; b) a polynucleotide encoding a polypeptide having a sequence as set forth in SEQ ID NO:3, or 30; and c) a polynucleotide having 70% sequence identity to a polynucleotide having a sequence as set forth in SEQ ID NO:1, 2, or 29. The length of the substantially identical double-stranded nucleotide sequences may be at least about 19, 20, 21, 22, 23, 24, 25, 50, 100, 200, 300, 400, 500, 1000, 1500, consecutive bases or up to the whole length of the OPR3-like gene. In a preferred embodiment, the length of the double-stranded nucleotide sequence is from approximately from about 19 to about 200-500 consecutive nucleotides in length. In another preferred embodiment, the dsRNA is substantially identical or is identical to bases 1 to 229 of SEQ ID NO: 2.

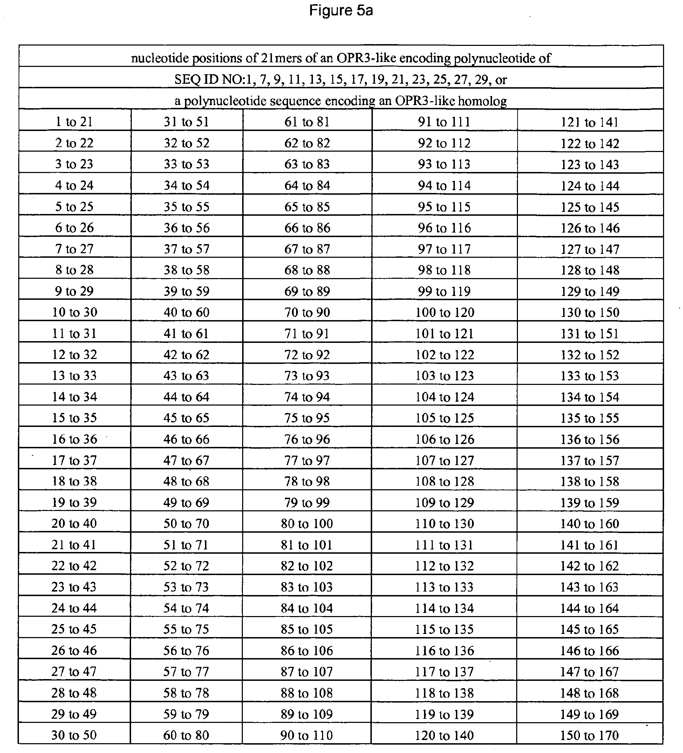

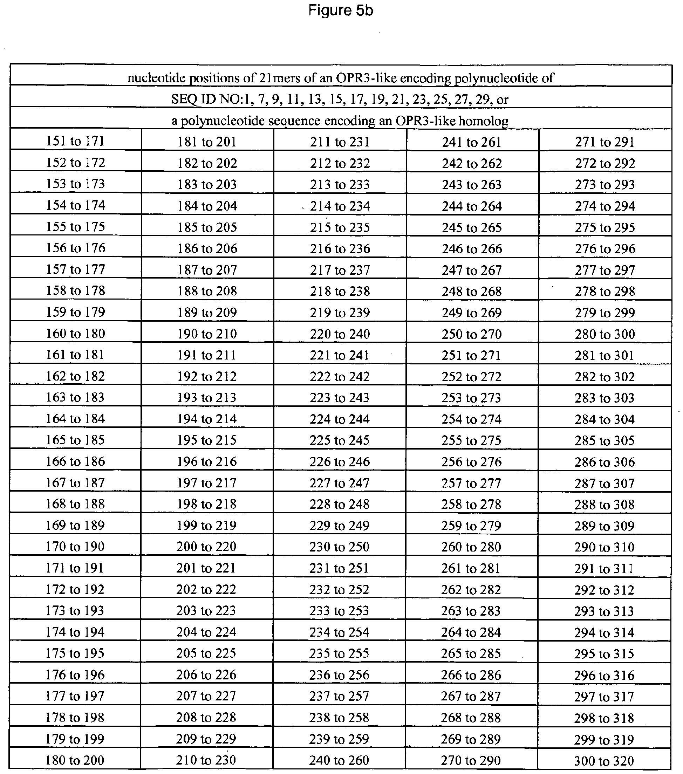

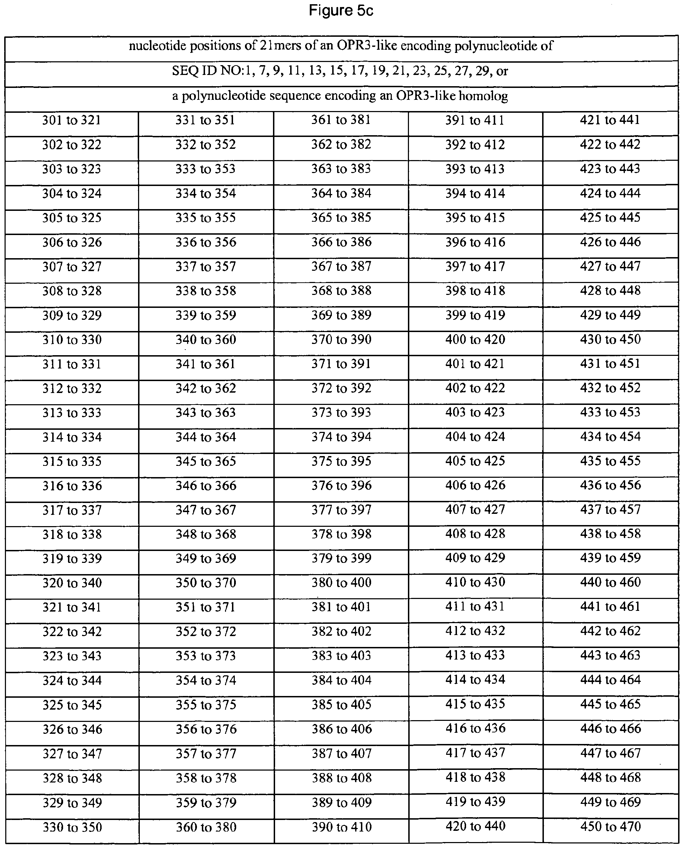

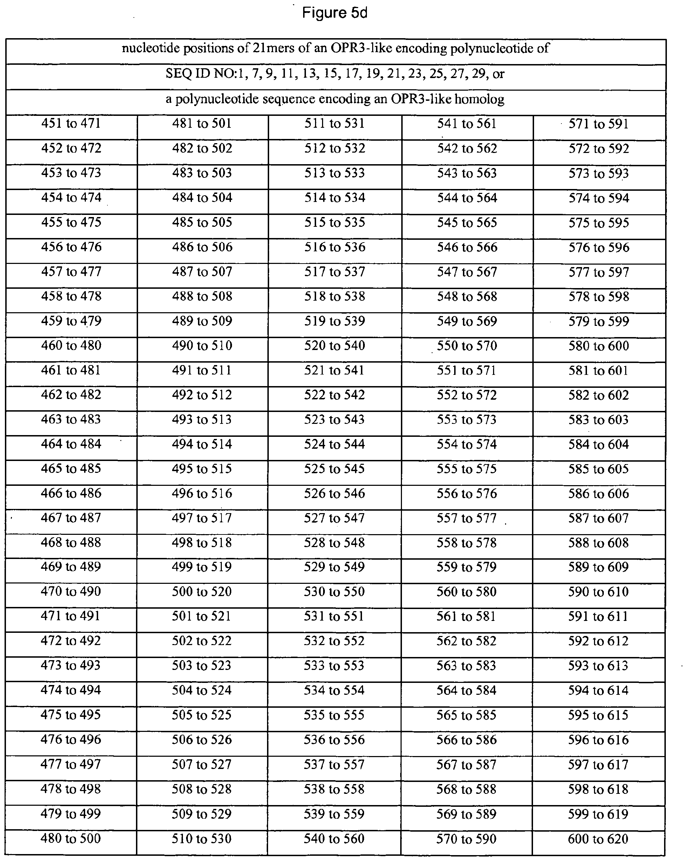

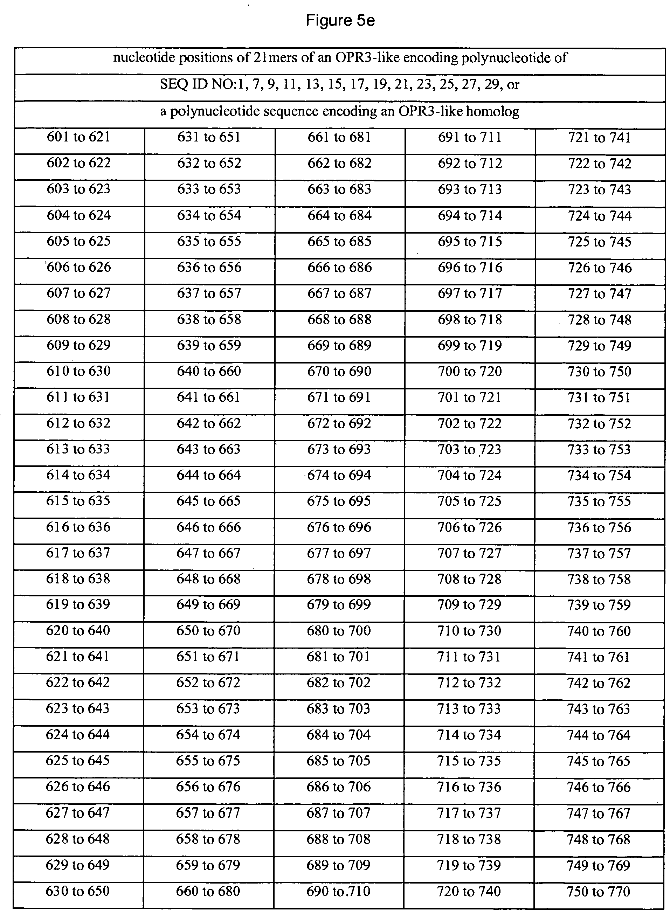

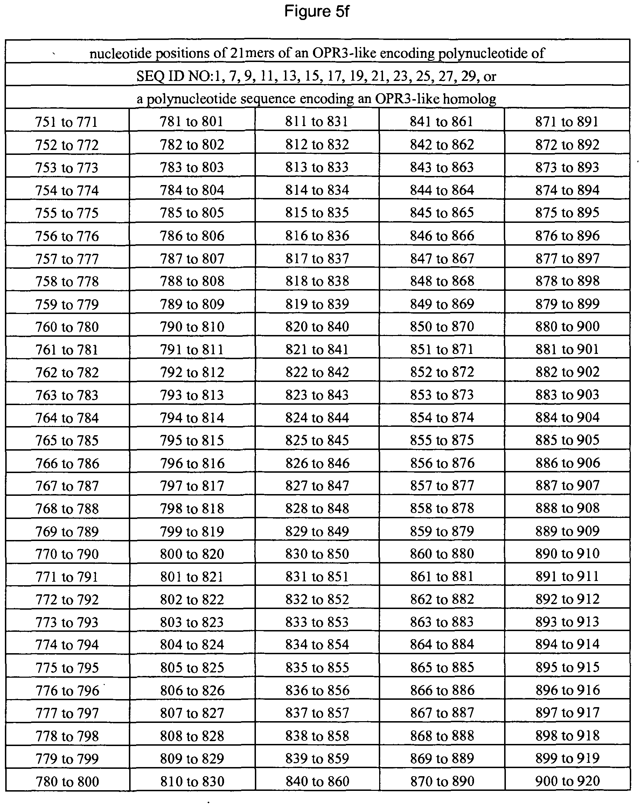

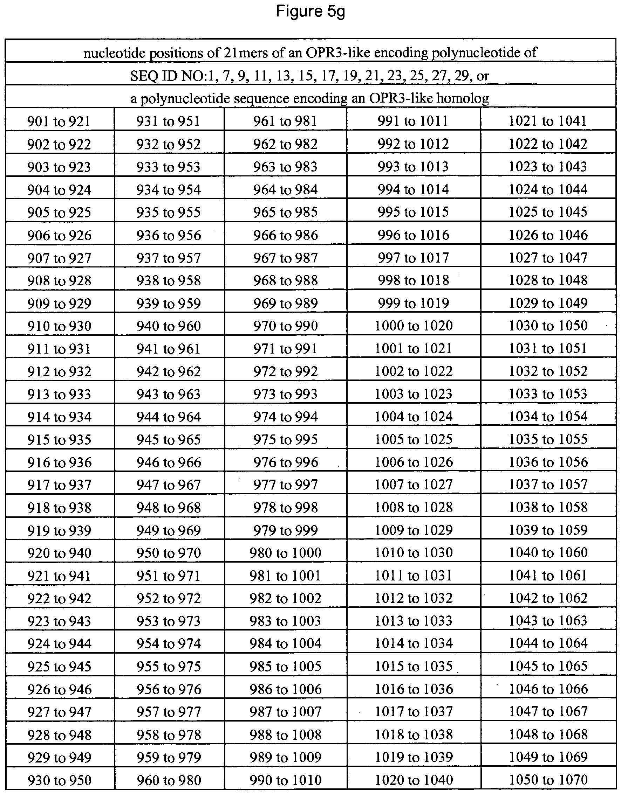

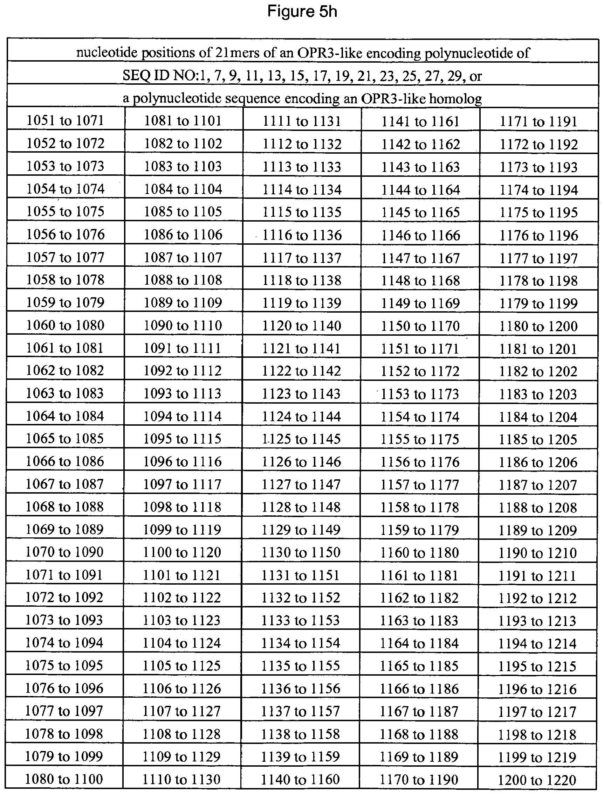

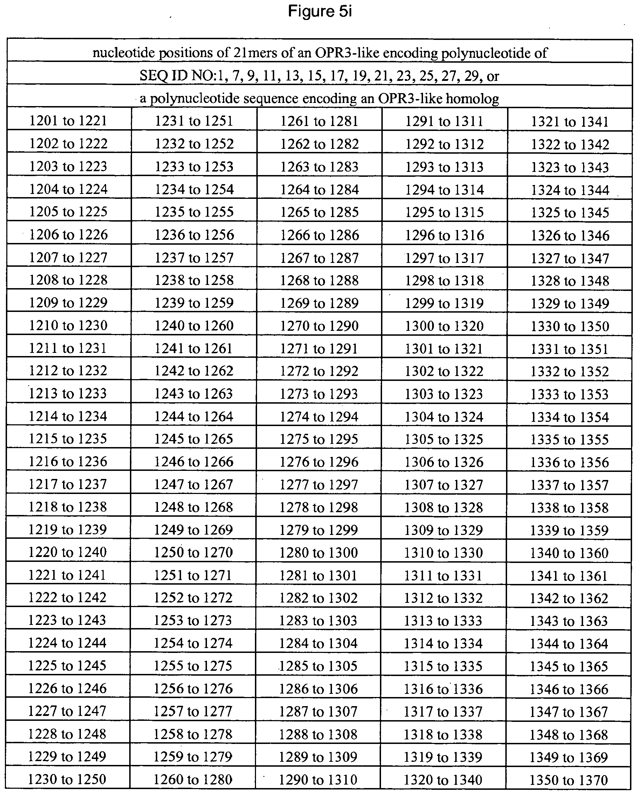

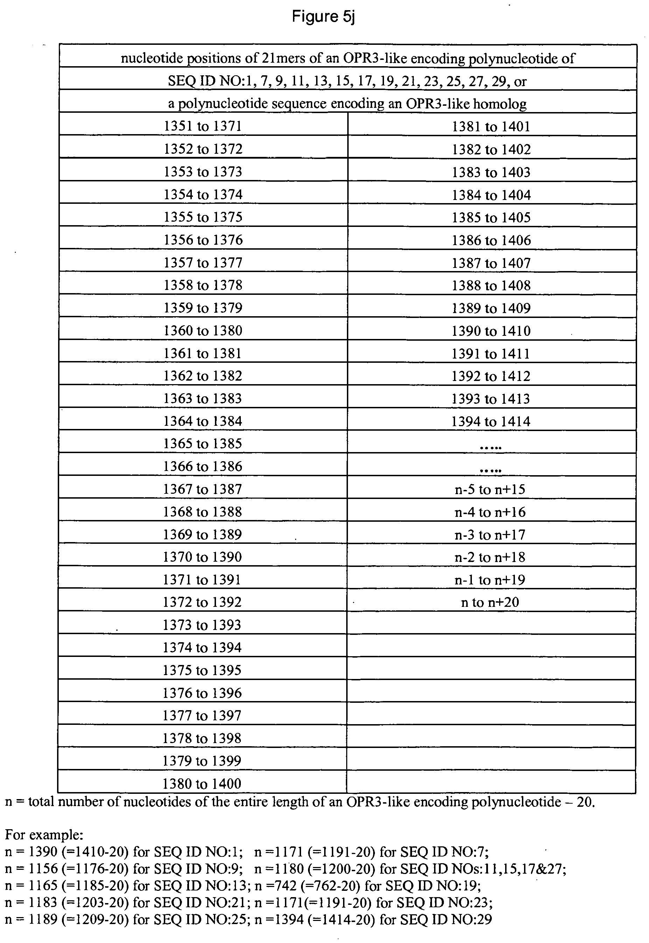

As discussed above, fragments of dsRNA larger than about 19-24 nucleotides in length are cleaved intracellularly by nematodes and plants to siRNAs of about 19-24 nucleotides in length, and these siRNAs are the actual mediators of the RNAi phenomenon. The table in Figures 5a-5j sets forth exemplary 21-mers of the OPR3-like genes defined herein. This table can also be used to calculate the 19, 20, 22, 23 or 24-mers by adding or subtracting the appropriate number of nucleotides from each 21 mer. Thus the dsRNA of the present invention may range in length from about 21 nucleotides to 200 nucleotides. Preferably, the dsRNA referred to in accordance with the invention has a length from about 21 nucleotides to 600 consecutive nucleotides or up to the whole length of the OPR3-like gene. More preferably, said dsRNA has a length from about 21 nucleotides to 500 consecutive nucleotides, or from about 21 nucleotides to about 200 consecutive nucleotides.

As disclosed herein, 100% sequence identity between the RNA and the target gene is not required to practice the present invention. While a dsRNA comprising a nucleotide sequence identical to a portion of the OPR3-like gene is preferred for inhibition, the invention can tolerate sequence variations that might be expected due to gene manipulation or synthesis, genetic mutation, strain polymorphism, or evolutionary divergence. Thus the dsRNAs also encompass dsRNAs comprising a mismatch with the target gene of at least 1, 2, or more nucleotides. For example, it is contemplated in the present invention that the 21 mer dsRNA sequences exemplified in Figures 5a-5j may contain an addition, deletion or substitution of 1, 2, or more nucleotides, so long as the resulting sequence still interferes with the OPR3-like gene function.

Sequence identity between the dsRNAs and the OPR3-like target genes may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). Greater than 80 % sequence identity, 90% sequence identity, or even 100% sequence identity, between the inhibitory RNA and the portion of the target gene is preferred. Alternatively, the duplex region of the RNA may be defined functionally as a nucleotide sequence that is capable of hybridizing with a portion of the target gene transcript under stringent conditions (e.g., 400 mM NaCl, 40 mM PIPES pH 6.4, 1 mM EDTA, 60°C hybridization for 12-16 hours; followed by washing at 65°C with 0.1 %SDS and 0.1 % SSC for about 15-60 minutes).

When dsRNA has a length longer than about 21 nucleotides, for example from 50 nucleotides to 1000 nucleotides, it will be cleaved randomly to dsRNAs of about 21 nucleotides within the plant or parasitic nematode cell, the siRNAs. The cleavage of a longer dsRNA of the invention will yield a pool of about 21 mer dsRNAs (ranging from 19mers to 24mers), derived from the longer dsRNA. This pool of about 21 mer dsRNAs is also encompassed within the scope of the present invention, whether generated intracellularly within the plant or nematode or synthetically using known methods of oligonucleotide synthesis.

The siRNAs referred to in accordance with the invention have sequences corresponding to fragments of 19-24 contiguous nucleotides across the entire sequence of an OPR3-like target gene. For example, a pool of siRNA of the invention derived from the OPR3-like genes as set forth in SEQ ID NO: 1, 2, or 29 may comprise a multiplicity of RNA molecules which are selected from the group consisting of oligonucleotides comprising one strand which is substantially identical to the 21 mer nucleotides of SEQ ID NO: 1, 2, or 29 found in Figures 5a-5j. A pool of siRNA as referred to in accordance with the invention derived from OPR3-like genes described by SEQ ID NO: 1, 2, or 29 may also comprise any combination of the specific RNA molecules having any of the 21 contiguous nucleotide sequences derived from SEQ ID NO: 1, 2, or 29 set forth in Figures 5a-5j. Further, as noted above, multiple specialized Dicers in plants generate siRNAs typically ranging in size from 19nt to 24nt (See Henderson et al., 2006. Nature Genetics 38:721-725.). The said siRNAs may range from about 19 contiguous nucleotide sequences to about 24 contiguous nucleotide sequences. Similarly, a pool of siRNA of the invention may comprise a multiplicity of RNA molecules having any of about 19, 20, 21, 22, 23, or 24 contiguous nucleotide sequences derived from SEQ ID NO: 1, 2. Alternatively, the pool of siRNA may comprise a multiplicity of RNA molecules having a combination of any of about 19, 20, 21, 22, 23, and/or 24 contiguous nucleotide sequences derived from SEQ ID NO: 1, 2, or 29.

The dsRNA referred to in accordance with the invention may optionally comprise a single stranded overhang at either or both ends. The double-stranded structure may be formed by a single self-complementary RNA strand (i.e. forming a hairpin loop) or two complementary RNA strands. RNA duplex formation may be initiated either inside or outside the cell. When the dsRNA of the invention forms a hairpin loop, it may optionally comprise an intron, as set forth in US 2003/0180945A1 or a nucleotide spacer, which is a stretch of sequence between the complementary RNA strands to stabilize the hairpin transgene in cells. Methods for making various dsRNA molecules are set forth, for example, in WO 99/53050 and in U.S. Pat. No. 6,506,559. The RNA may be introduced in an amount that allows delivery of at least one copy per cell. Higher doses of double-stranded material may yield more effective inhibition.

Disclosed herein is an isolated recombinant expression vector comprising a nucleic acid encoding a dsRNA molecule as described above, wherein expression of the vector in a host plant cell results in increased resistance to a parasitic nematode as compared to a wild-type variety of the host plant cell. As used herein, the term "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. Certain vectors are capable of autonomous replication in a host plant cell into which they are introduced. Other vectors are integrated into the genome of a host plant cell upon introduction into the host cell, and thereby are replicated along with the host genome. Moreover, certain vectors are capable of directing the expression of genes to which they are operatively linked. Such vectors are referred to herein as "expression vectors." In general, expression vectors of utility in recombinant DNA techniques are often in the form of plasmids. In the present specification, "plasmid" and "vector can be used interchangeably as the plasmid is the most commonly used form of vector. However, it is intended to include such other forms of expression vectors, such as viral vectors (e.g., potato virus X, tobacco rattle virus, and Geminivirus), which serve equivalent functions.

The said recombinant expression vectors comprise a nucleic acid of the invention in a form suitable for expression of the nucleic acid in a host plant cell, which means that the recombinant expression vector includes one or more regulatory sequences, e.g. promoters, selected on the basis of the host plant cells to be used for expression, which is operatively linked to the nucleic acid sequence to be expressed. With respect to a recombinant expression vector, the terms "operatively linked" and "in operative association" are interchangeable and are intended to mean that the nucleotide sequence of interest is linked to the regulatory sequence(s) in a manner which allows for expression of the nucleotide sequence (e.g., in a host plant cell when the vector is introduced into the host plant cell). The term "regulatory sequence" is intended to include promoters, enhancers, and other expression control elements (e.g., polyadenylation signals). Such regulatory sequences are described, for example, in Goeddel, Gene Expression Technology: Methods in Enzymology 185, Academic Press, San Diego, CA (1990) and Gruber and Crosby, in: Methods in Plant Molecular Biology and Biotechnology, Eds. Glick and Thompson, Chapter 7, 89-108, CRC Press: Boca Raton, Florida, including the references therein. Regulatory sequences include those that direct constitutive expression of a nucleotide sequence in many types of host cells and those that direct expression of the nucleotide sequence only in certain host cells or under certain conditions. It will be appreciated by those skilled in the art that the design of the expression vector can depend on such factors as the choice of the host cell to be transformed, the level of expression of dsRNA desired, etc. The expression vectors of the invention can be introduced into plant host cells to thereby produce dsRNA molecules of the invention encoded by nucleic acids as described herein.

The recombinant expression vector comprises a regulatory sequence operatively linked to a nucleotide sequence that is a template for one or both strands of the dsRNA molecules of the invention. The nucleic acid molecule further comprises a promoter flanking either end of the nucleic acid molecule, wherein the promoters drive expression of each individual DNA strand, thereby generating two complementary RNAs that hybridize and form the dsRNA. The nucleic acid molecule comprises a nucleotide sequence that is transcribed into both strands of the dsRNA on one transcription unit, wherein the sense strand is transcribed from the 5' end of the transcription unit and the antisense strand is transcribed from the 3' end, wherein the two strands are separated by about 3 to about 500 base pairs or more, and wherein after transcription, the RNA transcript folds on itself to form a hairpin. The spacer region in the hairpin transcript may be any DNA fragment.

An introduced polynucleotide may be maintained in the plant cell stably if it is incorporated into a non-chromosomal autonomous replicon or integrated into the plant chromosomes. Alternatively, the introduced polynucleotide may be present on an extra-chromosomal non-replicating vector and be transiently expressed or transiently active. Whether present in an extra-chromosomal non-replicating vector or a vector that is integrated into a chromosome, the polynucleotide preferably resides in a plant expression cassette. A plant expression cassette preferably contains regulatory sequences capable of driving gene expression in plant cells that are operatively linked so that each sequence can fulfill its function, for example, termination of transcription by polyadenylation signals. Preferred polyadenylation signals are those originating from Agrobacterium tumefaciens t-DNA such as the gene 3 known as octopine synthase of the Ti-plasmid pTiACH5 (Gielen et al., 1984, EMBO J. 3:835) or functional equivalents thereof, but also all other terminators functionally active in plants are suitable. As plant gene expression is very often not limited at transcriptional levels, a plant expression cassette preferably contains other operatively linked sequences like translational enhancers such as the overdrive-sequence containing the 5'-untranslated leader sequence from tobacco mosaic virus enhancing the polypeptide per RNA ratio (Gallie et al., 1987, Nucl. Acids Research 15:8693-8711). Examples of plant expression vectors include those detailed in: Becker, D. et al., 1992, New plant binary vectors with selectable markers located proximal to the left border, Plant Mol. Biol. 20:1195-1197; Bevan, M.W., 1984, Binary Agrobacterium vectors for plant transformation, Nucl. Acid. Res. 12:8711-8721; and Vectors for Gene Transfer in Higher Plants; in: Transgenic Plants, Vol. 1, Engineering and Utilization, eds.: Kung and R. Wu, Academic Press, 1993, S. 15-38.

Plant gene expression should be operatively linked to an appropriate promoter conferring gene expression in a temporal-preferred, spatial-preferred, cell type-preferred, and/or tissue-preferred manner. Promoters useful in the expression cassettes of the invention include any promoter that is capable of initiating transcription in a plant cell present in the plant's roots. Such promoters include, but are not limited to those that can be obtained from plants, plant viruses and bacteria that contain genes that are expressed in plants, such as Agrobacterium and Rhizobium. Preferably, the expression cassette of the invention comprises a root-specfic promoter, a pathogen inducible promoter, or a nematode inducible promoter. More preferably the nematode inducible promoter is a parasitic nematode feeding site-specific promoter. A parasitic nematode feeding site-specific promoter may be specific for syncytial cells or giant cells or specific for both kinds of cells. A promoter is inducible, if its activity, measured on the amount of RNA produced under control of the promoter, is at least 30%, 40%, 50% preferably at least 60%, 70%, 80%, 90% more preferred at least 100%, 200%, 300% higher in its induced state, than in its un-induced state. A promoter is cell-, tissue- or organ-specific, if its activity , measured on the amount of RNA produced under control of the promoter, is at least 30%, 40%, 50% preferably at least 60%, 70%, 80%, 90% more preferred at least 100%, 200%, 300% higher in a particular cell-type, tissue or organ, then in other cell-types or tissues of the same plant, preferably the other cell-types or tissues are cell types or tissues of the same plant organ, e.g. a root. In the case of organ specific promoters, the promoter activity has to be compared to the promoter activity in other plant organs, e.g. leaves, stems, flowers or seeds.

The promoter may be constitutive, inducible, developmental stage-preferred, cell type-preferred, tissue-preferred or organ-preferred. Constitutive promoters are active under most conditions. Non-limiting examples of constitutive promoters include the CaMV 19S and 35S promoters (Odell et al., 1985, Nature 313:810-812), the sX CaMV 35S promoter (Kay et al., 1987, Science 236:1299-1302), the Sep1 promoter, the rice actin promoter (McElroy et al., 1990, Plant Cell 2:163-171), the Arabidopsis actin promoter, the ubiquitin promoter (Christensen et al., 1989, Plant Molec. Biol. 18:675-689); pEmu (Last et al., 1991, Theor. Appl. Genet. 81:581-588), the figwort mosaic virus 35S promoter, the Smas promoter (Velten et al., 1984, EMBO J. 3:2723-2730), the GRP1-8 promoter, the cinnamyl alcohol dehydrogenase promoter (U.S. Patent No. 5,683,439), promoters from the T-DNA of Agrobacterium, such as mannopine synthase, nopaline synthase, and octopine synthase, the small subunit of ribulose biphosphate carboxylase (ssuRUBISCO) promoter, and the like. Promoters that express the dsRNA in a cell that is contacted by parasitic nematodes are preferred. Alternatively, the promoter may drive expression of the dsRNA in a plant tissue remote from the site of contact with the nematode, and the dsRNA may then be transported by the plant to a cell that is contacted by the parasitic nematode in particular cells of, or close by nematode feeding sites, e.g. syncytial cells or giant cells.

Inducible promoters are active under certain environmental conditions, such as the presence or absence of a nutrient or metabolite, heat or cold, light, pathogen attack, anaerobic conditions, and the like. For example, the promoters TobRB7, AtRPE, AtPyk10, Gemini19, and AtHMG1 have been shown to be induced by nematodes (for a review of nematode-inducible promoters, see Ann. Rev. Phytopathol. (2002) 40:191-219; see also U.S. Pat. No. 6,593,513). Method for isolating additional promoters, which are inducible by nematodes are set forth in U.S. Pat. Nos. 5,589,622 and 5,824,876. Other inducible promoters include the hsp80 promoter from Brassica, being inducible by heat shock; the PPDK promoter is induced by light; the PR-1 promoter from tobacco, Arabidopsis, and maize are inducible by infection with a pathogen; and the Adh1 promoter is induced by hypoxia and cold stress. Plant gene expression can also be facilitated via an inducible promoter (For review, see Gatz, 1997, Annu. Rev. Plant Physiol. Plant Mol. Biol. 48:89-108). Chemically inducible promoters are especially suitable if time-specific gene expression is desired. Non-limiting examples of such promoters are a salicylic acid inducible promoter (PCT Application No. WO 95/19443), a tetracycline inducible promoter (Gatz et al., 1992, Plant J. 2:397-404) and an ethanol inducible promoter (PCT Application No. WO 93/21334).

Developmental stage-preferred promoters are preferentially expressed at certain stages of development. Tissue and organ preferred promoters include those that are preferentially expressed in certain tissues or organs, such as leaves, roots, seeds, or xylem. Examples of tissue preferred and organ preferred promoters include, but are not limited to fruit-preferred, ovule-preferred, male tissue-preferred, seed-preferred, integument-preferred, tuber-preferred, stalk-preferred, pericarp-preferred, and leaf-preferred, stigma-preferred, pollen-preferred, anther-preferred, a petal-preferred, sepal-preferred, pedicel-preferred, silique-preferred, stem-preferred, root-preferred promoters and the like. Seed preferred promoters are preferentially expressed during seed development and/or germination. For example, seed preferred promoters can be embryo-preferred, endosperm preferred and seed coat-preferred. See Thompson et al., 1989, BioEssays 10:108. Examples of seed preferred promoters include, but are not limited to cellulose synthase (celA), Cim1, gamma-zein, globulin-1, maize 19 kD zein (cZ19B1) and the like.

Other suitable tissue-preferred or organ-preferred promoters include, but are not limited to, the napin-gene promoter from rapeseed (U.S. Patent No. 5,608,152), the USP-promoter from Vicia faba (Baeumlein et al., 1991, Mol Gen Genet. 225(3):459-67), the oleosin-promoter from Arabidopsis (PCT Application No. WO 98/45461), the phaseolin-promoter from Phaseolus vulgaris (U.S. Patent No. 5,504,200), the Bce4-promoter from Brassica (PCT Application No. WO 91/13980), or the legumin B4 promoter (LeB4; Baeumlein et al., 1992, Plant Journal, 2(2):233-9), as well as promoters conferring seed specific expression in monocot plants like maize, barley, wheat, rye, rice, etc. Suitable promoters to note are the Ipt2 or Ipt1-gene promoter from barley (PCT Application No. WO 95/15389 and PCT Application No. WO 95/23230) or those described in PCT Application No. WO 99/16890 (promoters from the barley hordein-gene, rice glutelin gene, rice oryzin gene, rice prolamin gene, wheat gliadin gene, wheat glutelin gene, oat glutelin gene, Sorghum kasirin-gene, and rye secalin gene).

Other promoters useful in the expression cassettes of the invention include, but are not limited to, the major chlorophyll a/b binding protein promoter, histone promoters, the Ap3 promoter, the β-conglycin promoter, the napin promoter, the soybean lectin promoter, the maize 15kD zein promoter, the 22kD zein promoter, the 27kD zein promoter, the g-zein promoter, the waxy, shrunken 1, shrunken 2, and bronze promoters, the Zm13 promoter (U.S. Patent No. 5,086,169), the maize polygalacturonase promoters (PG) (U.S. Patent Nos. 5,412,085 and 5,545,546), and the SGB6 promoter (U.S. Patent No. 5,470,359), as well as synthetic or other natural promoters.

An expression cassette comprises an expression control sequence operatively linked to a nucleotide sequence that is a template for one or both strands of the dsRNA. The dsRNA template comprises (a) a first stand having a sequence substantially identical to from about 19 to 500, or up to the full length, consecutive nucleotides of an OPR3-like gene; and (b) a second strand having a sequence substantially complementary to the first strand. In further embodiments, a promoter flanks either end of the template nucleotide sequence, wherein the promoters drive expression of each individual DNA strand, thereby generating two complementary RNAs that hybridize and form the dsRNA. In alternative embodiments, the nucleotide sequence is transcribed into both strands of the dsRNA on one transcription unit, wherein the sense strand is transcribed from the 5' end of the transcription unit and the anti-sense strand is transcribed from the 3' end, wherein the two strands are separated by 3 to 500 base pairs, and wherein after transcription, the RNA transcript folds on itself to form a hairpin.

The vector may also contain a bidirectional promoter, driving expression of two nucleic acid molecules, whereby one nucleic acid molecule codes for the sequence substantially identical to a portion of a OPR3-like gene and the other nucleic acid molecule codes for a second sequence being substantially complementary to the first strand and capable of forming a dsRNA, when both sequences are transcribed. A bidirectional promoter is a promoter capable of mediating expression in two directions.

The vector may also contain two promoters one mediating transcription of the sequence substantially identical to a portion of a OPR3-like gene and another promoter mediating transcription of a second sequence being substantially complementary to the first strand and capable of forming a dsRNA, when both sequences are transcribed. The second promoter might be a different promoter.

A different promoter means a promoter having a different activity in regard to cell or tissue specificity, or showing expression on different inducers for example, pathogens, abiotic stress or chemicals. For example, one promoter might by constitutive or tissue specific and another might be tissue specific or inducible by pathogens. In one embodiment one promoter mediates the transcription of one nucleic acid molecule suitable for overexpression of an OPR3-like gene, while another promoter mediates tissue- or cell-specific transcription or pathogen inducible expression of the complementary nucleic acid.

The invention is also embodied in a transgenic plant capable of expressing the dsRNA referred to above and thereby inhibiting the target genes e.g. in the roots, feeding site, syncytia and/or giant cell. The plant or transgenic plant may be any plant, such like, but not limited to trees, cut flowers, ornamentals, vegetables or crop plants. The plant may be from a genus selected from the group consisting of Medicago, Lycopersicon, Brassica, Cucumis, Solanum, Juglans, Gossypium, Malus, Vitis, Antirrhinum, Populus, Fragaria, Arabidopsis, Picea, Capsicum, Chenopodium, Dendranthema, Pharbitis, Pinus, Pisum, Oryza, Zea, Triticum, Triticale, Secale, Lolium, Hordeum, Glycine, Pseudotsuga, Kalanchoe, Beta, Helianthus, Nicotiana, Cucurbita, Rosa, Fragaria, Lotus, Medicago, Onobrychis, trifolium, Trigonella, Vigna, Citrus, Linum, Geranium, Manihot, Daucus, Raphanus, Sinapis, Atropa, Datura, Hyoscyamus, Nicotiana, Petunia, Digitalis, Majorana, Ciahorium, Lactuca, Bromus, Asparagus, Antirrhinum, Heterocallis, Nemesis, Pelargonium, Panieum, Pennisetum, Ranunculus, Senecio, Salpiglossis, Browaalia, Phaseolus, Avena, and Allium, or the plant may be selected from a genus selected from the group consisting of Arabidopsis, Medicago, Lycopersicon, Brassica, Cucumis, Solanum, Juglans, Gossypium, Malus, Vitis, Antirrhinum, Brachipodium, Populus, Fragaria, Arabidopsis, Picea, Capsicum, Chenopodium, Dendranthema, Pharbitis, Pinus, Pisum, Oryza, Zea, Triticum, Triticale, Secale, Lolium, Hordeum, Glycine, Pseudotsuga, Kalanchoe, Beta, Helianthus, Nicotiana, Cucurbita, Rosa, Fragaria, Lotus, Medicago, Onobrychis, trifolium, Trigonella, Vigna, Citrus, Linum, Geranium, Manihot, Daucus, Raphanus, Sinapis, Atropa, Datura, Hyoscyamus, Nicotiana, Petunia, Digitalis, Majorana, Ciahorium, Lactuca, Bromus, Asparagus, Antirrhinum, Heterocallis, Nemesis, Pelargonium, Panieum, Pennisetum, Ranunculus, Senecio, Salpiglossis, Browaalia, Phaseolus, Avena, and Allium. In one embodiment the plant is a monocotyledonous plant or a dicotyledonous plant.

In another embodiment the plant is a crop plant. Crop plants are all plants, used in agriculture. Accordingly in one embodiment the plant is a monocotyledonous plant, preferably a plant of the family Poaceae, Musaceae, Liliaceae or Bromeliaceae, preferably of the family Poaceae. Accordingly, in yet another embodiment the plant is a Poaceae plant of the genus Zea, Triticum, Oryza, Hordeum, Secale, Avena, Saccharum, Sorghum, Pennisetum, Setaria, Panicum, Eleusine, Miscanthus, Brachypodium, Festuca or Lolium. When the plant is of the genus Zea, the preferred species is Z. mays. When the plant is of the genus Triticum, the preferred species is T. aestivum, T. speltae or T. durum. When the plant is of the genus Oryza, the preferred species is O. sativa. When the plant is of the genus Hordeum, the preferred species is H. vulgare. When the plant is of the genus Secale, the preferred species, S. cereale. When the plant is of the genus Avena, the preferred species is A. sativa. When the plant is of the genus Saccarum, the preferred species is S. officinarum. When the plant is of the genus Sorghum, the preferred species is S. vulgare, S. bicolor or S. sudanense. When the plant is of the genus Pennisetum, the preferred species is P. glaucum. When the plant is of the genus Setaria, the preferred species is S. italica. When the plant is of the genus Panicum, the preferred species is P. miliaceum or P. virgatum. When the plant is of the genus Eleusine, the preferred species is E. coracana. When the plant is of the genus Miscanthus, the preferred species is M. sinensis. When the plant is a plant of the genus Festuca, the preferred species is F. arundinaria, F. rubra or F. pratensis. When the plant is of the genus Lolium, the preferred species is L. perenne or L. multiflorum. Alternatively, the plant may be Triticosecale.

Alternatively, in one embodiment the plant is a dicotyledonous plant, preferably a plant of the family Fabaceae, Solanaceae, Brassicaceae, Chenopodiaceae, Asteraceae, Malvaceae, Linacea, Euphorbiaceae, Convolvulaceae Rosaceae, Cucurbitaceae, Theaceae, Rubiaceae, Sterculiaceae or Citrus. In one embodiment the plant is a plant of the family Fabaceae, Solanaceae or Brassicaceae. Accordingly, in one embodiment the plant is of the family Fabaceae, preferably of the genus Glycine, Pisum, Arachis, Cicer, Vicia, Phaseolus, Lupinus, Medicago or Lens. Preferred species of the family Fabaceae are M. truncatula, M, sativa, G. max, P. sativum, A. hypogea, C. arietinum, V. faba, P. vulgaris, Lupinus albus, Lupinus luteus, Lupinus angustifolius or Lens culinaris. More preferred are the species G. max A. hypogea and M. sativa. Most preferred is the species G. max. When the plant is of the family Solanaceae, the preferred genus is Solanum, Lycopersicon, Nicotiana or Capsicum. Preferred species of the family Solanaceae are S. tuberosum, L. esculentum, N. tabaccum or C. chinense. More preferred is S. tuberosum. Accordingly, in one embodiment the plant is of the family Brassicaceae, preferably of the genus Brassica or Raphanus. Preferred species of the family Brassicaceae are the species B. napus, B. oleracea, B. juncea or B. rapa. More preferred is the species B. napus. When the plant is of the family Chenopodiaceae, the preferred genus is Beta and the preferred species is the B. vulgaris. When the plant is of the family Asteraceae, the preferred genus is Helianthus and the preferred species is H. annuus. When the plant is of the family Malvaceae, the preferred genus is Gossypium or Abelmoschus. When the genus is Gossypium, the preferred species is G. hirsutum or G. barbadense and the most preferred species is G. hirsutum. A preferred species of the genus Abelmoschus is the species A. esculentus. When the plant is of the family Linacea, the preferred genus is Linum and the preferred species is L. usitatissimum. When the plant is of the family Euphorbiaceae, the preferred genus is Manihot, Jatropa or Rhizinus and the preferred species are M. esculenta, J. curcas or R. comunis. When the plant is of the family Convolvulaceae, the preferred genus is Ipomea and the preferred species is I. batatas. When the plant is of the family Rosaceae, the preferred genus is Rosa, Malus, Pyrus, Prunus, Rubus, Ribes, Vaccinium or Fragaria and the preferred species is the hybrid Fragaria x ananassa. When the plant is of the family Cucurbitaceae, the preferred genus is Cucumis, Citrullus or Cucurbita and the preferred species is Cucumis sativus, Citrullus lanatus or Cucurbita pepo. When the plant is of the family Theaceae, the preferred genus is Camellia and the preferred species is C. sinensis. When the plant is of the family Rubiaceae, the preferred genus is Coffea and the preferred species, is C. arabica or C. canephora. When the plant is of the family Sterculiaceae, the preferred genus is Theo-broma and the preferred species is T. cacao. When the plant is of the genus Citrus, the preferred species is C. sinensis, C. limon, C. reticulata, C. maxima and hybrids of Citrus species, or the like. In a preferred embodiment of the invention, the plant is a soybean, a potato or a corn plant. In one embodiment the plant is a Fabaceae plant and the target gene is substantially similar to SEQ ID NO: 1, 2, or 29.

Suitable methods for transforming or transfecting host cells including plant cells are well known in the art of plant biotechnology. Any method may be used to transform the recombinant expression vector into plant cells to yield the transgenic plants of the invention. General methods for transforming dicotyledenous plants are disclosed, for example, in U.S. Pat. Nos. 4,940,838; 5,464,763, and the like. Methods for transforming specific dicotyledenous plants, for example, cotton, are set forth in U.S. Pat. Nos. 5,004,863; 5,159,135; and 5,846,797. Soybean transformation methods are set forth in U.S. Pat. Nos. 4,992,375; 5,416,011; 5,569,834; 5,824,877; 6,384,301 and in EP 0301749B1 may be used.

Transformation methods may include direct and indirect methods of transformation. Suitable direct methods include polyethylene glycol induced DNA uptake, liposome-mediated transformation (US 4,536,475), biolistic methods using the gene gun (Fromm ME et al., Biol/Technology 8(9):833-9, 1990; Gordon-Kamm et al. Plant Cell 2:603, 1990), electroporation, incubation of dry plant embryos in DNA-comprising solution, and microinjection. In the case of these direct transformation methods, the plasmids used need not meet any particular requirements. Simple plasmids, such as those of the pUC series, pBR322, M13mp series, pACYC184 and the like can be used. If intact plants are to be regenerated from the transformed cells, an additional selectable marker gene is preferably located on the plasmid. The direct transformation techniques are equally suitable for dicotyledonous and monocotyledonous plants.

Transformation can also be carried out by bacterial infection by means of Agrobacterium (for example EP 0 116 718), viral infection by means of viral vectors (EP 0 067 553; US 4,407,956; WO 95/34668; WO 93/03161) or by means of pollen (EP 0 270 356; WO 85/01856; US 4,684,611). Agrobacterium based transformation techniques (especially for dicotyledonous plants) are well known in the art. The Agrobacterium strain (e.g., Agrobacterium tumefaciens or Agrobacterium rhizogenes) comprises a plasmid (Ti or Ri plasmid) and a T-DNA element which is transferred to the plant following infection with Agrobacterium. The T-DNA (transferred DNA) is integrated into the genome of the plant cell. The T-DNA may be localized on the Ri- or Ti-plasmid or is separately comprised in a so-called binary vector. Methods for the Agrobacterium-mediated transformation are described, for example, in Horsch RB et al. (1985) Science 225:1229. The Agrobacterium-mediated transformation is best suited to dicotyledonous plants but has also been adapted to monocotyledonous plants. The transformation of plants by Agrobacteria is described in, for example, White FF, Vectors for Gene Transfer in Higher Plants, Transgenic Plants, Vol. 1, Engineering and Utilization, edited by S.D. Kung and R. Wu, Academic Press, 1993, pp. 15 - 38; Jenes B et al. Techniques for Gene Transfer, Transgenic Plants, Vol. 1, Engineering and Utilization, edited by S.D. Kung and R. Wu, Academic Press, 1993, pp. 128-143; Potrykus (1991) Annu Rev Plant Physiol Plant Molec Biol 42:205- 225.

Transformation may result in transient or stable transformation and expression. Although a nucleotide sequence of the present invention can be inserted into any plant and plant cell falling within these broad classes, it is particularly useful in crop plant cells.

Various tissues are suitable as starting material (explant) for the Agrobacterium-mediated transformation process including but not limited to callus (US 5,591,616; EP-A1 604 662), immature plant embryos (EP-A1 672 752), pollen (US 54,929,300), shoot apex (US 5,164,310), or in planta transformation (US 5,994,624). The method and material described herein can be combined with virtually all Agrobacterium mediated transformation methods known in the art. The transgenic plants of the invention may be crossed with similar transgenic plants or with transgenic plants lacking the nucleic acids of the invention or with non-transgenic plants, using known methods of plant breeding, to prepare seeds. Further, the transgenic plant of the present invention may comprise, and/or be crossed to another transgenic plant that comprises one or more nucleic acids, thus creating a "stack" of transgenes in the plant and/or its progeny. The seed is then planted to obtain a crossed fertile transgenic plant comprising the nucleic acid of the invention. The crossed fertile transgenic plant may have the particular expression cassette inherited through a female parent or through a male parent. The second plant may be an inbred plant. The crossed fertile transgenic may be a hybrid. Also included within the present invention are seeds of any of these crossed fertile transgenic plants. The seeds of this invention can be harvested from fertile transgenic plants and be used to grow progeny generations of transformed plants of this invention including hybrid plant lines comprising the DNA construct.

"Gene stacking" can also be accomplished by transferring two or more genes into the cell nucleus by plant transformation. Multiple genes may be introduced into the cell nucleus during transformation either sequentially or in unison. Multiple genes in plants or target pathogen species can be down-regulated by gene silencing mechanisms, specifically RNAi, by using a single transgene targeting multiple linked partial sequences of interest. Stacked, multiple genes under the control of individual promoters can also be over-expressed to attain a desired single or multiple phenotype. Constructs containing gene stacks of both over-expressed genes and silenced targets can also be introduced into plants yielding single or multiple agronomically important phenotypes. In certain embodiments the nucleic acid sequences of the present invention can be stacked with any combination of polynucleotide sequences of interest to create desired phenotypes. The combinations can produce plants with a variety of trait combinations including but not limited to disease resistance, herbicide tolerance, yield enhancement, cold and drought tolerance. These stacked combinations can be created by any method including but not limited to cross breeding plants by conventional methods or by genetic transformation. If the traits are stacked by genetic transformation, the polynucleotide sequences of interest can be combined sequentially or simultaneously in any order. For example if two genes are to be introduced, the two sequences can be contained in separate transformation cassettes or on the same transformation cassette. The expression of the sequences can be driven by the same or different promoters.

In accordance with this embodiment, the transgenic plant of the invention is produced by a method comprising the steps of providing an OPR3-like target gene, preparing an expression cassette having a first region that is substantially identical to a portion of the selected OPR3-like gene and a second region which is complementary to the first region, transforming the expression cassette into a plant, and selecting progeny of the transformed plant which express the dsRNA construct referred to above.

Increased resistance to nematode infection is a general trait wished to be inherited into a wide variety of plants. The present invention may be used to reduce crop destruction by any plant parasitic nematode. Preferably, the parasitic nematodes belong to nematode families inducing giant or syncytial cells. Nematodes inducing giant or syncytial cells are found in the families Longidoridae, Trichodoridae, Heterodidae, Meloidogynidae, Pratylenchidae or Tylenchulidae. In particular in the families Heterodidae and Meloidogynidae.

Accordingly, parasitic nematodes targeted by the present invention belong to one or more genus selected from the group of Naccobus, Cactodera, Dolichodera, Globodera, Heterodera, Punctodera, Longidorus or Meloidogyne. In a preferred embodiment the parasitic nematodes belong to one or more genus selected from the group of Naccobus, Cactodera, Dolichodera, Globodera, Heterodera, Punctodera or Meloidogyne. In a more preferred embodiment the parasitic nematodes belong to one or more genus selected from the group of Globodera, Heterodera, or Meloidogyne. In an even more preferred embodiment the parasitic nematodes belong to one or both genus selected from the group of Globodera or Heterodera. In another embodiment the parasitic nematodes belong to the genus Meloidogyne.

When the parasitic nematodes are of the genus Globodera, the species are preferably from the group consisting of G. achilleae, G. artemisiae, G. hypolysi, G. mexicana, G. millefolii, G. mali, G. pallida, G. rostochiensis, G. tabacum, and G. virginiae. In another preferred embodiment the parasitic Globodera nematodes includes at least one of the species G. pallida, G. tabacum, or G. rostochiensis. When the parasitic nematodes are of the genus Heterodera, the species may be preferably from the group consisting of H. avenae, H. carotae, H. ciceri, H. cruciferae, H. delvii, H. elachista, H. filipjevi, H. gambiensis, H. glycines, H. goettingiana, H. graduni, H. humuli, H. hordecalis, H. latipons, H. major, H. medicaginis, H. oryzicola, H. pakistanensis, H. rosii, H. sacchari, H. schachtii, H. sorghi, H. trifolii, H. urticae, H. vigni and H. zeae. In another preferred embodiment the parasitic Heterodera nematodes include at least one of the species H. glycines, H. avenae, H. cajani, H. gottingiana, H. trifolii, H. zeae or H. schachtii. In a more preferred embodiment the parasitic nematodes includes at least one of the species H. glycines or H. schachtii. In a most preferred embodiment the parasitic nematode is the species H. glycines.

When the parasitic nematodes are of the genus Meloidogyne, the parasitic nematode may be selected from the group consisting of M. acronea, M. arabica, M. arenaria, M. artiellia, M. brevicauda, M. camelliae, M. chitwoodi, M. cofeicola, M. esigua, M. graminicola, M. hapla, M. incognita, M. indica, M. inomata, M. javanica, M. lini, M. mali, M. microcephala, M. microtyla, M. naasi, M. salasi and M. thamesi. In a preferred embodiment the parasitic nematodes includes at least one of the species M. javanica, M. incognita, M. hapla, M. arenaria or M. chitwoodi.

The following examples are not intended to limit the scope of the claims to the invention, but are rather intended to be exemplary of certain embodiments. Any variations in the exemplified methods that occur to the skilled artisan are intended to fall within the scope of the present invention.

### BRIEF DESCRIPTION OF THE DRAWINGS

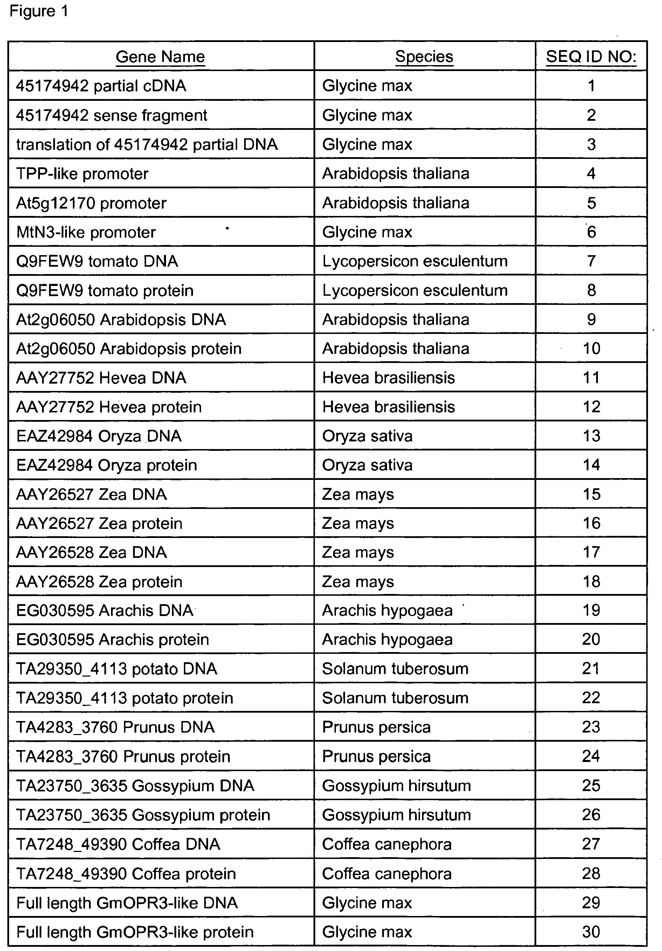

Figure 1 shows the table of SEQ ID NOs assigned to corresponding sequences.

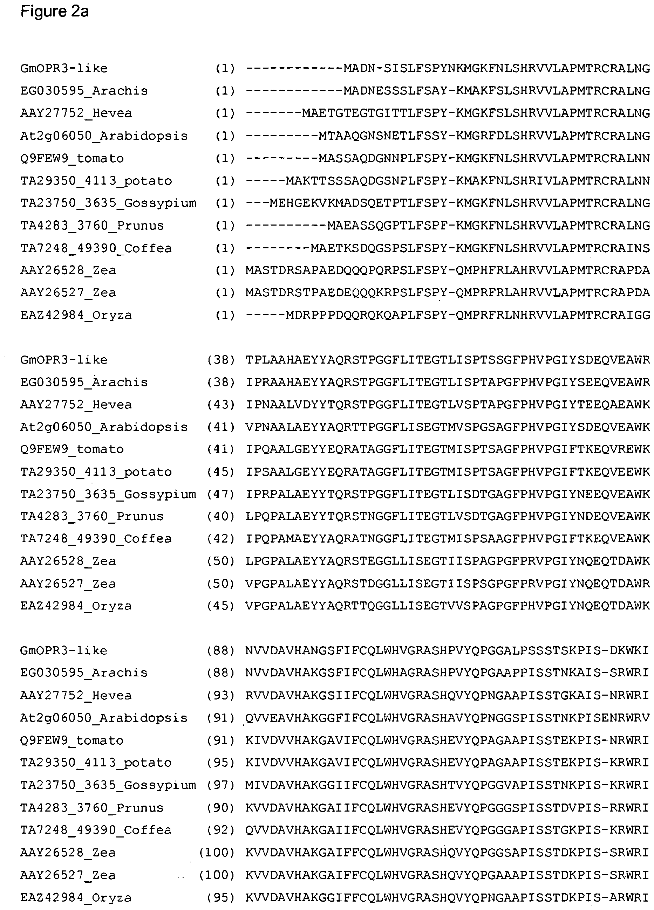

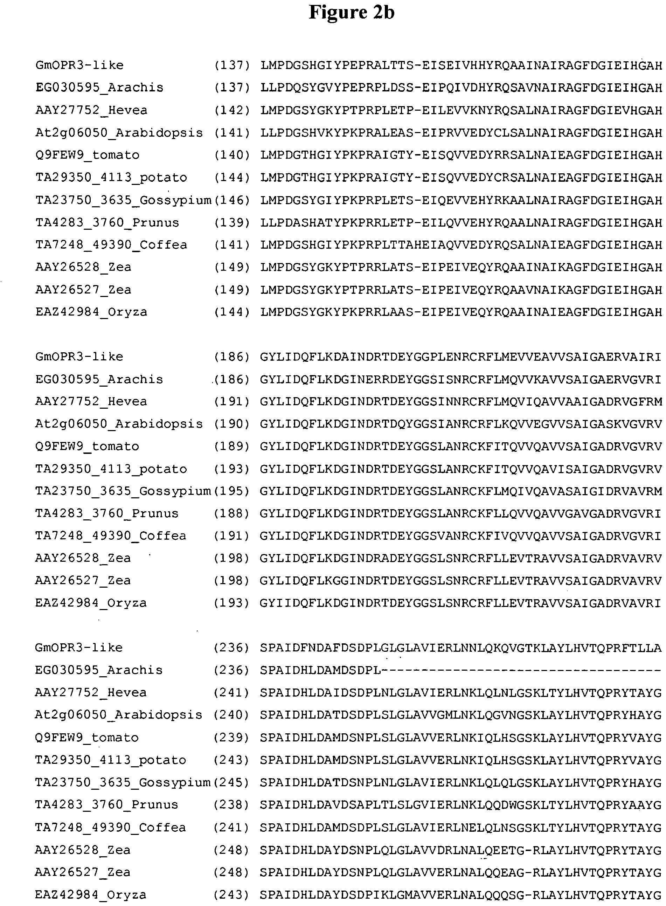

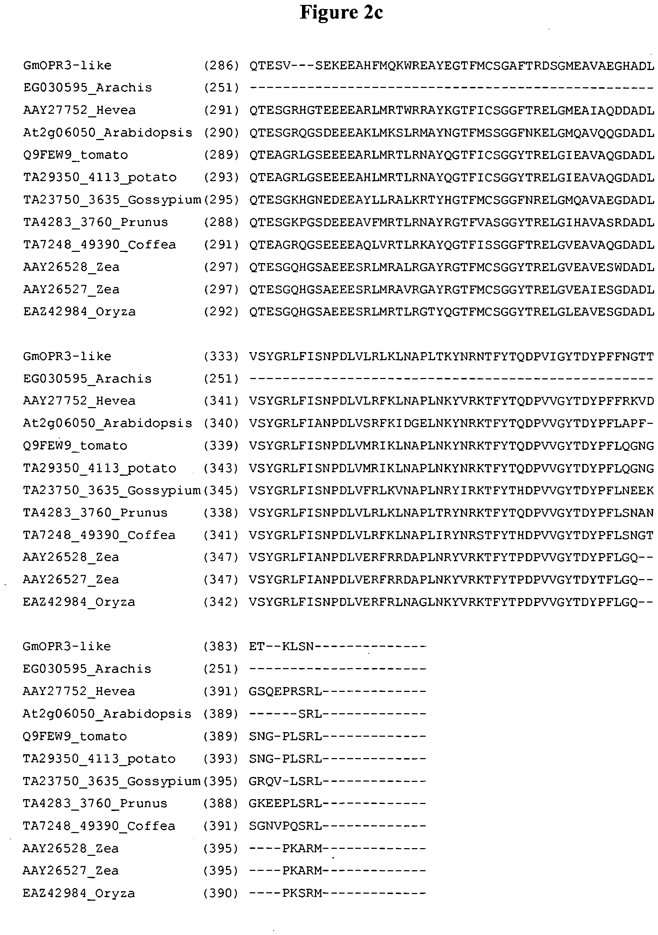

Figures 2a-2c show amino acid alignment of OPR3-like proteins: the full length GmOPR3-like protein (SEQ ID NO:30), Q9FEW9 from tomato (Seq ID NO: 8), the protein encoded by At2g06050 from Arabidopsis (SEQ ID NO: 10), AAY27752 from Hevea (SEQ ID NO: 12), EAZ42984 from rice (SEQ ID NO: 14), AAY26527 from maize (SEQ ID NO: 16), AAY26528 from maize (SEQ ID NO: 18), EG030595 from Arachis (SEQ ID NO: 20), the protein encoded by TA29350_4113 from Solanum (SEQ ID NO: 22), the protein encoded by TA4283_3760 from Prunus (SEQ ID NO: 24), the protein encoded by TA23750_3635 from Gossypium (SEQ ID NO: 26) and the protein encoded by TA7248_49390 from Coffea (SEQ ID NO:28). The alignment was performed in Vector NTI software suite (gap opening penalty = 10, gap extension penalty = 0.05, gap separation penalty = 8). The full length GmOPR3-like sequence was determined through 5' RACE PCR as described in Example 4.

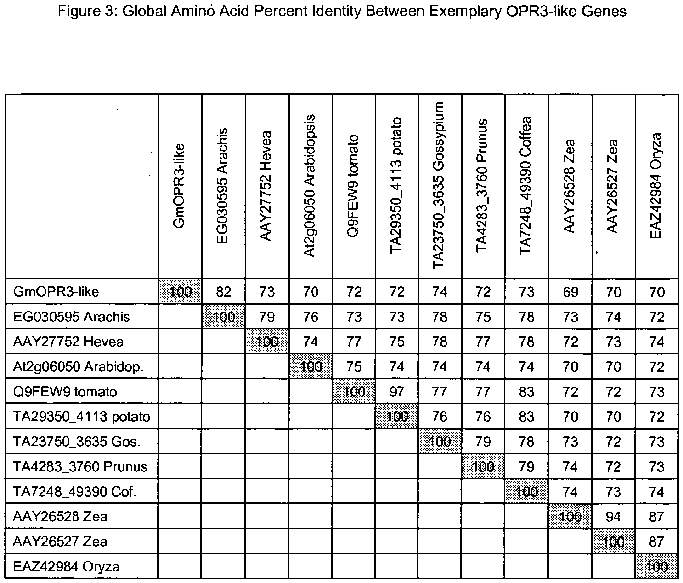

Figure 3 shows the global amino acid percent identity of exemplary OPR3-like genes: the full-length GmOPR3-like protein (SEQ ID NO:30), Q9FEW9 from tomato (Seq ID NO: 8), the protein encoded by At2g06050 from Arabidopsis (SEQ ID NO: 10). AAY27752 from Hevea (SEQ ID NO: 12), EAZ42984 from rice (SEQ ID NO: 14), AAY26527 from maize (SEQ ID NO: 16), AAY26528 from maize (SEQ ID NO: 18), EG030595 from Arachis (SEQ ID NO: 20), the protein encoded by TA29350_4113 from Solanum (SEQ ID NO: 22), the protein encoded by TA4283_3760 from Prunus (SEQ ID NO: 24), the protein encoded by TA23750_3635 from Gossypium (SEQ ID NO: 26) and the protein encoded by TA7248_49390 from Coffea (SEQ ID NO:28). Pairwise alignments and percent identities were calculated using Needle of EMBOSS-4.0.0 (Needleman, S. B. and Wunsch, C. D. (1970) J. Mol. Biol. 48, 443-453).

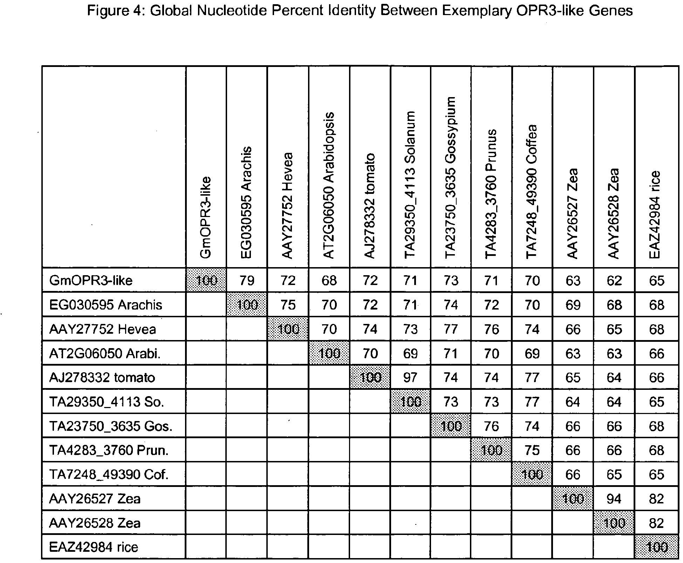

Figure 4 shows the global nucleotide percent identity of exemplary OPR3-like genes: the full-length GmOPR3-like DNA (SEQ ID NO:29), Q9FEW9 DNA from tomato (Seq ID NO: 7), At2g06050 DNA from Arabidopsis (SEQ ID NO: 9), AAY27752 DNA from Hevea (SEQ ID NO: 11), EAZ42984 DNA from rice (SEQ ID NO: 13), AAY26527 DNA from maize (SEQ ID NO: 15), AAY26528 DNA from maize (SEQ ID NO: 17), EG030595 DNA from Arachis (SEQ ID NO: 19), TA29350_4113 DNA from Solanum (SEQ ID NO: 21), TA4283_3760 DNA from Prunus (SEQ ID NO: 23), TA23750_3635 DNA from Gossypium (SEQ ID NO: 25) and TA7248_49390 DNA from Coffea (SEQ ID NO:27). Pairwise alignments and percent identities were calculated using Needle of EMBOSS-4.0.0.

Figures 5a-5j show various 21mers by nucleotide position possible for exemplary OPR3-like encoding polynucleotide of SEQ ID NO:1, 7, 9, 11, 13, 15, 17, 19, 21, 23, 25, 27, 29, or a polynucleotide sequence encoding an OPR3-like homolog.

### EXAMPLES

### Example 1: Cloning of OPR3-Like Gene from Soybean

Glycine max cv. Williams 82 was germinated and one day later, each seedling was inoculated with second stage juveniles (J2) of H. glycines race 3. Six days after inoculation, new root tissue was sliced into 1 cm long pieces, fixed, embedded in a cryomold, and sectioned using known methods. Syncytia cells were identified by their unique morphology of enlarged cell size, thickened cell wall, and dense cytoplasm and dissected into RNA extraction buffer using a PALM microscope (P.A.L.M. Microlaser Technologies GmbH, Bemried, Germany).

Total cellular RNA was extracted, amplified, and fluorescently labeled using known methods. As controls, total RNA was isolated from both "non-syncytia" and untreated control roots subjected to the same RNA amplification process. The amplified RNA was hybridized to proprietary soybean cDNA arrays.

Soybean cDNA clone 45174942 was identified as being up-regulated in syncytia of SCN-infected soybean roots. The 45174942 cDNA sequence (SEQ ID NO:1) was determined not to be full-length as there is no ATG start codon. The remaining residues were identified in the full length sequence (SEQ ID NO: 29) corresponding to 45174942 through 5' RACE PCR as described in Example 4.

**Table 2**

| Gene Name | Syncytia #1(N)^{¶} | Syncytia #2 (N) | Non-Syncytia | Control Roots |
|---|---|---|---|---|
| 45174942^{§} | 311± 54(4) | 194±46(5) | not detected | not detected |

### Example 2. GENERATION OF TRANSGENIC SOYBEAN ROOTS AND NEMATODE BIOASSAY

This exemplified method employs binary vectors containing the 45174942 target gene. The vector consists of a sense fragment (SEQ ID NO:2) of the target 45174942 gene, a spacer, antisense fragment of the target gene and a vector backbone. The target gene fragment (SEQ ID NO:2) corresponding to nucleotides 556 to 784 of SEQ ID NO:1 was used to construct the binary vectors RCB564, RCB573 and RCB582. In these vectors, dsRNA for the OPR3-like target gene was expressed under a syncytia or root preferred promoter, TPP promoter (SEQ ID NO: 4) in RCB564, A. thaliana promoter of locus At5g12170 (SEQ ID NO: 5), in RCB573 or MtN3-like promoter (SEQ ID NO: 6) in RCB582. These promoters drive transgene expression preferentially in roots and/or syncytia or giant cells. The selection marker for transformation was the mutated form of the acetohydroxy acid synthase (AHAS) selection gene (also referred to as AHAS2) from Arabidopsis thaliana (Sathasivan et al., Plant Phys. 97:1044-50, 1991), conferring resistance to the herbicide ARSENAL (imazapyr, BASF Corporation, Mount Olive, NJ). The expression of AHAS2 was driven by a ubiquitin promoter from parsley (WO 03/102198).

### Example 3. Rooted Explant Assays

The rooted explant assay was employed to demonstrate dsRNA expression and the resulting nematode resistance. This assay can be found in co-pending application 12/001,234, the contents of which are incorporated herein by reference.

Clean soybean seeds from soybean cultivar were surface sterilized and germinated. Three days before inoculation, an overnight liquid culture of the disarmed Agrobacterium culture, for example, the disarmed A. rhizogenes strain K599 containing the binary vector RCB564, RCB573 or RCB582, was initiated. The next day the culture was spread onto an LB agar plate containing kanamycin as a selection agent. The plates were incubated at 28_{°}C for two days. One plate was prepared for every 50 explants to be inoculated. Cotyledons containing the proximal end from its connection with the seedlings were used as the explant for transformation. After removing the cotyledons the surface was scraped with a scalpel around the cut site. The cut and scraped cotyledon was the target for Agrobacterium inoculation. The prepared explants were dipped onto the disarmed thick A. rhizogenes colonies prepared above so that the colonies were visible on the cut and scraped surface. The explants were then placed onto 1 % agar in Petri dishes for co-cultivation under light for 6-8 days.

After the transformation and co-cultivation, soybean explants were transferred to rooting induction medium with a selection agent, for example S-B5-708 for the mutated acetohydroxy acid synthase (AHAS) gene (Sathasivan et al., Plant Phys. 97:1044-50, 1991). Cultures were maintained in the same condition as in the co-cultivation step. The S-B5-708 medium comprises: 0.5X B5 salts, 3mM MES, 2% sucrose, 1X B5 vitamins, 400µg/ml Timentin, 0.8% Noble agar, and 1 µM Imazapyr (selection agent for AHAS gene) (BASF Corporation, Florham Park, NJ) at pH5.8.

Two to three weeks after the selection and root induction, transformed roots were formed on the cut ends of the explants. Explants were transferred to the same selection medium (S-B5-708 medium) for further selection. Transgenic roots proliferated well within one week in the medium and were ready to be subcultured.

Strong and white soybean roots were excised from the rooted explants and cultured in root growth medium supplemented with 200 mg/l Timentin (S-MS-606 medium) in six-well plates. Cultures were maintained at room temperature under the dark condition. The S-MS-606 medium comprises: 0.2X MS salts and B5 vitamins, 2% sucrose, and 200mg/l Timentin at pH5.8.

One to five days after sub-culturing, the roots were inoculated with surface sterilized nematode juveniles in multi-well plates for the gene of interest construct assay. As a control, soybean cultivar Williams 82 control vector and Jack control vector roots were used. The root cultures of each line that occupied at least half of the well were inoculated with surface-decontaminated race 3 of soybean cyst nematode (SCN) second stage juveniles (J2) at the level of 500 J2/well. The plates were then sealed and put back into the incubator at 25C in darkness. Several independent root lines were generated from each binary vector transformation and the lines were used for bioassay. Four weeks after nematode inoculation, the cysts in each well were counted.. For each transformed line, the average number of cysts per line, the female index and the standard error values were determined across several replicated wells (Female index = average number of SCN cysts developing on the transgenic roots expressed as percentage of the average number of cysts developing on the W82 wild type susceptible control roots). Multiple independent, biologically replicated experiments were run for each expression construct. Rooted explant cultures transformed with constructs RCB564, RCB573 and RCB582 exhibited a general trend of reduced cyst numbers and female index relative to the known susceptible variety, Williams82.

### Example 4. RACE PCR to Clone Full-Length 45174942 Coding Region

A full length transcript sequence with 100% homology to the partial cDNA clone 45174942 (SEQ ID NO: 1) was isolated using the GeneRacer Kit (L1502-01) from Invitrogen by following the manufacturers instructions. Total RNA from soybean roots harvested 6 days after infection with SCN was prepared according to the Invitrogen GeneRacer Kit protocol. The prepared RNA was reverse transcribed according to the GeneRacer Kit protocol and used as the RACE library template for PCR to isolate 5' cDNA ends using primary and secondary (nested) PCR reactions according to the GeneRacer Kit protocol. Nested PCR reactions were performed according to manufacturer's instructions to obtain the desired amplification product.

Specific products from secondary PCR reaction were separated by gel electrophoresis. Fragments were purified from agarose gel and cloned into pCR4-TOPO vectors (Invitrogen) following manufacturers instructions. Resulting colonies were miniprepped and sequenced. One of the full length fragments described as SEQ ID NO:29 (Full length GmOPR3-like DNA) had 100% percent identity with the overlapping region of SEQ ID NO:1 (45174942 DNA sequence). The alignment between proteins encoded by the full length Glycine max GmOPR3-iike sequence and OPR3-like genes from other plant species is shown in Figures 2a-2c.

Those skilled in the art will recognize, or will be able to ascertain using no more than routine experimentation, many equivalents to the specific embodiments of the invention described herein. Such equivalents are intended to be encompassed by the following claims.

## Claims

1. Use of a dsRNA molecule comprising i) a first strand comprising a sequence at least 80% identical to 20 or more contiguous nucleotides of a portion of a OPR3-like gene, and ii) a second strand comprising a sequence complementary over at least 80% of their nucleotides to the first strand, wherein the portion of the OPR3-like gene is from a polynucleotide selected from the group consisting of:
a) a polynucleotide comprising a sequence as set forth in SEQ ID NO:1, 2, or 29;
b) a polynucleotide encoding a polypeptide having a sequence as set forth in SEQ ID NO:3, or 30;
c) a polynucleotide having at least 70% sequence identity to a polynucleotide having a sequence as set forth in SEQ ID NO:1, 2, or 29;
d) a polynucleotide encoding a polypeptide having at least 70% sequence identity to a polypeptide having a sequence as set forth in SEQ ID NO:3, or 30;
for the generation of transgenic plants resisting nematode infestation.

2. Use of a pool of dsRNA molecules comprising a multiplicity of RNA molecules each comprising a double stranded region having a length of 19 to 24 nucleotides, wherein said dsRNA molecules are derived from a portion of a polynucleotide selected from the group consisting of:
a) a polynucleotide comprising a sequence as set forth in SEQ ID NO:1, 2, or 29;
b) a polynucleotide encoding a polypeptide having a sequence as set forth in SEQ ID NO:3, or 30;
c) a polynucleotide having at least 90% sequence identity to a polynucleotide having a sequence as set forth in SEQ ID NO:1, 2, or 29; and
d) a polynucleotide encoding a polypeptide having 90% sequence identity to a polypeptide having a sequence as set forth in SEQ ID NO:3 or 30
for the generation of transgenic plants resisting nematode infestation.

3. A transgenic nematode-resistant plant capable of expressing a dsRNA that is at least 80% identical to 20 or more contiguous nucleotides of a portion of an OPR3-like gene, wherein the portion of the OPR3-like gene is from a polynucleotide selected from the group consisting of:
a) a polynucleotide comprising a sequence as set forth in SEQ ID NO:1, 2, or 29;
b) a polynucleotide encoding a polypeptide having a sequence as set forth in SEQ ID NO:3, or 30;
c) a polynucleotide having at least 70% sequence identity to a polynucleotide having a sequence as set forth in SEQ ID NO:1, 2, or 29;
d) a polynucleotide encoding a polypeptide having at least 70% sequence identity to a polypeptide having a sequence as set forth in SEQ ID NO:3, or 30.

4. The transgenic nematode-resistant plant of claim 3, wherein the dsRNA comprises a multiplicity of RNA molecules each comprising a double stranded region having a length of 19 to 24 nucleotides, wherein said RNA molecules are derived from a portion of a polynucleotide selected from the group consisting of:
a) a polynucleotide comprising a sequence as set forth in SEQ ID NO:1, 2, or 29;
b) a polynucleotide encoding a polypeptide having a sequence as set forth in SEQ ID NO:3, or 30;
c) a polynucleotide having at least 90% sequence identity to a polynucleotide having a sequence as set forth in SEQ ID NO:1, 2, or 29; and
d) a polynucleotide encoding a polypeptide having at least 90% sequence identity to a polypeptide having a sequence as set forth in SEQ ID NO:3, or 30.

5. The transgenic plant of claim 3 or 4, wherein the plant is selected from the group consisting of maize, wheat, barley, sorghum, rye, triticale, rice, sugarcane, citrus trees, pineapple, coconut, banana, coffee, tea, tobacco, sunflower, pea, alfalfa, soybean, carrot, celery, tomato, potato, cotton, tobacco, eggplant, pepper, oilseed rape, canola, beet, cabbage, cauliflower, broccoli, lettuce, *Lotus sp., Medicago truncatula,* prerennial grass, ryegrass, and *Arabidopsis thaliana.*

6. A method for making a transgenic nematode-resistant plant capable of expressing a dsRNA that inhibits expression of an OPR3-like target gene in the plant, said method comprises the steps of i) preparing a nucleic acid having a region that is at least 80% identical to 20 or more contiguous nucleotides of a portion of the OPR3-like gene, wherein the nucleic acid is able to form a double-stranded transcript once expressed in the plant; ii) transforming a recipient plant with said nucleic acid; iii) producing one or more transgenic offspring of said recipient plant; and iv) selecting the offspring for expression of said transcript,
wherein the portion of the target gene is from 19 to 500 nucleotides of a polynucleotide selected from the group consisting of:
a) a polynucleotide comprising a sequence as set forth in SEQ ID NO:1, 2, or 29;
b) a polynucleotide encoding a polypeptide having a sequence as set forth in SEQ ID NO:3, or 30;
c) a polynucleotide having at least 70% sequence identity to a polynucleotide having a sequence as set forth in SEQ ID NO: 1, 2, or 29;
d) a polynucleotide encoding a polypeptide having at least 70% sequence identity to a polypeptide having a sequence as set forth in SEQ ID NO:3, or 30.

7. The method of claim 6, wherein the dsRNA comprises a multiplicity of RNA molecules each comprising a double stranded region having a length of 19 to 24 nucleotides, wherein said RNA molecules are derived from a polynucleotide selected from the group consisting of:
a) a polynucleotide comprising a sequence as set forth in SEQ ID NO:1, 2, or 29;
b) a polynucleotide encoding a polypeptide having a sequence as set forth in SEQ ID NO:3, or 30;
c) a polynucleotide having at least 90% sequence identity to a polynucleotide having a sequence as set forth in SEQ ID NO: 1, 2, or 29; and
d) a polynucleotide encoding a polypeptide having at least 90% sequence identity to a polypeptide having a sequence as set forth in SEQ ID NO:3, or 30.

8. The method of claim 6 or 7, wherein the plant is selected from the group consisting of soybean, potato, tomato, peanuts, cotton, cassava, coffee, coconut, pineapple, citrus trees, banana, corn, rape, beet, sunflower, sorghum, wheat, oats, rye, barley, rice, green bean, lima bean, pea, and tobacco.

9. The method of any one of claims 6 to 8, wherein the dsRNA is expressed in plant roots or nematode feeding sites.

10. A method for conferring nematode resistance to a plant, said method comprising the steps of:
a) preparing a nucleic acid encoding a dsRNA molecule having a region that is at least 80% identical to 20 or more contiguous nucleotides of a portion of an OPR3-like gene, wherein the nucleic acid is able to form a double-stranded transcript of a portion of a OPR3-like gene once expressed in the plant, wherein the portion of the OPR3-like gene is from a polynucleotide selected from the group consisting of:
a) a polynucleotide comprising a sequence as set forth in SEQ ID NO:1, 2, or 29;
b) a polynucleotide encoding a polypeptide having a sequence as set forth in SEQ ID NO:3, or 30;
c) a polynucleotide having at least 70% sequence identity to a polynucleotide having a sequence as set forth in SEQ ID NO:1, 2, or 29;
d) a polynucleotide encoding a polypeptide having at least 70% sequence identity to a polypeptide having a sequence as set forth in SEQ ID NO:3, or 30;
b) transforming a recipient plant with said nucleic acid;
c) producing one or more transgenic offspring of said recipient plant; and
d) selecting the offspring for nematode resistance.

11. A method for controlling the infection of a plant by a parasitic nematode, comprising the steps of transforming the plant with a nucleic acid encoding a dsRNA molecule operably linked to a root-preferred, nematode inducible or nematode feeding site-preferred promoter, whereby the dsRNA comprising one strand that is at least 80% identical to 20 or more contiguous nucleotides of a portion of a target nucleic acid essential to the formation, development or support of the feeding site, in particular the formation, development or support of a syncytia or giant cell, thereby controlling the infection of the plant by the nematode by removing or functionally incapacitating the feeding site, syncytia or giant cell, wherein the target nucleic acid is an OPR3-like gene, wherein the portion of the OPR3-like gene is from a polynucleotide selected from the group consisting of:
a) a polynucleotide comprising a sequence as set forth in SEQ ID NO:1, 2, or 29;
b) a polynucleotide encoding a polypeptide having a sequence as set forth in SEQ ID NO:3, or 30;
c) a polynucleotide having at least 70% sequence identity to a polynucleotide having a sequence as set forth in SEQ ID NO:1, 2, or 29;
d) a polynucleotide encoding a polypeptide having at least 70% sequence identity to a polypeptide having a sequence as set forth in SEQ ID NO:3, or 30.

## Patentansprüche

1. Verwendung eines dsRNA-Moleküls, das Folgendes umfasst: i) einen ersten Strang, umfassend eine Sequenz mit mindestens 80% Identität zu 20 oder mehr aufeinanderfolgenden Nukleotiden eines Abschnitts eines OPR3-like-Gens, sowie (ii) einen zweiten Strang, umfassend eine Sequenz mit Komplementarität über mindestens 80% ihrer Nukleotide zu dem ersten Strang, wobei der Abschnitt des OPR3-like-Gens von einem Polynukleotid, ausgewählt aus der folgenden Gruppe, stammt:
a) einem Polynukleotid, umfassend eine Sequenz gemäß SEQ ID NO: 1, 2 oder 29;
b) einem Polynukleotid, das für ein Polypeptid mit einer Sequenz gemäß SEQ. ID NO: 3 oder 30 codiert;
c) einem Polynukleotid mit mindestens 70% Sequenzidentität zu einem Polynukleotid mit einer Sequenz gemäß SEQ ID NO: 1, 2 oder 29;
d) einem Polynukleotid, das für ein Polypeptid mit mindestens 70% Sequenzidentität zu einem Polypeptid mit einer Sequenz gemäß SEQ ID NO: 3 oder 30 codiert;
für die Herstellung von transgenen Pflanzen mit Resistenz gegen Nematodenbefall.

2. Verwendung eines Pools von dsRNA-Molekülen, umfassend eine Mehrzahl von RNA-Molekülen, die jeweils eine doppelsträngige Region mit einer Länge von 19 bis 24 Nukleotiden umfassen, wobei die dsRNA-Moleküle von einem Abschnitt eines Polynukleotids, ausgewählt aus der folgenden Gruppe, abstammen:
a) einem Polynukleotid, umfassend eine Sequenz gemäß SEQ ID NO: 1, 2 oder 29;
b) einem Polynukleotid, das für ein Polypeptid mit einer Sequenz gemäß SEQ ID NO: 3 oder 30 codiert;
c) einem Polynukleotid mit mindestens 90% Sequenzidentität zu einem Polynukleotid mit einer Sequenz gemäß SEQ ID NO: 1, 2 oder 29; und
d) einem Polynukleotid, das für ein Polypeptid mit mindestens. 90% Sequenzidentität zu einem Polypeptid mit einer Sequenz gemäß SEQ ID NO: 3 oder 30 codiert;
für die Herstellung von transgenen Pflanzen mit Resistenz gegen Nematodenbefall.

3. Transgene nematodenresistente Pflanze, die fähig ist, eine dsRNA mit mindestens 80% Identität zu 20 oder mehr aufeinanderfolgenden Nukleotiden eines Abschnitts eines OPR3-like-Gens zu exprimieren, wobei der Abschnitt des OPR3-like-Gens von einem Polynukleotid, ausgewählt aus der folgenden Gruppe, stammt:
a) einem Polynukleotid, umfassend eine Sequenz gemäß SEQ ID NO: 1, 2 oder 29;
b) einem Polynukleotid, das für ein Polypeptid mit einer Sequenz gemäß SEQ ID NO: 3 oder 30 codiert;
c) einem Polynukleotid mit mindestens 70% Sequenzidentität zu einem Polynukleotid mit einer Sequenz gemäß SEQ ID NO: 1, 2 oder 29;
d) einem Polynukleotid, das für ein Polypeptid mit mindestens 70% Sequenzidentität zu einem Polypeptid mit einer Sequenz gemäß SEQ ID NO: 3 oder 30 codiert.

4. Transgene nematodenresistente Pflanze nach Anspruch 3, wobei die dsRNA eine Mehrzahl von RNA-Molekülen umfasst, die jeweils eine doppelsträngige Region mit einer Länge von 19 bis 24 Nukleotiden umfassen, wobei die RNA-Moleküle von einem Abschnitt eines Polynukleotids, ausgewählt aus der folgenden Gruppe, abstammen:
a) einem Polynukleotid, umfassend eine Sequenz gemäß SEQ ID NO: 1, 2 oder 29;
b) einem Polynukleotid, das für ein Polypeptid mit einer Sequenz gemäß SEQ ID NO: 3 oder 30 codiert;
c) einem Polynukleotid mit mindestens 90% Sequenzidentität zu einem Polynukleotid mit einer Sequenz gemäß SEQ ID NO: 1, 2 oder 29; und
d) einem Polynukleotid, das für ein Polypeptid mit mindestens 90% Sequenzidentität zu einem Polypeptid mit einer Sequenz gemäß SEQ ID NO: 3 oder 30 codiert.

5. Transgene Pflanze nach Anspruch 3 oder 4, wobei die Pflanze aus der Gruppe Mais, Weizen, Gerste, Sorghum, Roggen, Triticale, Reis, Zuckerrohr, Zitrusbäume, Ananas, Kokosnuss, Banane, Kaffee, Tee, Tabak, Sonnenblume, Erbse, Luzerne, Sojabohne, Karotte, Sellerie, Tomate, Kartoffel, Baumwolle, Tabak, Aubergine, Pfeffer, Raps, Canola, Rübe, Kohl, Blumenkohl, Brokkoli, Salat, *Lotus sp., Medicago truncatula*, mehrjähriges Gras, Raigras und *Arabidopsis thaliana* ausgewählt ist.

6. Verfahren zur Herstellung einer transgenen nematodenresistenten Pflanze, die fähig ist, eine dsRNA zu exprimieren, die die Expression eines OPR3-like-Zielgens in der Pflanze inhibiert, wobei das Verfahren die folgenden Schritte umfasst: i) Herstellen einer Nukleinsäure mit einer Region mit mindestens 80% Identität zu 20 oder mehr aufeinanderfolgenden Nukleotiden eines Abschnitts des OPR3-like-Gens, wobei die Nukleinsäure fähig ist, sobald sie in der Pflanze exprimiert ist, ein doppelsträngiges Transkript zu exprimiert ist, ein doppelsträngiges Transkript zu bilden; ii) Transformieren einer Empfängerpflanze mit der Nukleinsäure; iii) Herstellen von einem oder mehreren transgenen Nachkommen der Empfängerpflanze; und iv) Selektieren des/der Nachkommen auf Expression des Transkripts,
wobei der Abschnitt des Zielgens 19 bis 500 Nukleotide eines Polynukleotids, ausgewählt aus der folgenden Gruppe, beträgt:
a) einem Polynukleotid, umfassend eine Sequenz gemäß SEQ ID NO: 1, 2 oder 29;
b) einem Polynukleotid, das für ein Polypeptid mit einer Sequenz gemäß SEQ ID NO: 3 oder 30 codiert;
c) einem Polynukleotid mit mindestens 70% Sequenzidentität zu einem Polynukleotid mit einer Sequenz gemäß SEQ ID NO: 1, 2 oder 29;
d) einem Polynukleotid, das für ein Polypeptid mit mindestens 70% Sequenzidentität zu einem Polypeptid mit einer Sequenz gemäß SEQ ID NO: 3 oder 30 codiert.

7. Verfahren nach Anspruch 6, wobei die dsRNA eine Mehrzahl von RNA-Molekülen umfasst, die jeweils eine doppelsträngige Region mit einer Länge von 19 bis 24 Nukleotiden umfassen, wobei die RNA-Moleküle von einem Polynukleotid, ausgewählt aus der folgenden Gruppe, abstammen:
a) einem Polynukleotid, umfassend eine Sequenz gemäß SEQ ID NO: 1, 2 oder 29;
b) einem Polynukleotid, das für ein Polypeptid mit einer Sequenz gemäß SEQ ID NO: 3 oder 30 codiert;
c) einem Polynukleotid mit mindestens 90% Sequenzidentität zu einem Polynukleotid mit einer Sequenz gemäß SEQ ID NO: 1, 2 oder 29; und
d) einem Polynukleotid, das für ein Polypeptid mit mindestens 90% Sequenzidentität zu einem Polypeptid mit einer Sequenz gemäß SEQ ID NO: 3 oder 30 codiert.

8. Verfahren nach Anspruch 6 oder 7, wobei die Pflanze aus der Gruppe Sojabohne, Kartoffel, Tomate, Erdnüsse, Baumwolle, Maniok, Kaffee, Kokosnuss, Ananas, Zitrusbäume, Banane, Mais, Raps, Rübe, Sonnenblume, Sorghum, Weizen, Hafer, Roggen, Gerste, Reis, grüne Bohne, Limabohne, Erbse und Tabak ausgewählt ist.

9. Verfahren nach einem der Ansprüche 6 bis 8, wobei die dsRNA in Pflanzenwurzeln oder Nematodenfraßstellen exprimiert wird.

10. Verfahren zum Verleihen von Nematodenresistenz an eine Pflanze, wobei das Verfahren Folgendes umfasst:
a) Herstellen einer für ein dsRNA-Molekül codierenden Nukleinsäure mit einer Region mit mindestens 80% Identität zu 20 oder mehr aufeinanderfolgenden Nukleotiden eines Abschnitts des OPR3-like-Gens, wobei die Nukleinsäure fähig ist, sobald sie in der Pflanze exprimiert ist, ein doppelsträngiges Transkript eines Abschnitts eines OPR3-like-Gens zu bilden;
wobei der Abschnitt des OPR3-like-Gens eines Abschnitts eines OPR3-like-Gens von einem Polynukleotid, ausgewählt aus der folgenden Gruppe, stammt :
a) einem Polynukleotid, umfassend eine Sequenz gemäß SEQ ID NO: 1, 2 oder 29;
b) einem Polynukleotid, das für ein Polypeptid mit einer Sequenz gemäß SEQ ID NO: 3 oder 30 codiert;
c) einem Polynukleotid mit mindestens 70% Sequenzidentität zu einem Polynukleotid mit einer Sequenz gemäß SEQ ID NO: 1, 2 oder 29;
d) einem Polynukleotid, das für ein Polypeptid mit mindestens 70% Sequenzidentität zu einem Polypeptid mit einer Sequenz gemäß SEQ ID NO: 3 oder 30 codiert;
b) Transformieren einer Empfängerpflanze mit der Nukleinsäure;
c) Herstellen von einem oder mehreren transgenen Nachkommen der Empfängerpflanze; und
d) Selektieren des/der Nachkommen auf Nematodenresistenz.

11. Verfahren zum Bekämpfen der Infektion einer Pflanze durch einen parasitischen Nematoden, umfassend die Schritte des Transformierens der Pflanze mit einer Nukleinsäure, die für ein dsRNA-Molekül in operativer Verknüpfung mit einem Promoter mit Bevorzugung für die Wurzel, einem nematodeninduzierbaren Promoter oder einem Promoter mit Bevorzugung für Nematodenfraßstellen codiert, wobei die dsRNA einen Strang mit mindestens 80% Identität zu 20 oder mehr aufeinanderfolgenden Nukleotiden eines Abschnitts einer Zielnukleinsäure, die für die Bildung, Entwicklung oder Förderung der Fraßstelle, insbesondere die Bildung, Entwicklung oder Förderung eines Syncytiums oder einer Riesenzelle, unabdingbar ist, umfasst, wodurch die Infektion der Pflanze durch den Nematoden dadurch bekämpft wird, dass die Fraßstelle, das Syncytium oder die Riesenzelle entfernt oder funktionell ausgeschaltet wird, wobei die Zielnukleinsäure ein OPR3-like-Gen ist, wobei der Abschnitt des OPR3-like-Gens von einem Polynukleotid, ausgewählt aus der folgenden Gruppe, stammt:
a) einem Polynukleotid, umfassend eine Sequenz gemäß SEQ ID NO: 1, 2 oder 29;
b) einem Polynukleotid, das für ein Polypeptid mit einer Sequenz gemäß SEQ ID NO: 3 oder 30 codiert;
c) einem Polynukleotid mit mindestens 70% Sequenzidentität zu einem Polynukleotid mit einer Sequenz gemäß SEQ ID NO: 1, 2 oder 29;
d) einem Polynukleotid, das für ein Polypeptid mit mindestens 70% Sequenzidentität zu einem Polypeptid mit einer Sequenz gemäß SEQ ID NO: 3 oder 30 codiert.

## Revendications

1. Utilisation d'une molécule d'ARN double brin comprenant i) un premier brin comprenant une séquence ayant une identité d'au moins 80 % avec 20 nucléotides contigus, ou plus, d'une partie d'un gène de type OPR3, et ii) un deuxième brin comprenant une séquence complémentaire, sur au moins 80 % de ses nucléotides, du premier brin, la partie du gène de type OPR3 provenant d'un polynucléotide choisi dans le groupe consistant en :
a) un polynucleotide comprenant une séquence telle que présentée dans SEQ ID N° 1, 2 ou 29 ;
b) un polynucléotide codant pour un polypeptide ayant une séquence telle que présentée dans SEQ ID N° 3 ou 30 ;
c) un polynucléotide ayant une identité de séquence d'au moins 70 % avec un polynucléotide ayant une séquence telle que présentée dans SEQ ID N° 1, 2 ou 29 ;
d) un polynucléotide codant pour un polypeptide ayant une identité de séquence d'au moins 70 % avec un polypeptide ayant une séquence telle que présentée dans SEQ ID N° 3 ou 30 ;
pour la production de plantes transgéniques résistant à une infestation par des nématodes.

2. Utilisation d'un ensemble de molécules d'ARN double brin comprenant une multiplicité de molécules d'ARN, chacune comprenant une région double brin ayant une longueur de 19 à 24 nucléotides, lesdites molécules d'ARN double brin dérivant d'une partie d'un polynucléotide choisi dans le groupe consistant en :
a) un polynucléotide comprenant une séquence telle que présentée dans SEQ ID N° 1, 2 ou 29 ;
b) un polynucléotide codant pour un polypeptide ayant une séquence telle que présentée dans SEQ ID N° 3 ou 30 ;
c) un polynucléotide ayant une identité de séquence d'au moins 90 % avec un polynucléotide ayant une séquence telle que présentée dans SEQ ID N° 1, 2 ou 29 ;
d) un polynucléotide codant pour un polypeptide ayant une identité de séquence d'au moins 90 % avec un polypeptide ayant une séquence telle que présentée dans SEQ ID N° 3 ou 30 ;
pour la production de plantes transgéniques résistant à une infestation par les nématodes.

3. Plante transgénique résistante aux nématodes, capable d'exprimer un ARN double brin qui a une identité d'au moins 80 % avec 20 nucléotides contigus, ou plus, d'une partie d'un gène de type OPR3, la partie du gène de type OPR3 provenant d'un polynucléotide choisi dans le groupe consistant en :
a) un polynucléotide comprenant une séquence telle que présentée dans SEQ ID N° 1, 2 ou 29 ;
b) un polynucléotide codant pour un polypeptide ayant une séquence telle que présentée dans SEQ ID N° 3 ou 30 ;
c) un polynucléotide ayant une identité de séquence d'au moins 70 % avec un polynucléotide ayant une séquence telle que présentée dans SEQ ID N° 1, 2 ou 29 ;
d) un polynucléotide codant pour un polypeptide ayant une identité de séquence d'au moins 70 % avec un polypeptide ayant une séquence telle que présentée dans SEQ ID N° 3 ou 30.

4. Plante transgénique résistante aux nématodes de la revendication 3, dans laquelle l'ARN double brin comprend une multiplicité de molécules d'ARN, chacune comprenant une région double brin ayant une longueur de 19 à 24 nucléotides, lesdites molécules d'ARN dérivant d'une partie d'un polynucléotide choisi dans le groupe consistant en :
a) un polynucléotide comprenant une séquence telle que présentée dans SEQ ID N° 1, 2 ou 29 ;
b) un polynucléotide codant pour un polypeptide ayant une séquence telle que présentée dans SEQ ID N° 3 ou 30 ;
c) un polynucléotide ayant une identité de séquence d'au moins 90 % avec un polynucléotide ayant une séquence telle que présentée dans SEQ ID N° 1, 2 ou 29 ;
d) un polynucléotide codant pour un polypeptide ayant une identité de séquence d'au moins 90 % avec un polypeptide ayant une séquence telle que présentée dans SEQ ID N° 3 ou 30.

5. Plante transgénique de la revendication 3 ou 4, la plante étant choisie dans le groupe consistant en le maïs, le blé, l'orge, le sorgho, le seigle, le triticale, le riz, la canne a sucre, les agrumes, l'ananas, la noix de coco, la banane, le café, le thé, le tabac, le tournesol, le pois, la luzerne, le soja, la carotte, le céleri, la tomate, la pomme de terre, le coton, le tabac, l'aubergine, le poivre, le colza oléagineux, le canola, la betterave, le chou, le chou-fleur, le brocoli, la laitue, Lotus sp., *Medicago* truncatula, les graminées vivaces, le raygrass et *Arabidopsis thaliana*.

6. Procédé de fabrication d'une plante transgénique résistante aux nématodes, capable d'exprimer un ARN double brin qui inhibe l'expression d'un gène cible de type OPR3 dans la plante, ledit procédé comprenant les étapes de i) préparation d'un acide nucléique ayant une région ayant une identité d'au moins 80 % avec 20 nucléotides contigus, ou plus, d'une partie du gène de type OPR3, l'acide nucléique étant capable de former un transcrit double brin, une fois qu'il a été exprimé dans la plante ; ii) transformation d'une plante receveuse avec ledit acide nucléique ; iii) production d'un ou plusieurs descendants transgéniques de ladite plante receveuse ; et iv) sélection du descendant pour expression dudit transcrit,
dans lequel la partie du gène cible comprend 19 à 500 nucléotides d'un polypeptide choisi dans le groupe consistant en :
a) un polynucléotide comprenant une séquence telle que présentée dans SEQ ID N° 1, 2 ou 29 ;
b) un polynucléotide codant pour un polypeptide ayant une séquence telle que présentée dans SEQ ID N° 3 ou 30 ;
c) un polynucléotide ayant une identité de séquence d'au moins 70 % avec un polynucléotide ayant une séquence telle que présentée dans SEQ ID N° 1, 2 ou 29 ;
d) un polynucléotide codant pour un polypeptide ayant une identité de séquence d'au moins 70 % avec un polypeptide ayant une séquence telle que présentée dans SEQ ID N° 3 ou 30.

7. Procédé de la revendication 6, dans lequel l'ARN double brin comprend une multiplicité de molécules d'ARN, chacune comprenant une région double brin ayant une longueur de 19 à 24 nucléotides, lesdites molécules d'ARN dérivant d'un polynucléotide choisi dans le groupe consistant en :
a) un polynucléotide comprenant une séquence telle que présentée dans SEQ ID N° 1, 2 ou 29 ;
b) un polynucléotide codant pour un polypeptide ayant une séquence telle que présentée dans SEQ ID N° 3 ou 30 ;
c) un polynucléotide ayant une identité de séquence d'au moins 90 % avec un polynucléotide ayant une séquence telle que présentée dans SEQ ID N° 1, 2 ou 29 ;
d) un polynucléotide codant pour un polypeptide ayant une identité de séquence d'au moins 90 % avec un polypeptide ayant une séquence telle que présentée dans SEQ ID N° 3 ou 30.

8. Procédé de la revendication 6 ou 7, dans lequel la plante est choisie dans le groupe consistant en le soja, la pomme de terre, la tomate, l'arachide, le coton, le manioc, le café, la noix de coco, l'ananas, les agrumes, la banane, le maïs, le colza, la betterave, le tournesol, le sorgho, le blé, l'avoine, le seigle, l'orge, le riz, le haricot vert, le haricot de Lima, le pois et le tabac.

9. Procédé de l'une quelconque des revendications 6 à 8, dans lequel l'ARN double brin est exprimé dans les racines d'une plante ou dans les sites d'alimentation des nématodes.

10. Procédé pour conférer à une plante une résistance aux nématodes, ledit procédé comprenant les étapes de :
a) préparation d'un acide nucléique codant pour une molécule d'ARN double brin ayant une région ayant une identité d'au moins 80 % avec 20 nucléotides contigus, ou plus, d'une partie d'un gène de type OPR3, l'acide nucléique étant capable de former un transcrit double brin d'une partie d'un gène de type OPR3, une fois qu'il a été exprimé dans la plante, la partie du gène de type OPR3 provenant d'un polynucléotide choisi dans le groupe consistant en :
a) un polynucléotide comprenant une séquence telle que présentée dans SEQ ID N° 1, 2 ou 29 ;
b) un polynucléotide codant pour un polypeptide ayant une séquence telle que présentée dans SEQ ID N° 3 ou 30 ;
c) un polynucléotide ayant une identité de séquence d'au moins 70 % avec un polynucléotide ayant une séquence telle que présentée dans SEQ ID N° 1, 2 ou 29 ;
d) un polynucléotide codant pour un polypeptide ayant une identité de séquence d'au moins 70 % avec un polypeptide ayant une séquence telle que présentée dans SEQ ID N° 3 ou 30 ;
b) transformation d'une plante receveuse avec ledit acide nucléique ;
c) production d'un ou plusieurs descendants transgéniques de ladite plante receveuse ; et
d) sélection du descendant pour ce qui est de la résistance aux nématodes.

11. Procédé pour maîtriser l'infection d'une plante par un nématode parasite, comprenant les étapes de transformation de la plante avec un acide nucléique codant pour une molécule d'ARN double brin liée d'une manière opérationnelle à un promoteur à préférence de site, à préférence de racine, inductible par les nématodes ou pour l'alimentation des nématodes, l'ARN double brin comprenant un brin qui a une identité d'au moins 80 % avec 20 nucléotides contigus, ou plus, d'une partie d'un acide nucléique cible essentiel à la formation, au développement et au soutien du site d'alimentation, en particulier à la formation, au développement ou au soutien d'un syncytium ou d'une cellule géante, de façon à maîtriser l'infection d'une plante par le nématode, par élimination ou incapacitation fonctionnelle du site d'alimentation, du syncytium ou de la cellule géante, l'acide nucléique cible étant un gène de type OPR3, la partie du gène de type OPR3 provenant d'un polynucléotide choisi dans le groupe consistant en :
a) un polynucléotide comprenant une séquence telle que présentée dans SEQ ID N° 1, 2 ou 29 ;
b) un polynucléotide codant pour un polypeptide ayant une séquence telle que présentée dans SEQ ID N° 3 ou 30 ;
c) un polynucléotide ayant une identité de séquence d'au moins 70 % avec un polynucléotide ayant une séquence telle que présentée dans SEQ ID N° 1, 2 ou 29 ;
d) un polynucléotide codant pour un polypeptide ayant une identité de séquence d'au moins 70 % avec un polypeptide ayant une séquence telle que présentée dans SEQ ID N° 3 ou 30.


### SEQUENCE LISTING

<110> BASF Plant Science GmbH
   Wiig, Aaron
<120> Compositions and Methods Using RNA Interference of OPR3-Like
<130> PF 58862
<160> 30
<170> PatentIn version 3.4
<210> 1
   <211> 1410
   <212> DNA
   <213> Glycine max
<400> 1
   cagataactc aattagctta ttttctccat acaacaagat gggcaaattc aacctctctc     60
   atagggtggt attggctccc atgaccagat gcagagcgct caatgggact ccactggcag    120
   cacatgctga atactacgct cagagatcaa caccgggtgg atttctcatc actgaaggca    180
   ccttgatctc tccaacttct tctgggtttc ctcatgttcc tggaatatac tcagatgaac    240
   aggtagaggc atggagaaat gtagtggacg ccgtgcatgc caacggcagc tttatcttct    300
   gtcaactctg gcatgttggc cgtgcatcac atccagtgta tcagcctggt ggggctctac    360
   cctcttcgtc caccagcaaa cccatatcag acaagtggaa aattctcatg cccgatggct    420
   cccatggcat ctatccagag cctcgtgcac ttaccacttc tgagatatct gaaatagtgc    480
   atcattatcg ccaagcagct attaatgcaa ttcgagcagg ttttgatgga atcgagattc    540
   atggagcaca tgggtatctc attgatcaat tcttaaagga tgcaatcaat gatagaacag    600
   atgaatacgg tggaccacta gaaaaccggt gcaggttctt aatggaggta gttgaagctg    660
   ttgtctctgc cattggagcg gaaagagttg ctatcagaat ttcaccagca attgatttca    720
   atgacgcctt tgactctgac ccacttgggc taggcttagc agtgattgaa agactcaaca    780
   atttgcagaa acaagtgggc acaaaactcg cttatcttca tgttactcag cctcgattca    840
   cacttttggc gcaaaccgag tcagtgagtg aaaaggagga agctcatttc atgcagaaat    900
   ggagagaggc ttatgaggga acattcatgt gtagtggagc ttttactagg gactcaggaa    960
   tggaagctgt agctgaaggc catgctgatt tggtatccta tggtcgtctt ttcatctcca   1020
   atccagactt ggttttaagg cttaagctca atgcacctct taccaagtat aacaggaaca   1080
   cattttacac ccaagatcct gttataggct acacagatta tcctttcttt aatggaacaa   1140
   ctgagacaaa attaagtaac tagctaaggc catgcatgcc ctttaatttt aatctccata   1200
   tggctttttg aataataatg ttcataacat tcaaaactct tcagttgagt ttatcctcag   1260
   acaaacaaat taagtggttc attcacttgt tagggtattt agatcttagg ttaattagtc   1320
   tccggcattt tgatttcatt tcaatttgta ttcagtcttt cattttgaat aaaataatat   1380
   taagtttttt gccttaaaaa aaaaaaaaaa                                    1410
<210> 2
   <211> 229
   <212> DNA
   <213> Glycine max
<400> 2
   atctcattga tcaattctta aaggatgcaa tcaatgatag aacagatgaa tacggtggac     60
   cactagaaaa ccggtgcagg ttcttaatgg aggtagttga agctgttgtc tctgccattg    120
   gagcggaaag agttgctatc agaatttcac cagcaattga tttcaatgac gcctttgact    180
   ctgacccact tgggctaggc ttagcagtga ttgaaagact caacaattt                229
<210> 3
   <211> 386
   <212> PRT
   <213> Glycine max
<400> 3
   Asp Asn Ser Ile Ser Leu Phe Ser Pro Tyr Asn Lys Met Gly Lys Phe
   1               5                   10                  15
   Asn Leu Ser His Arg Val Val Leu Ala Pro Met Thr Arg Cys Arg Ala
               20                  25                  30
   Leu Asn Gly Thr Pro Leu Ala Ala His Ala Glu Tyr Tyr Ala Gln Arg
           35                  40                  45
   Ser Thr Pro Gly Gly Phe Leu Ile Thr Glu Gly Thr Leu Ile Ser Pro
       50                  55                  60
   Thr Ser Ser Gly Phe Pro His Val Pro Gly Ile Tyr Ser Asp Glu Gln
   65                  70                  75                  80
   Val Glu Ala Trp Arg Asn Val Val Asp Ala Val His Ala Asn Gly Ser
                   85                  90                  95
   Phe Ile Phe Cys Gln Leu Trp His Val Gly Arg Ala Ser His Pro Val
               100                 105                 110
   Tyr Gln Pro Gly Gly Ala Leu Pro Ser Ser Ser Thr Ser Lys Pro Ile
           115                 120                 125
   Ser Asp Lys Trp Lys Ile Leu Met Pro Asp Gly Ser His Gly Ile Tyr
       130                 135                 140
   Pro Glu Pro Arg Ala Leu Thr Thr Ser Glu Ile Ser Glu Ile Val His
   145                 150                 155                 160
   His Tyr Arg Gln Ala Ala Ile Asn Ala Ile Arg Ala Gly Phe Asp Gly
                   165                 170                 175
   Ile Glu Ile His Gly Ala His Gly Tyr Leu Ile Asp Gln Phe Leu Lys
               180                 185                 190
   Asp Ala Ile Asn Asp Arg Thr Asp Glu Tyr Gly Gly Pro Leu Glu Asn
   195 200 205
   Arg Cys Arg Phe Leu Met Glu Val Val Glu Ala Val Val Ser Ala Ile
   210 215 220
   Gly Ala Glu Arg Val Ala Ile Arg Ile Ser Pro Ala Ile Asp Phe Asn
   225 230 235 240
   Asp Ala Phe Asp Ser Asp Pro Leu Gly Leu Gly Leu Ala Val Ile Glu
   245 250 255
   Arg Leu Asn Asn Leu Gln Lys Gln Val Gly Thr Lys Leu Ala Tyr Leu
   260 265 270
   His Val Thr Gln Pro Arg Phe Thr Leu Leu Ala Gln Thr Glu Ser Val
   275 280 285
   Ser Glu Lys Glu Glu Ala His Phe Met Gln Lys Trp Arg Glu Ala Tyr
   290 295 300
   Glu Gly Thr Phe Met Cys Ser Gly Ala Phe Thr Arg Asp Ser Gly Met
   305 310 315 320
   Glu Ala Val Ala Glu Gly His Ala Asp Leu Val Ser Tyr Gly Arg Leu
   325 330 335
   Phe Ile Ser Asn Pro Asp Leu Val Leu Arg Leu Lys Leu Asn Ala Pro
   340 345 350
   Leu Thr Lys Tyr Asn Arg Asn Thr Phe Tyr Thr Gln Asp Pro Val Ile
   355 360 365
   Gly Tyr Thr Asp Tyr Pro Phe Phe Asn Gly Thr Thr Glu Thr Lys Leu
   370 375 380
   Ser Asn
   385
<210> 4
   <211> 1999
   <212> DNA
   <213> Arabidopsis thaliana
<400> 4
   gtagtgccct tcatggatac caaaagagaa aatttgattt agtgcataca tataacaata     60
   taacgccgca taataatact gtataaaaca gtcatgtaac gatatgacag cagtaataca    120
   gttccaagag acgttataat cgtatgcaat catatgcttg cgtagatttt ccaacagttt    180
   tgtttcgttg ataggaggaa ctcaacactc tagggtagtg attggtagac actattagca    240
   caaaaaatat taattttact ctgatgttta ccaaaaaagt taccaatcaa atatttaaga    300
   gatcgtactc ttccacggcg actctaaaaa ccaaagatat aggttagact cataactact    360
   ttataaagaa aatgtttaac gataactacc gagatctaat aaataaacct tcattttcaa    420
   gtatattata tttgcttctt ttgtttatat atcaaaccaa gttctggttt ataaaaatat    480
   tagataaaac tcgtctaaat aggtaggtgt aaaataaaat tttaaatttt tatcgataat    540
   atttaaaatt tgaaaagtta ataatgatcc acacattttt tctaatattt aatttagtaa    600
   tttttgtatt aaataaaatt tcaatcatat acattcgatt tttctataca ttttaactat    660
   ctatttctgc ataataaact gtattttcat tttatacgct tcatcttatg gatgatattt    720
   aaattttaaa tagtaattca tacacttttt aatatttaat ttagtatttt cttaaatcca    780
   aattttaatc ttacaattta aatatctact ttaacataat acaaatacaa tttaatttca    840
   ttgtattaaa ttcaaatata atttgattat aataaaatac aatttaattc taaaaagtcc    900
   atcttagatt ttaattttcc tttttagttt tgaaaattaa aaatttaaat ttattagata    960
   tatatgttac tttttcagtt ttcctattta tttaagaaaa aaatattttt taacacatgt   1020
   caacttgtaa acaatagact gaacacgtca ttttatatta tgtttagttt tgaaaattaa   1080
   agttaattaa atatttatat ttcttttttt tagcttttct aattattttt aaaatagtaa   1140
   atatttttaa tacaaatcaa tatctgaaca atagatttga tacataacat aatcctataa   1200
   attattaact tggaaaacga tagtttatat aataaaatta ttttcttaag ttctctaacc   1260
   ataacaatta aactatattt tagcgaagaa aagaagagaa taccgagaga acgcaacttg   1320
   cactaaaagc taccactttg gcaaatcact catttatatt attatatact atcacctcaa   1380
   ttcaatcgaa acctcaaaat aacactaata tatacacaaa gaaacaacag aataacaccg   1440
   aagaatatag gtttaggaaa atccagaatt tgttgagact aaagagatca aattttcgat   1500
   acaaggtttt gctcaatttg tattttcata ataaaattct ttatttcacc atagacttac   1560
   atgattagtt tttcttttaa taaaaaaaaa cacgcgacat gaaaattata ttatctcagt   1620
   gttgtcgaat ttgaatttga attttgagtt aaatactaca catttgttga caacttatta   1680
   aactttacaa gtctgctaca aatattgtca aatatttact aattaatgga ccaaaatcct   1740
   ctaacttgca aatttgtatc tacatcaact taaaaattag gaatatgcga cccaaaaaaa   1800
   aaaaaactag gaataataat aaaaaaatgg aatgatgtgg aggaagctct ttactctttg   1860
   agaggaagtt tataaattga ccacacattt agtctattat catcacatgt attaagactt   1920
   gacaacttgt ctttctcaca ccaaacccct ctcctctgtt tcataacatc tgctctttct   1980
   tttttttcct aagccccta                                                 1999
<210> 5
   <211> 1476
   <212> DNA
   <213> Arabidopsis thaliana
<400> 5
   gctcgcgtta gttccactca aggagtatcc tttcttcctt gcgcaactct ccaccttcgg      60
   gtaaagtacc atctctagca tcttgagtct tgatcaactt ctgttttgct tactctcaaa     120
   atgcattaat ttttttttat actagatcat agtattatat ctcttaatct acctattgaa     180
   atctacttaa tgtttttact aaaacctacg tgtttctctt tagagaattt tgtgctatgc     240
   atgaattaga ggttagtaat gtgtaatact tcataagtct agatttattt gttggttaac     300
   acgtttagta attcacacac acacaccacc ttagatattt tactgtgaat tagaaaaaga     360
   tacatagtta ggagtgtttt tttaaaaaaa ttcaatcatg agaaaattag aggtgtgatg     420
   ttatacatta tgaaaatgca aagggcagat acgaataaat tagaaacttg tttaacgggt     480
   cagagttggc ttctagtctc tttcgacttg gatacttctt cttctacaat tgggacatta     540
   ttgtaggcgc attatatcat ttctctacat gcaatgaatg tacatacatt aattcacatt     600
   tatttttgga ataatcatat gagtgatcga agtttgtatt tatatattca atcttcacaa     660
   actactttta tttaaaaatc atttgcaaaa tgctatttta ttgacaaaaa gatatatgct     720
   ataaaataaa ataaaattca caaactatag tcattaatac aaaaagaaat cattgaatat     780
   ggtagagggg aaacaaaaaa aaaacacgac gatgtaagtt ggtggaacca cattatcaaa     840
   ataaaagaag gtggtggaac caaattgaat aaagtccgtc catatcatta tccgtccctt     900
   aggagcctct aattagtaat attcttatgg gtccactgtg gcttagagga cttgattaaa     960
   accattctta tttagtgcta actttgtgag ggttggaata acgaaccaag ctgattcaaa    1020
   ccattccaaa acaaagttgt cacatatttc aaaaccaaag tttaccggac agagaaatat    1080
   ggtgtgtttt tctcaaacca agctaaatgg aatccattgt aaaccaaaat gttcacacct    1140
   acctattctt ttggagtccc ttttccatgt gtttgctgtc tgctagtcaa gtttcattag    1200
   ctgattgcct tgcatcatat tcttggatca actttttttt tttttttttt tggggtaatt    1260
   aacaaaatgc ttaaatttct caagactata ggatcacatt acctgtgtgc ttaacataac    1320
   ttttagatag gctagagaat tgatctatta caagataatc aataatttac agaagaaaac    1380
   attctttttt ttgttctatt tccttcatgt aggtatgtag ctgtatatta tactatcttg    1440
   tattttcgat atcgtgctgg aactgtcaca gatgca                              1476
<210> 6
   <211> 609
   <212> DNA
   <213> Glycine max
<400> 6
   gaagccacgt catgaagagt atatcatttc agtaatgttt tgagacgcct ctataatgct 60
   ttaccaacaa aacaaaacaa aaaaaagaac atttgaaacc atttgtatta aaaaaaaaaa 120
   ggtatattag gccataatat tataggtaac atgaaatatc aaatgacacg caagagtttt 180
   gtcaaaaatg aaaccatcac acatcagaga ttatggcaaa taatgttttg tgtgtctctt 240
   gcttcaccca taacataagc ctctataact ggagagaaga aaaaaaaaag tggaggggct 300
   agggtgggaa tttggaagaa tacagttata ttgagcattg agcaagttga tagaaagctt 360
   ctcaatttgt acaaaatttg catccacatg attattaaag acgtagacag cacttcttcc 420
   ttcttttttt ctataagttt cttatatatt gttcttcatg ttttaatatt attactttat 480
   gtacgcgtct aacagtagtc ctcccaaact gctataaata gagcctcttc aacgcacctc 540
   ttggcagtac aaaaattatt catctcttct aagttctaat tttctaagca ttcagtaaaa 600
   gaactaacc 609
<210> 7
   <211> 1191
   <212> DNA
   <213> Lycopersicon esculentum
<400> 7
   atggcgtctt cagctcaaga tggaaacaat ccccttttct ctccttacaa gatgggcaag 60
   ttcaatctat cccacagggt agtattggct ccgatgacaa ggtgcagagc actgaataat 120
   attccacagg cggcgctagg ggagtattac gagcagagag cgacggccgg tggatttctg 180
   atcactgaag gcactatgat ttctccgact tcagctgggt ttcctcatgt gccagggatt 240
   ttcacaaagg aacaagtaag ggaatggaag aaaatagttg atgtagtgca tgcaaagggt 300
   gctgtcatat tttgtcagct gtggcatgtt ggtcgtgcat ctcatgaagt gtatcaacct 360
   gctggagctg caccaatatc atccactgag aagcctatat caaataggtg gagaattcta 420
   atgcctgatg gaactcatgg gatttatcca aaaccaagag caattggaac ctatgagatc 480
   tcacaagttg ttgaagatta tcgcaggtcg gccttgaatg ctattgaagc aggtttcgat 540
   ggtattgaaa tccatggagc tcacggttac ttgattgatc aattcttgaa agatgggatc 600
   aatgaccgga cagatgagta tggtggatca ctagccaacc ggtgcaaatt catcacacag 660
   gtggttcaag cagtagtctc agcaatagga gctgatcgcg taggcgttag agtttcacca 720
   gcaatagatc atcttgatgc catggactct aatccactca gccttggctt agcagttgtt 780
   gaaagactaa acaaaatcca actccattct ggttccaagc ttgcctatct tcatgtaaca 840
   cagccacgat acgtagcata tgggcaaact gaagcaggca gacttggcag tgaagaggaa 900
   gaggctcgtt taatgaggac tttgaggaac gcgtatcagg ggacattcat ttgcagtggt 960
   ggatacacta gggaactagg aattgaggct gtggcacaag gtgatgctga tctcgtgtca 1020
   tatggtcgtc ttttcatctc taatcctgat ttggttatga gaatcaagct aaatgcacct 1080
   ctaaataagt ataacaggaa gacattctat actcaagatc cagttgtggg atacacagat 1140
   taccctttcc ttcaaggaaa tggaagcaat ggaccgttat cgcgtctgtg a 1191
<210> 8
   <211> 396
   <212> PRT
   <213> Lycopersicon esculentum
<400> 8
   Met Ala Ser Ser Ala Gln Asp Gly Asn Asn Pro Leu Phe Ser Pro Tyr
   1 5 10 15
   Lys Met Gly Lys Phe Asn Leu Ser His Arg Val Val Leu Ala Pro Met
   20 25 30
   Thr Arg Cys Arg Ala Leu Asn Asn Ile Pro Gln Ala Ala Leu Gly Glu
   35 40 45
   Tyr Tyr Glu Gln Arg Ala Thr Ala Gly Gly Phe Leu Ile Thr Glu Gly
   50 55 60
   Thr Met Ile Ser Pro Thr Ser Ala Gly Phe Pro His Val Pro Gly Ile
   65 70 75 80
   Phe Thr Lys Glu Gln Val Arg Glu Trp Lys Lys Ile Val Asp Val Val
   85 90 95
   His Ala Lys Gly Ala Val Ile Phe Cys Gln Leu Trp His Val Gly Arg
   100 105 110
   Ala Ser His Glu Val Tyr Gln Pro Ala Gly Ala Ala Pro Ile Ser Ser
   115 120 125
   Thr Glu Lys Pro Ile Ser Asn Arg Trp Arg Ile Leu Met Pro Asp Gly
   130 135 140
   Thr His Gly Ile Tyr Pro Lys Pro Arg Ala Ile Gly Thr Tyr Glu Ile
   145 150 155 160
   Ser Gln Val Val Glu Asp Tyr Arg Arg Ser Ala Leu Asn Ala Ile Glu
   165 170 175
   Ala Gly Phe Asp Gly Ile Glu Ile His Gly Ala His Gly Tyr Leu Ile
   180 185 190
   Asp Gln Phe Leu Lys Asp Gly Ile Asn Asp Arg Thr Asp Glu Tyr Gly
   195 200 205
   Gly Ser Leu Ala Asn Arg Cys Lys Phe Ile Thr Gln Val Val Gln Ala
   210 215 220
   Val Val Ser Ala Ile Gly Ala Asp Arg Val Gly Val Arg Val Ser Pro
   225 230 235 240
   Ala Ile Asp His Leu Asp Ala Met Asp Ser Asn Pro Leu Ser Leu Gly
   245 250 255
   Leu Ala Val Val Glu Arg Leu Asn Lys Ile Gln Leu His Ser Gly Ser
   260 265 270
   Lys Leu Ala Tyr Leu His Val Thr Gln Pro Arg Tyr Val Ala Tyr Gly
   275 280 285
   Gln Thr Glu Ala Gly Arg Leu Gly Ser Glu Glu Glu Glu Ala Arg Leu
   290 295 300
   Met Arg Thr Leu Arg Asn Ala Tyr Gln Gly Thr Phe Ile Cys Ser Gly
   305 310 315 320
   Gly Tyr Thr Arg Glu Leu Gly Ile Glu Ala Val Ala Gln Gly Asp Ala
   325 330 335
   Asp Leu Val Ser Tyr Gly Arg Leu Phe Ile Ser Asn Pro Asp Leu Val
   340 345 350
   Met Arg Ile Lys Leu Asn Ala Pro Leu Asn Lys Tyr Asn Arg Lys Thr
   355 360 365
   Phe Tyr Thr Gln Asp Pro Val Val Gly Tyr Thr Asp Tyr Pro Phe Leu
   370 375 380
   Gln Gly Asn Gly Ser Asn Gly Pro Leu Ser Arg Leu
   385 390 395
<210> 9
   <211> 1176
   <212> DNA
   <213> Arabidopsis thaliana
<400> 9
   atgacggcgg cacaagggaa ctctaacgag actctgtttt cttcttacaa gatgggaaga 60
   ttcgatctct ctcatcgagt ggttctggcg ccgatgacgc ggtgcagggc gttgaacgga 120
   gtaccaaacg cggcgttggc agagtattat gctcaacgga ccactcccgg cggttttctc 180
   atctccgaag gcaccatggt ctctcccgga tccgcagggt tcccacatgt gcctggaatc 240
   tattcagatg aacaagtaga agcatggaag caagttgtgg aagcagttca cgctaaggga 300
   ggtttcatct tttgtcaatt atggcatgtt ggacgtgctt ctcatgcagt gtatcaacct 360
   aatggaggat caccaatatc gtcaacgaac aaaccaatct cggaaaacag gtggcgagtt 420
   ttgttgcccg atggttccca cgtgaagtac ccgaaacctc gggctttaga agcttccgag 480
   atacctcggg tggtggagga ttattgcctt tctgctttga atgcgattcg agctggtttc 540
   gatgggattg agatccacgg ggcgcatggt taccttattg atcagttttt gaaagatggg 600
   atcaatgacc gtactgacca atacggagga tccattgcaa accgttgtag attcttgaaa 660
   caagtagtgg aaggtgtagt ttcagccata ggagctagta aagttggtgt gagggtatct 720
   ccggctatag atcacttgga cgcaactgat tctgacccac tatcactcgg gctagccgtg 780
   gttggcatgc tcaataagtt acaaggtgtt aatggctcaa agctcgctta ccttcacgtt 840
   acacaacctc gctaccacgc ctacgggcaa acagagtctg gaaggcaagg gagtgatgag 900
   gaagaagcta agctaatgaa gagcttgagg atggcttata atggaacctt tatgtctagt 960
   ggaggattca ataaggaact aggtatgcaa gctgttcaac aaggtgatgc tgatttggtt 1020
   tcatatggca gactgtttat cgcaaacccg gatttggttt cgcggttcaa gattgatgga 1080
   gagttgaata aatataatcg gaagacgttt tacactcaag atccagttgt tggctacacg 1140
   gattatcctt tcttggctcc tttttcccgc ctctga 1176
<210> 10
   <211> 391
   <212> PRT
   <213> Arabidopsis thaliana
<400> 10
   Met Thr Ala Ala Gln Gly Asn Ser Asn Glu Thr Leu Phe Ser Ser Tyr
   1               5                   10                  15
   Lys Met Gly Arg Phe Asp Leu Ser His Arg Val Val Leu Ala Pro Met
               20                  25                  30
   Thr Arg Cys Arg Ala Leu Asn Gly Val Pro Asn Ala Ala Leu Ala Glu
           35                  40                  45
   Tyr Tyr Ala Gln Arg Thr Thr Pro Gly Gly Phe Leu Ile Ser Glu Gly
       50                  55                  60
   Thr Met Val Ser Pro Gly Ser Ala Gly Phe Pro His Val Pro Gly Ile
   65                  70                  75                  80
   Tyr Ser Asp Glu Gln Val Glu Ala Trp Lys Gln Val Val Glu Ala Val
                   85                  90                  95
   His Ala Lys Gly Gly Phe Ile Phe Cys Gln Leu Trp His Val Gly Arg
               100                 105                 110
   Ala Ser His Ala Val Tyr Gln Pro Asn Gly Gly Ser Pro Ile Ser Ser
           115                 120                 125
   Thr Asn Lys Pro Ile Ser Glu Asn Arg Trp Arg Val Leu Leu Pro Asp
       130                 135                 140
   Gly Ser His Val Lys Tyr Pro Lys Pro Arg Ala Leu Glu Ala Ser Glu
   145                 150                 155                 160
   Ile Pro Arg Val Val Glu Asp Tyr Cys Leu Ser Ala Leu Asn Ala Ile
                   165                 170                 175
   Arg Ala Gly Phe Asp Gly Ile Glu Ile His Gly Ala His Gly Tyr Leu
               180                 185                 190
   Ile Asp Gln Phe Leu Lys Asp Gly Ile Asn Asp Arg Thr Asp Gln Tyr
           195                 200                 205
   Gly Gly Ser Ile Ala Asn Arg Cys Arg Phe Leu Lys Gln Val Val Glu
       210                 215                 220
   Gly Val Val Ser Ala Ile Gly Ala Ser Lys Val Gly Val Arg Val Ser
   225                 230                 235                 240
   Pro Ala Ile Asp His Leu Asp Ala Thr Asp Ser Asp Pro Leu Ser Leu
                   245                 250                 255
   Gly Leu Ala Val Val Gly Met Leu Asn Lys Leu Gln Gly Val Asn Gly
               260                 265                 270
   Ser Lys Leu Ala Tyr Leu His Val Thr Gln Pro Arg Tyr His Ala Tyr
           275                 280                 285
   Gly Gln Thr Glu Ser Gly Arg Gln Gly Ser Asp Glu Glu Glu Ala Lys
       290                 295                 300
   Leu Met Lys Ser Leu Arg Met Ala Tyr Asn Gly Thr Phe Met Ser Ser
   305                 310                 315                 320
   Gly Gly Phe Asn Lys Glu Leu Gly Met Gln Ala Val Gln Gln Gly Asp
                   325                 330                 335
   Ala Asp Leu Val Ser Tyr Gly Arg Leu Phe Ile Ala Asn Pro Asp Leu
               340                 345                 350
   Val Ser Arg Phe Lys Ile Asp Gly Glu Leu Asn Lys Tyr Asn Arg Lys
           355                 360                 365
   Thr Phe Tyr Thr Gln Asp Pro Val Val Gly Tyr Thr Asp Tyr Pro Phe
       370                 375                 380
   Leu Ala Pro Phe Ser Arg Leu
   385                 390
<210> 11
   <211> 1200
   <212> DNA
   <213> Hevea brasiliensis
<400> 11
   atggctgaaa ctggaacaga agggaccggg atcaccaccc tattttctcc ttacaaaatg 60
   ggcaagttca gcctctccca cagggtggtg ctggctccca tgactagatg cagagcgttg 120
   aacgggatac caaacgcagc gctggtggat tactacacgc agagatcaac tcccggcgga 180
   tttctcatca cggaaggcac tctggtttcc cctactgccc ctgggtttcc tcatgtccct 240
   ggaatttata cagaagaaca agcggaggca tggaagaggg tggtggatgc agttcatgcc 300
   aaagggagca tcatattctg tcaattatgg catgttggcc gcgcatctca tcaggtttat 360
   caacctaatg gagctgcacc aatatcatcg acgggcaagg ccatctcaaa cagatggaga 420
   attctcatgc cagatggatc atatgggaaa tacccaacac ctaggccctt ggaaacacct 480
   gaaatactag aggtagtgaa gaattatcgc cagtcagcct tgaatgccat tcgagcaggc 540
   tttgatggaa ttgaggtcca cggggctcat ggttacctta ttgatcaatt cttaaaagac 600
   gggatcaatg accgaacaga tgagtatggt ggatcaatca acaatcgatg cagattccta 660
   atgcaggtga ttcaggcagt agttgcagct attggtgctg atcgagttgg tttcagaatg 720
   tcaccggcaa ttgatcacct agatgccata gattctgatc cgctcaactt gggtcttgct 780
   gtaatcgaga gacttaacaa acttcagttg aaccttggat caaaactcac ttatctccat 840
   gtcactcagc ctcgctacac agcttatggc caaacagaat caggcagaca tggtactgaa 900
   gaagaggaag ctagattaat gagaacttgg agaagggctt ataagggaac tttcatctgt 960
   agcggtgggt tcacgaggga gctaggaatg gaagctatag ctcaagatga tgcagatttg 1020
   gtatcttatg gccgactttt tatttcaaac ccagacttag tcttgagatt taagctcaat 1080
   gcgcccttga ataagtatgt caggaaaaca ttctacaccc aagatcctgt tgttgggtac 1140
   acagactacc catttttcag aaaagtagac gggagccagg agccacgatc acgcctttga 1200
<210> 12
   <211> 399
   <212> PRT
   <213> Hevea brasiliensis
<400> 12
   Met Ala Glu Thr Gly Thr Glu Gly Thr Gly Ile Thr Thr Leu Phe Ser
   1               5                   10                  15
   Pro Tyr Lys Met Gly Lys Phe Ser Leu Ser His Arg Val Val Leu Ala
               20                  25                  30
   Pro Met Thr Arg Cys Arg Ala Leu Asn Gly Ile Pro Asn Ala Ala Leu
           35                  40                  45
   Val Asp Tyr Tyr Thr Gln Arg Ser Thr Pro Gly Gly Phe Leu Ile Thr
       50                  55                  60
   Glu Gly Thr Leu Val Ser Pro Thr Ala Pro Gly Phe Pro His Val Pro
   65                  70                  75                  80
   Gly Ile Tyr Thr Glu Glu Gln Ala Glu Ala Trp Lys Arg Val Val Asp
                   85                  90                  95
   Ala Val His Ala Lys Gly Ser Ile Ile Phe Cys Gln Leu Trp His Val
               100                 105                 110
   Gly Arg Ala Ser His Gln Val Tyr Gln Pro Asn Gly Ala Ala Pro Ile
           115                 120                 125
   Ser Ser Thr Gly Lys Ala Ile Ser Asn Arg Trp Arg Ile Leu Met Pro
       130                 135                 140
   Asp Gly Ser Tyr Gly Lys Tyr Pro Thr Pro Arg Pro Leu Glu Thr Pro
   145                 150                 155                 160
   Glu Ile Leu Glu Val Val Lys Asn Tyr Arg Gln Ser Ala Leu Asn Ala
                   165                 170                 175
   Ile Arg Ala Gly Phe Asp Gly Ile Glu Val His Gly Ala His Gly Tyr
               180                 185                 190
   Leu Ile Asp Gln Phe Leu Lys Asp Gly Ile Asn Asp Arg Thr Asp Glu
           195                 200                 205
   Tyr Gly Gly Ser Ile Asn Asn Arg Cys Arg Phe Leu Met Gln Val Ile
       210                 215                 220
   Gln Ala Val Val Ala Ala Ile Gly Ala Asp Arg Val Gly Phe Arg Met
   225                 230                 235                 240
   Ser Pro Ala Ile Asp His Leu Asp Ala Ile Asp Ser Asp Pro Leu Asn
                   245                 250                 255
   Leu Gly Leu Ala Val Ile Glu Arg Leu Asn Lys Leu Gln Leu Asn Leu
               260                 265                 270
   Gly Ser Lys Leu Thr Tyr Leu His Val Thr Gln Pro Arg Tyr Thr Ala
           275                 280                 285
   Tyr Gly Gln Thr Glu Ser Gly Arg His Gly Thr Glu Glu Glu Glu Ala
       290                 295                 300
   Arg Leu Met Arg Thr Trp Arg Arg Ala Tyr Lys Gly Thr Phe Ile Cys
   305                 310                 315                 320
   Ser Gly Gly Phe Thr Arg Glu Leu Gly Met Glu Ala Ile Ala Gln Asp
                   325                 330                 335
   Asp Ala Asp Leu Val Ser Tyr Gly Arg Leu Phe Ile Ser Asn Pro Asp
               340                 345                 350
   Leu Val Leu Arg Phe Lys Leu Asn Ala Pro Leu Asn Lys Tyr Val Arg
           355                 360                 365
   Lys Thr Phe Tyr Thr Gln Asp Pro Val Val Gly Tyr Thr Asp Tyr Pro
       370                 375                 380
   Phe Phe Arg Lys Val Asp Gly Ser Gln Glu Pro Arg Ser Arg Leu
   385                 390                 395
<210> 13
   <211> 1185
   <212> DNA
   <213> Oryza sativa
<400> 13
   atggatcggc cgccgccgga tcagcagcgg cagaagcagg cgccgctctt ctcgccgtac      60
   cagatgcccc gcttccgcct caaccaccgg gtggtgctgg cgccgatgac gcggtgcagg     120
   gcgatcggcg gggtgcccgg cccggcgctg gcggagtact acgctcagcg gaccacccag     180
   ggtggcctgc tcatctccga gggcaccgtc gtctcgcccg ctggcccggg gtttcctcat     240
   gtccctggga tatacaatca agagcagact gatgcatgga agaaggtggt ggatgctgtt     300
   catgccaagg gaggcatctt tttctgccag ttatggcatg taggcagagc ttctcaccaa     360
   gtataccagc caaacggtgc tgcaccaata tcctcaactg ataagccaat atcagcaaga     420
   tggagaatac tgatgcctga tggctcctat ggcaagtatc ctaaacctag gcgcctggca     480
   gcatcggaaa tacctgaaat tgtcgaacaa tatcgtcaag ccgccattaa tgccattgaa     540
   gcaggttttg atggcattga gatccatggt gctcatggct atatcattga tcaattccta     600
   aaggatggaa tcaatgaccg cactgacgag tatggtggct cactttccaa ccgctgccgg     660
   ttcctacttg aggtaactag ggctgtggtt tctgccattg gagcagaccg agtcgcggtg     720
   aggatatcac cagccattga tcaccttgac gcctatgatt cagaccccat taagctcggc     780
   atggccgttg ttgagcggct gaatgctctc cagcagcagt cagggcggct cgcctacctc     840
   cacgtcacgc agccacggta caccgcctac gggcagaccg agtctgggca gcatggcagt     900
   gccgaggagg agagccgcct gatgcgcacc ctccggggca cgtaccaggg cacattcatg     960
   tgcagtggcg gctacacgcg ggagcttggg ttggaagcag tggagagcgg cgatgccgac    1020
   ctggtgtcgt acgggcggct cttcatatca aacccggacc tggtcgagcg gttcaggctg    1080
   aacgccgggc tgaacaagta cgtgcgcaag acattctaca cgcccgatcc tgtcgtgggt    1140
   tacacggact atccgttcct cggacagcct aagtcgcgga tgtaa                    1185
<210> 14
   <211> 394
   <212> PRT
   <213> oryza sativa
<400> 14
   Met Asp Arg Pro Pro Pro Asp Gln Gln Arg Gln Lys Gln Ala Pro Leu
   1               5                   10                  15
   Phe Ser Pro Tyr Gln Met Pro Arg Phe Arg Leu Asn His Arg Val Val
               20                  25                  30
   Leu Ala Pro Met Thr Arg Cys Arg Ala Ile Gly Gly Val Pro Gly Pro
           35                  40                  45
   Ala Leu Ala Glu Tyr Tyr Ala Gln Arg Thr Thr Gln Gly Gly Leu Leu
       50                  55                  60
   Ile Ser Glu Gly Thr Val Val Ser Pro Ala Gly Pro Gly Phe Pro His
   65                  70                  75                  80
   Val Pro Gly Ile Tyr Asn Gln Glu Gln Thr Asp Ala Trp Lys Lys Val
                   85                  90                  95
   Val Asp Ala Val His Ala Lys Gly Gly Ile Phe Phe Cys Gln Leu Trp
               100                 105                 110
   His Val Gly Arg Ala Ser His Gln Val Tyr Gln Pro Asn Gly Ala Ala
           115                 120                 125
   Pro Ile Ser Ser Thr Asp Lys Pro Ile Ser Ala Arg Trp Arg Ile Leu
       130                     135                     140
   Met Pro Asp Gly Ser Tyr Gly Lys Tyr Pro Lys Pro Arg Arg Leu Ala
   145                 150                 155                 160
   Ala Ser Glu Ile Pro Glu Ile Val Glu Gln Tyr Arg Gln Ala Ala Ile
                   165                 170                 175
   Asn Ala Ile Glu Ala Gly Phe Asp Gly Ile Glu Ile His Gly Ala His
               180                 185                 190
   Gly Tyr Ile Ile Asp Gln Phe Leu Lys Asp Gly Ile Asn Asp Arg Thr
           195                 200                 205
   Asp Glu Tyr Gly Gly Ser Leu Ser Asn Arg Cys Arg Phe Leu Leu Glu
       210                 215                 220
   Val Thr Arg Ala Val Val Ser Ala Ile Gly Ala Asp Arg Val Ala Val
   225                 230                 235                 240
   Arg Ile Ser Pro Ala Ile Asp His Leu Asp Ala Tyr Asp Ser Asp Pro
                   245                 250                 255
   Ile Lys Leu Gly Met Ala Val Val Glu Arg Leu Asn Ala Leu Gln Gln
               260                 265                 270
   Gln Ser Gly Arg Leu Ala Tyr Leu His Val Thr Gln Pro Arg Tyr Thr
           275                 280                 285
   Ala Tyr Gly Gln Thr Glu Ser Gly Gln His Gly Ser Ala Glu Glu Glu
       290                 295                 300
   Ser Arg Leu Met Arg Thr Leu Arg Gly Thr Tyr Gln Gly Thr Phe Met
   305                 310                 315                 320
   Cys Ser Gly Gly Tyr Thr Arg Glu Leu Gly Leu Glu Ala Val Glu Ser
                   325                 330                 335
   Gly Asp Ala Asp Leu Val Ser Tyr Gly Arg Leu Phe Ile Ser Asn Pro
               340                 345                 350
   Asp Leu Val Glu Arg Phe Arg Leu Asn Ala Gly Leu Asn Lys Tyr Val
           355                 360                 365
   Arg Lys Thr Phe Tyr Thr Pro Asp Pro Val Val Gly Tyr Thr Asp Tyr
       370                 375                 380
   Pro Phe Leu Gly Gln Pro Lys Ser Arg Met
   385                 390
<210> 15
   <211> 1200
   <212> DNA
   <213> zea mays
<400> 15
   atggcctcca cggatcgctc cacgccggcg gaggacgagc aacagcagaa gcgcccgtct     60
   ctcttctcgc cgtaccagat gccccgcttc cgcctcgccc accgggtggt gctggcgccg    120
   atgaccaggt gcagggcgcc cgacgcggtc ccaggccccg cgctcgcgga gtactacgcg    180
   cagcggtcca cggacggcgg cttgctcatc tccgagggca ccatcatctc gccgtccggc    240
   cctgggttcc ctcgtgtccc tgggatatac aatcaagaac agactgatgc atggagaaag    300
   gtggttgatg ctgttcatgc caagggagct atctttttct gccaactatg gcatgtaggc    360
   cgagcttctc accaagtata tcagccgggt gctgctgctc cgatatcctc aactgataag    420
   ccaatatcat caagatggag gatactgatg cccgatggat cctatggcaa gtatccaact    480
   ccgaggcgcc tagccacatc cgagatacca gaaattgttg agcaataccg tcaagcagcc    540
   gtaaacgcca tcaaagcagg tttcgatggc atcgagatcc atggcgccca tggctacctc    600
   atcgatcagt tcctcaaggg cggtatcaac gaccggactg acgagtacgg tggctcactc    660
   tccaaccgtt gccggttcct cctggaggtg acccgagccg tggtctctgc gataggggca    720
   gaccgcgtcg cggtccgagt gtccccggcc atcgaccatc tcgacgccta cgactccaac    780
   cccctgcagc tcggcctggc cgtggtggag cgtctcaacg ctctccagca ggaggccggg    840
   cggctggcct acctccacgt gacgcagcca cggtacacgg cgtacgggca gacagagtct    900
   ggccagcacg ggagtgccga ggaggagagc cggctgatgc gtgccgtgcg aggtgcctac    960
   cgtggcacgt tcatgtgcag cggtgggtac acgcgggagc tcggggtcga ggccatcgag   1020
   tccggggacg ctgacctggt gtcctacggg cggctgttca tcgctaatcc cgacctggtg   1080
   gagcggtttc ggcgcgacgc cccgctgaac aaatacgtgc gcaagacgtt ctacacgccg   1140
   gaccccgtcg tcggttacac ggactacaca ttcctcggcc agcctaaggc acgcatgtga   1200
<210> 16
   <211> 399
   <212> PRT
   <213> Zea mays
<400> 16
   Met Ala Ser Thr Asp Arg Ser Thr Pro Ala Glu Asp Glu Gln Gln Gln
   1               5                   10                  15
   Lys Arg Pro Ser Leu Phe Ser Pro Tyr Gln Met Pro Arg Phe Arg Leu
               20                  25                  30
   Ala His Arg Val Val Leu Ala Pro Met Thr Arg Cys Arg Ala Pro Asp
           35                  40                  45
   Ala Val Pro Gly Pro Ala Leu Ala Glu Tyr Tyr Ala Gln Arg Ser Thr
       50                  55                  60
   Asp Gly Gly Leu Leu Ile Ser Glu Gly Thr Ile Ile Ser Pro Ser Gly
   65 70 75 80
   Pro Gly Phe Pro Arg Val Pro Gly Ile Tyr Asn Gln Glu Gln Thr Asp
   85 90 95
   Ala Trp Arg Lys Val Val Asp Ala Val His Ala Lys Gly Ala Ile Phe
   100 105 110
   Phe Cys Gln Leu Trp His Val Gly Arg Ala Ser His Gln Val Tyr Gln
   115 120 125
   Pro Gly Ala Ala Ala Pro Ile Ser Ser Thr Asp Lys Pro Ile Ser Ser
   130 135 140
   Arg Trp Arg Ile Leu Met Pro Asp Gly Ser Tyr Gly Lys Tyr Pro Thr
   145 150 155 160
   Pro Arg Arg Leu Ala Thr Ser Glu Ile Pro Glu Ile Val Glu Gln Tyr
   165 170 175
   Arg Gln Ala Ala Val Asn Ala Ile Lys Ala Gly Phe Asp Gly Ile Glu
   180 185 190
   Ile His Gly Ala His Gly Tyr Leu Ile Asp Gln Phe Leu Lys Gly Gly
   195 200 205
   Ile Asn Asp Arg Thr Asp Glu Tyr Gly Gly Ser Leu Ser Asn Arg Cys
   210 215 220
   Arg Phe Leu Leu Glu Val Thr Arg Ala Val Val Ser Ala Ile Gly Ala
   225 230 235 240
   Asp Arg Val Ala Val Arg Val Ser Pro Ala Ile Asp His Leu Asp Ala
   245 250 255
   Tyr Asp Ser Asn Pro Leu Gln Leu Gly Leu Ala Val Val Glu Arg Leu
   260 265 270
   Asn Ala Leu Gln Gln Glu Ala Gly Arg Leu Ala Tyr Leu His Val Thr
   275 280 285
   Gln Pro Arg Tyr Thr Ala Tyr Gly Gln Thr Glu Ser Gly Gln His Gly
   290 295 300
   Ser Ala Glu Glu Glu Ser Arg Leu Met Arg Ala Val Arg Gly Ala Tyr
   305 310 315 320
   Arg Gly Thr Phe Met Cys Ser Gly Gly Tyr Thr Arg Glu Leu Gly Val
   325 330 335
   Glu Ala Ile Glu Ser Gly Asp Ala Asp Leu Val Ser Tyr Gly Arg Leu
   340 345 350
   Phe Ile Ala Asn Pro Asp Leu Val Glu Arg Phe Arg Arg Asp Ala Pro
   355 360 365
   Leu Asn Lys Tyr Val Arg Lys Thr Phe Tyr Thr Pro Asp Pro Val Val
   370 375 380
   Gly Tyr Thr Asp Tyr Thr Phe Leu Gly Gln Pro Lys Ala Arg Met
   385 390 395
<210> 17
   <211> 1200
   <212> DNA
   <213> Zea mays
<400> 17
   atggcctcca cggatcgctc cgcgccggcg gaggaccagc aacagccgca gcgcccgtcc      60
   ctcttctcgc cgtaccagat gccccacttc cgcctcgcgc accgggtggt gctggcgccg     120
   atgaccaggt gccgggcgcc cgatgcgctc ccgggccccg cgctcgcgga gtactacgcg     180
   cagcggtcca cggaaggcgg cttgctcatc tccgagggca ccatcatctc gcccgccggc     240
   cctgggttcc ctcgtgtccc tgggatatac aatcaagagc agactgatgc atggaaaaag     300
   gtggttgatg ctgttcatgc caagggagcc atctttttct gccaactatg gcatgtaggt     360
   cgagcttctc accaagtata tcagccgggt ggttctgctc caatatcctc tactgataaa     420
   ccaatatcat caagatggag gatactgatg cccgatggat cctatggcaa gtatccaact     480
   ccgaggcgcc tagccacatc cgagatacca gaaattgtcg agcaataccg acaggctgcc     540
   ataaacgcca tcaaagcagg tttcgatggc atcgagatcc acggtgccca tggctaccta     600
   atcgaccagt tcctcaagga cggtatcaac gacagggctg acgagtacgg tggctcactc     660
   tccaaccgct gccggttcct cctggaggtg acccgcgccg tggtctccgc gataggggca     720
   gaccgggtgg cggtccgggt gtccccggcc atcgaccacc tcgacgcgta cgactccaac     780
   ccgctgcagc tcggcctggc cgtagtggac cgcctcaacg ctctccagga ggagaccggg     840
   cggctggcct acctgcacgt gacgcagcca cggtacacgg cgtacgggca gacggagtcc     900
   ggccagcacg ggagcgccga ggaggagagc cggctgatgc gcgccctgcg gggcgcctac     960
   cgcggcacgt tcatgtgcag cggtgggtac acgcgcgagc tcggcgtgga ggccgtcgag    1020
   tcgtgggacg ccgacctggt gtcctacggg cggctgttca tcgctaaccc ggacctggtg    1080
   gagcggttcc ggcgcgacgc cccgctgaac agatacgtgc gcaagacgtt ctacaccccg    1140
   gatcccgtcg ttggttacac ggactacccg ttcctcggcc agcctaaggc gcgcatgtga    1200
<210> 18
   <211> 399
   <212> PRT
   <213> Zea mays
<400> 18
   Met Ala Ser Thr Asp Arg Ser Ala Pro Ala Glu Asp Gln Gln Gln Pro
   1               5                   10                  15
   Gln Arg Pro Ser Leu Phe Ser Pro Tyr Gln Met Pro His Phe Arg Leu
               20                  25                  30
   Ala His Arg Val Val Leu Ala Pro Met Thr Arg Cys Arg Ala Pro Asp
           35                  40                  45
   Ala Leu Pro Gly Pro Ala Leu Ala Glu Tyr Tyr Ala Gln Arg Ser Thr
       50                  55                  60
   Glu Gly Gly Leu Leu Ile Ser Glu Gly Thr Ile Ile Ser Pro Ala Gly
   65                  70                  75                  80
   Pro Gly Phe Pro Arg Val Pro Gly Ile Tyr Asn Gln Glu Gln Thr Asp
                   85                  90                  95
   Ala Trp Lys Lys Val Val Asp Ala Val His Ala Lys Gly Ala Ile Phe
               100                 105                 110
   Phe Cys Gln Leu Trp His Val Gly Arg Ala Ser His Gln Val Tyr Gln
           115                 120                 125
   Pro Gly Gly Ser Ala Pro Ile Ser Ser Thr Asp Lys Pro Ile Ser Ser
       130                 135                 140
   Arg Trp Arg Ile Leu Met Pro Asp Gly Ser Tyr Gly Lys Tyr Pro Thr
   145                 150                 155                 160
   Pro Arg Arg Leu Ala Thr Ser Glu Ile Pro Glu Ile Val Glu Gln Tyr
                   165                 170                 175
   Arg Gln Ala Ala Ile Asn Ala Ile Lys Ala Gly Phe Asp Gly Ile Glu
               180                 185                 190
   Ile His Gly Ala His Gly Tyr Leu Ile Asp Gln Phe Leu Lys Asp Gly
           195                 200                 205
   Ile Asn Asp Arg Ala Asp Glu Tyr Gly Gly Ser Leu Ser Asn Arg Cys
       210                 215                 220
   Arg Phe Leu Leu Glu Val Thr Arg Ala Val Val Ser Ala Ile Gly Ala
   225                 230                 235                 240
   Asp Arg Val Ala Val Arg Val Ser Pro Ala Ile Asp His Leu Asp Ala
                   245                 250                 255
   Tyr Asp Ser Asn Pro Leu Gln Leu Gly Leu Ala Val Val Asp Arg Leu
               260                 265                 270
   Asn Ala Leu Gln Glu Glu Thr Gly Arg Leu Ala Tyr Leu His Val Thr
           275                 280                 285
   Gln Pro Arg Tyr Thr Ala Tyr Gly Gln Thr Glu Ser Gly Gln His Gly
       290                 295                 300
   Ser Ala Glu Glu Glu Ser Arg Leu Met Arg Ala Leu Arg Gly Ala Tyr
   305                 310                 315                 320
   Arg Gly Thr Phe Met Cys Ser Gly Gly Tyr Thr Arg Glu Leu Gly Val
                   325                 330                 335
   Glu Ala Val Glu Ser Trp Asp Ala Asp Leu Val Ser Tyr Gly Arg Leu
               340                 345                 350
   Phe Ile Ala Asn Pro Asp Leu Val Glu Arg Phe Arg Arg Asp Ala Pro
           355                 360                 365
   Leu Asn Arg Tyr Val Arg Lys Thr Phe Tyr Thr Pro Asp Pro Val Val
       370                 375                 380
   Gly Tyr Thr Asp Tyr Pro Phe Leu Gly Gln Pro Lys Ala Arg Met
   385                 390                 395
<210> 19
   <211> 762
   <212> DNA
   <213> Arachis hypogaea
<400> 19
   atggcagaca acgagtccag cagcctgttt tctgcttaca agatggcaaa attcagtctc      60
   tcgcacaggg tggtgttggc gcccatgacc aggtgcagag ccttgaacgg catcccacgt     120
   gccgctcacg cggagtatta cgctcagaga tccacacccg gtggattcct catcaccgaa     180
   gggactttga tctctcccac tgctcctggc ttccctcatg tacctggaat atactctgag     240
   gagcaagttg aggcatggag aaacgtcgtg gatgccgtgc atgccaaagg cagcttcatc     300
   ttctgtcaac tctggcatgc tggccgtgca tctcatcccg tgtatcagcc tggggcggcg     360
   ccgcccattt cctccacaaa caaggctatt tcctccagat ggagaattct cttgccggat     420
   cagtcctacg gcgtgtatcc agagccccga ccacttgact cttctgagat accacaaata     480
   gtggaccact atcgccagtc agcggtcaac gctatccgag caggtttcga tggaattgag     540
   attcacggtg cacacggcta tctgattgat caattcttga aggacgggat caatgagcga     600
   agagatgagt atggtggatc catttcaaat aggtgcaggt tcttaatgca ggtagttaaa     660
   gcagttgttt ctgcaattgg agcagaaaga gtaggtgtta gaatctcacc ggcaatcgac     720
   cacctggatg ccatggactc cgacccgctt gcctggggct ag                        762
<210> 20
   <211> 250
   <212> PRT
   <213> Arachis hypogaea
<400> 20
   Met Ala Asp Asn Glu Ser Ser Ser Leu Phe Ser Ala Tyr Lys Met Ala
   1               5                   10                  15
   Lys Phe Ser Leu Ser His Arg Val Val Leu Ala Pro Met Thr Arg Cys
               20                  25                  30
   Arg Ala Leu Asn Gly Ile Pro Arg Ala Ala His Ala Glu Tyr Tyr Ala
           35                  40                  45
   Gln Arg Ser Thr Pro Gly Gly Phe Leu Ile Thr Glu Gly Thr Leu Ile
       50                  55                  60
   Ser Pro Thr Ala Pro Gly Phe Pro His Val Pro Gly Ile Tyr Ser Glu
   65                  70                  75                  80
   Glu Gln Val Glu Ala Trp Arg Asn Val Val Asp Ala Val His Ala Lys
                   85                  90                  95
   Gly Ser Phe Ile Phe Cys Gln Leu Trp His Ala Gly Arg Ala Ser His
               100                 105                 110
   Pro Val Tyr Gln Pro Gly Ala Ala Pro Pro Ile Ser Ser Thr Asn Lys
           115                 120                 125
   Ala Ile Ser Ser Arg Trp Arg Ile Leu Leu Pro Asp Gln Ser Tyr Gly
       130                 135                 140
   Val Tyr Pro Glu Pro Arg Pro Leu Asp Ser Ser Glu Ile Pro Gln Ile
   145                 150                 155                 160
   Val Asp His Tyr Arg Gln Ser Ala Val Asn Ala Ile Arg Ala Gly Phe
                   165                 170                 175
   Asp Gly Ile Glu Ile His Gly Ala His Gly Tyr Leu Ile Asp Gln Phe
               180                 185                 190
   Leu Lys Asp Gly Ile Asn Glu Arg Arg Asp Glu Tyr Gly Gly Ser Ile
           195                 200                 205
   Ser Asn Arg Cys Arg Phe Leu Met Gln Val Val Lys Ala Val Val Ser
       210                 215                 220
   Ala Ile Gly Ala Glu Arg Val Gly Val Arg Ile Ser Pro Ala Ile Asp
   225                 230                 235                 240
   His Leu Asp Ala Met Asp Ser Asp Pro Leu
                   245                 250
<210> 21
   <211> 1203
   <212> DNA
   <213> Solanum tuberosum
<400> 21
   atggctaaaa cgacatcgtc ttcagctcaa gatggaagca atcccctctt ctctccttac      60
   aagatggcaa agttcaatct atcccacagg atagtattgg ctccgatgac aaggtgcaga     120
   gcattgaata atattccttc ggcggcgctg ggggaatatt acgagcagag agcgacggcc      180
   ggtggatttc tgatcactga aggcactatg atttctccga cttcagctgg gtttcctcat     240
   gtgccaggga ttttcacaaa ggagcaagta gaggaatgga agaaaatagt tgatgtagtg     300
   catgcaaagg gtgctgtcat attttgtcag ttgtggcatg ttggtcgtgc atctcatgaa     360
   gtgtatcaac ctgctggagc tgcaccaata tcatctactg agaagcctat atcaaagagg     420
   tggagaattc tgatgcctga tggaactcat gggatttatc caaaaccaag agcaattgga     480
   acctatgaga tctcacaagt ggttgaagat tattgcaggt cggccttgaa tgctattgaa     540
   gcaggttttg atggtattga aatccatgga gctcacggtt acttgattga ccaattcttg     600
   aaagatggga tcaatgaccg gacagatgag tatggtggat cactagccaa ccggtgcaaa     660
   ttcatcacac aggtggttca agcagtcatc tcagcaatag gagctgatcg tgtaggcgtt     720
   agagtttcac cagcaataga tcatcttgat gccatggact ctaatccact cagcctaggc     780
   ttagcagttg ttgaaagact aaacaaaatc caactccatt ctggttccaa gcttgcctat     840
   cttcatgtaa cacagccacg atacgtagca tatgggcaaa ccgaagcagg cagacttggc     900
   agtgaagagg aggaggcgca tttaatgagg actttgagga acgcatatca ggggacattc     960
   atttgcagtg gtggatacac tagggagcta ggaattgagg ctgtggcaca aggtgatgct    1020
   gatctcgtgt catatggacg tcttttcatc tctaatcctg atttggttat gagaatcaag    1080
   ctaaatgcac ctctaaataa gtataacagg aagacattct atactcaaga tccagttgtg    1140
   ggatacacag attacccttt ccttcaagga aatggaagca acggaccgtt atcgcgtctg    1200
   tga                                                                  1203
<210> 22
   <211> 400
   <212> PRT
   <213> Solanum tuberosum
<400> 22
   Met Ala Lys Thr Thr Ser Ser Ser Ala Gln Asp Gly Ser Asn Pro Leu
   1               5                   10                  15
   Phe Ser Pro Tyr Lys Met Ala Lys Phe Asn Leu Ser His Arg Ile Val
               20                  25                  30
   Leu Ala Pro Met Thr Arg Cys Arg Ala Leu Asn Asn Ile Pro Ser Ala
           35                  40                  45
   Ala Leu Gly Glu Tyr Tyr Glu Gln Arg Ala Thr Ala Gly Gly Phe Leu
       50                  55                  60
   Ile Thr Glu Gly Thr Met Ile Ser Pro Thr Ser Ala Gly Phe Pro His
   65 70 75 80
   Val Pro Gly Ile Phe Thr Lys Glu Gln Val Glu Glu Trp Lys Lys Ile
   85 90 95
   Val Asp Val Val His Ala Lys Gly Ala Val Ile Phe Cys Gln Leu Trp
   100 105 110
   His Val Gly Arg Ala Ser His Glu Val Tyr Gln Pro Ala Gly Ala Ala
   115 120 125
   Pro Ile Ser Ser Thr Glu Lys Pro Ile Ser Lys Arg Trp Arg Ile Leu
   130 135 140
   Met Pro Asp Gly Thr His Gly Ile Tyr Pro Lys Pro Arg Ala Ile Gly
   145 150 155 160
   Thr Tyr Glu Ile Ser Gln Val Val Glu Asp Tyr Cys Arg Ser Ala Leu
   165 170 175
   Asn Ala Ile Glu Ala Gly Phe Asp Gly Ile Glu Ile His Gly Ala His
   180 185 190
   Gly Tyr Leu Ile Asp Gln Phe Leu Lys Asp Gly Ile Asn Asp Arg Thr
   195 200 205
   Asp Glu Tyr Gly Gly Ser Leu Ala Asn Arg Cys Lys Phe Ile Thr Gln
   210 215 220
   Val Val Gln Ala Val Ile Ser Ala Ile Gly Ala Asp Arg Val Gly Val
   225 230 235 240
   Arg Val Ser Pro Ala Ile Asp His Leu Asp Ala Met Asp Ser Asn Pro
   245 250 255
   Leu Ser Leu Gly Leu Ala Val Val Glu Arg Leu Asn Lys Ile Gln Leu
   260 265 270
   His Ser Gly Ser Lys Leu Ala Tyr Leu His Val Thr Gln Pro Arg Tyr
   275 280 285
   Val Ala Tyr Gly Gln Thr Glu Ala Gly Arg Leu Gly Ser Glu Glu Glu
   290 295 300
   Glu Ala His Leu Met Arg Thr Leu Arg Asn Ala Tyr Gln Gly Thr Phe
   305 310 315 320
   Ile Cys Ser Gly Gly Tyr Thr Arg Glu Leu Gly Ile Glu Ala Val Ala
   325 330 335
   Gln Gly Asp Ala Asp Leu Val Ser Tyr Gly Arg Leu Phe Ile Ser Asn
               340                 345                 350
   Pro Asp Leu Val Met Arg Ile Lys Leu Asn Ala Pro Leu Asn Lys Tyr
           355                 360                 365
   Asn Arg Lys Thr Phe Tyr Thr Gln Asp Pro Val Val Gly Tyr Thr Asp
       370                 375                 380
   Tyr Pro Phe Leu Gln Gly Asn Gly Ser Asn Gly Pro Leu Ser Arg Leu
   385                 390                 395                 400
<210> 23
   <211> 1191
   <212> DNA
   <213> Prunus persica
<400> 23
   atggcggagg cttcatctca gggacccact ctcttttctc cgttcaagat gggcaagttc      60
   aatctgtctc acagggtggt gcttgcgccg atgacgaggt gccgagcgtt gaacggcttg     120
   ccgcagccgg cgctggccga gtactacact caaaggtcaa ccaacggcgg ctttctgatc     180
   accgaaggca ctttggtctc cgacactggc gccgggtttc cacatgttcc tgggatttac     240
   aatgatgaac aggtggaggc atggaagaag gtggtggatg ccgttcacgc caaaggtgcc     300
   attattttct gtcaactttg gcatgtaggt cgtgcttctc atgaagttta ccaacctggt     360
   gggggttcac caatatcttc aaccgatgtt cccatttcga ggaggtggag aattctatta     420
   ccggatgcgt ctcatgccac ttaccctaag cctagacgct tagaaacccc tgaaatcctc     480
   caagtggtgg agcattatcg acaggctgcc ttgaatgcca ttagagcagg ttttgatgga     540
   attgagattc atggggcaca tggctacctc attgatcaat tcttgaaaga tgggatcaat     600
   gatcgaacag atgagtatgg cggatcactt gcaaaccgtt gcaaattctt gcttcaggtg     660
   gttcaagcag tagttggagc cgtaggtgct gatagggttg gtgtcagaat ctcaccagcc     720
   attgatcacc ttgatgcagt tgactctgct ccacttaccc taagccttgg agtgattgaa     780
   aggctcaaca agcttcaaca agactggggc tcaaaactca cttatctcca tgttactcag     840
   ccccgttacg cagcatatgg ccaaaccgaa tctggcaaac ctggcagtga tgaagaggaa     900
   gctgtgttta tgaggacttt aagaaatgct tatcgtggta catttgttgc tagtggtggg     960
   tacactaggg agcttggaat tcatgctgtg gcttctaggg atgctgattt agtgtcttat    1020
   ggtcgccttt ttatctcgaa ccccgacttg gttttgagat tgaagcttaa tgcacctttg    1080
   accaggtata acaggaagac tttctacacg caagaccctg ttgttgggta cacagactac    1140
   ccttttctga gcaatgcaaa tgggaaagag gaaccactct cccgcctctg a             1191
<210> 24
   <211> 396
   <212> PRT
   <213> Prunus persica
<400> 24
   Met Ala Glu Ala Ser Ser Gln Gly Pro Thr Leu Phe Ser Pro Phe Lys
   1               5                   10                  15
   Met Gly Lys Phe Asn Leu Ser His Arg Val Val Leu Ala Pro Met Thr
               20                  25                  30
   Arg Cys Arg Ala Leu Asn Gly Leu Pro Gln Pro Ala Leu Ala Glu Tyr
           35                  40                  45
   Tyr Thr Gln Arg Ser Thr Asn Gly Gly Phe Leu Ile Thr Glu Gly Thr
       50                  55                  60
   Leu Val Ser Asp Thr Gly Ala Gly Phe Pro His Val Pro Gly Ile Tyr
   65                  70                  75                  80
   Asn Asp Glu Gln Val Glu Ala Trp Lys Lys Val Val Asp Ala Val His
                   85                  90                  95
   Ala Lys Gly Ala Ile Ile Phe Cys Gln Leu Trp His Val Gly Arg Ala
               100                 105                 110
   Ser His Glu Val Tyr Gln Pro Gly Gly Gly Ser Pro Ile Ser Ser Thr
           115                 120                 125
   Asp Val Pro Ile Ser Arg Arg Trp Arg Ile Leu Leu Pro Asp Ala Ser
       130                 135                 140
   His Ala Thr Tyr Pro Lys Pro Arg Arg Leu Glu Thr Pro Glu Ile Leu
   145                 150                 155                 160
   Gln Val Val Glu His Tyr Arg Gln Ala Ala Leu Asn Ala Ile Arg Ala
                   165                 170                 175
   Gly Phe Asp Gly Ile Glu Ile His Gly Ala His Gly Tyr Leu Ile Asp
               180                 185                 190
   Gln Phe Leu Lys Asp Gly Ile Asn Asp Arg Thr Asp Glu Tyr Gly Gly
           195                 200                 205
   Ser Leu Ala Asn Arg Cys Lys Phe Leu Leu Gln Val Val Gln Ala Val
       210                 215                 220
   Val Gly Ala Val Gly Ala Asp Arg Val Gly Val Arg Ile Ser Pro Ala
   225                 230                 235                 240
   Ile Asp His Leu Asp Ala Val Asp Ser Ala Pro Leu Thr Leu Ser Leu
                   245                 250                 255
   Gly Val Ile Glu Arg Leu Asn Lys Leu Gln Gln Asp Trp Gly Ser Lys
               260                 265                 270
   Leu Thr Tyr Leu His Val Thr Gln Pro Arg Tyr Ala Ala Tyr Gly Gln
           275                 280                 285
   Thr Glu Ser Gly Lys Pro Gly Ser Asp Glu Glu Glu Ala Val Phe Met
       290                 295                 300
   Arg Thr Leu Arg Asn Ala Tyr Arg Gly Thr Phe Val Ala Ser Gly Gly
   305                 310                 315                 320
   Tyr Thr Arg Glu Leu Gly Ile His Ala Val Ala Ser Arg Asp Ala Asp
                   325                 330                 335
   Leu Val Ser Tyr Gly Arg Leu Phe Ile Ser Asn Pro Asp Leu Val Leu
               340                 345                 350
   Arg Leu Lys Leu Asn Ala Pro Leu Thr Arg Tyr Asn Arg Lys Thr Phe
           355                 360                 365
   Tyr Thr Gln Asp Pro Val Val Gly Tyr Thr Asp Tyr Pro Phe Leu Ser
       370                 375                 380
   Asn Ala Asn Gly Lys Glu Glu Pro Leu Ser Arg Leu
   385                 390                 395
<210> 25
   <211> 1209
   <212> DNA
   <213> Gossypium hirsutum
<400> 25
   atggagcatg gagaaaaagt aaaaatggcg gattctcaag aaacccctac gctgttctct      60
   ccttacaaga tgggcaaatt caatctttcc cacagggtgg tgctagcgcc tatgacgaga     120
   tgcagggcgt tgaatggaat tccaaggccg gcgcttgctg aatattacac gcagaggtcc     180
   actcctggcg gctttctcat cactgaagga acgttgatct ccgacactgg agcagggttt     240
   ccacatgttc ctggaatcta caatgaagaa caggtggagg catggaagat gattgtggat     300
   gctgttcatg ccaaaggggg catcattttc tgtcaactat ggcatgttgg ccgagcatct     360
   catacagtgt atcaacctgg cggagtggca ccaatatcct caacaaacaa gcccatctca     420
   aagaggtgga gaattcttat gccagatggt agctatggca tatatcccaa acctcgaccc     480
   ctggaaacat cagaaataca agaggttgta gagcattacc gcaaagcagc cttgaatgcc     540
   attcgagcag gttttgatgg gattgagatt catggagcac atggttatct catcgaccaa     600
   ttcttaaaag atgggatcaa tgatcgcaca gatgagtacg gtggatcatt ggcaaaccgc     660
   tgcaaattct taatgcaaat tgttcaagca gtagcttcag ccattggtat agatagagtt     720
   gcggtcagaa tgtcgcctgc aattgatcac ctcgatgcaa ccgactctaa tccgctcaac     780
   ctaggcttgg ctgtgattga gagacttaac aagctccagc tacagctggg gtcaaaactc     840
   gcttatcttc atgtgacgca acctcgttat catgcatacg ggcaaactga atcaggcaaa     900
   cacgggaatg aagacgagga agcttattta ttgagggcac tgaagcggac ctatcacgga     960
   actttcatgt gtagtggcgg gttcaatagg gagctgggaa tgcaagctgt ggccgagggt    1020
   gatgcagatc ttgtatctta tggccgcctt ttcatctcaa atcctgacct agtctttagg    1080
   ttgaaggtca atgcaccatt aaataggtac attaggaaga cgttctatac tcatgatcct    1140
   gttgttgggt acacagacta tccattcctg aacgaagaga agggtagaca agtactgtca    1200
   cgcctttga                                                            1209
<210> 26
   <211> 402
   <212> PRT
   <213> Gossypium hirsutum
<400> 26
   Met Glu His Gly Glu Lys Val Lys Met Ala Asp Ser Gln Glu Thr Pro
   1               5                   10                  15
   Thr Leu Phe Ser Pro Tyr Lys Met Gly Lys Phe Asn Leu Ser His Arg
               20                  25                  30
   Val Val Leu Ala Pro Met Thr Arg Cys Arg Ala Leu Asn Gly Ile Pro
           35                  40                  45
   Arg Pro Ala Leu Ala Glu Tyr Tyr Thr Gln Arg Ser Thr Pro Gly Gly
       50                  55                  60
   Phe Leu Ile Thr Glu Gly Thr Leu Ile Ser Asp Thr Gly Ala Gly Phe
   65                  70                  75                  80
   Pro His Val Pro Gly Ile Tyr Asn Glu Glu Gln Val Glu Ala Trp Lys
                   85                  90                  95
   Met Ile Val Asp Ala Val His Ala Lys Gly Gly Ile Ile Phe Cys Gln
               100                 105                 110
   Leu Trp His Val Gly Arg Ala Ser His Thr Val Tyr Gln Pro Gly Gly
           115                 120                 125
   Val Ala Pro Ile Ser Ser Thr Asn Lys Pro Ile Ser Lys Arg Trp Arg
       130                 135                 140
   Ile Leu Met Pro Asp Gly Ser Tyr Gly Ile Tyr Pro Lys Pro Arg Pro
   145                 150                 155                 160
   Leu Glu Thr Ser Glu Ile Gln Glu Val Val Glu His Tyr Arg Lys Ala
                   165                 170                 175
   Ala Leu Asn Ala Ile Arg Ala Gly Phe Asp Gly Ile Glu Ile His Gly
               180                 185                 190
   Ala His Gly Tyr Leu Ile Asp Gln Phe Leu Lys Asp Gly Ile Asn Asp
               195                 200                 205
   Arg Thr Asp Glu Tyr Gly Gly Ser Leu Ala Asn Arg Cys Lys Phe Leu
       210                 215                 220
   Met Gln Ile Val Gln Ala Val Ala Ser Ala Ile Gly Ile Asp Arg Val
   225                 230                 235                 240
   Ala Val Arg Met Ser Pro Ala Ile Asp His Leu Asp Ala Thr Asp Ser
                   245                 250                 255
   Asn Pro Leu Asn Leu Gly Leu Ala Val Ile Glu Arg Leu Asn Lys Leu
               260                 265                 270
   Gln Leu Gln Leu Gly Ser Lys Leu Ala Tyr Leu His Val Thr Gln Pro
           275                 280                 285
   Arg Tyr His Ala Tyr Gly Gln Thr Glu Ser Gly Lys His Gly Asn Glu
       290                 295                 300
   Asp Glu Glu Ala Tyr Leu Leu Arg Ala Leu Lys Arg Thr Tyr His Gly
   305                 310                 315                 320
   Thr Phe Met Cys Ser Gly Gly Phe Asn Arg Glu Leu Gly Met Gln Ala
                   325                 330                 335
   Val Ala Glu Gly Asp Ala Asp Leu Val Ser Tyr Gly Arg Leu Phe Ile
               340                 345                 350
   Ser Asn Pro Asp Leu Val Phe Arg Leu Lys Val Asn Ala Pro Leu Asn
           355                 360                 365
   Arg Tyr Ile Arg Lys Thr Phe Tyr Thr His Asp Pro Val Val Gly Tyr
       370                 375                 380
   Thr Asp Tyr Pro Phe Leu Asn Glu Glu Lys Gly Arg Gln Val Leu Ser
   385                 390                 395                 400
   Arg Leu
<210> 27
   <211> 1200
   <212> DNA
   <213> Coffea canephora
<400> 27
   atggctgaaa ctaagtcaga tcaaggaagc ccatctctct tttctccata caagatggga     60
   aagttcaatc tgtctcacag ggtggttctg gcgccgatga caagatgcag ggccataaat    120
   agcattcctc agcctgccat ggcggagtac tacgcccaaa gagcaaccaa tggtggcttt    180
   ctcatcacgg agggcaccat gatctcccca agtgctgccg ggtttccgca tgtgccgggg    240
   atctttacaa aggaacaagt ggaggcatgg aagcaagtgg ttgatgcagt acatgccaag    300
   ggtgctatta ttttctgtca actgtggcac gttggccgtg catcacatga agtttatcaa    360
   cctggtggtg gtgcacccat atcatcaacg ggaaagccta tatcaaagag gtggaggata    420
   ttgatgcctg atggcagcca tgggatctac cctaaaccac gtccattaac aacagcgcat    480
   gagattgcgc aagttgtgga agattaccgc cagtcggcct tgaatgccat tgaagccggt    540
   tttgatggta ttgaaatcca tggagcacat ggctacctaa ttgaccagtt cttgaaagat    600
   gggatcaatg atcggacaga tgaatatggt ggatctgttg caaatcgctg caaattcatt    660
   gtgcaggtgg ttcaggctgt tgtttcagca attggtgcag atcgtgttgg tgtcagaatt    720
   tcccctgcta ttgaccatct tgatgccatg gactctgatc cactaagctt aggcctggca    780
   gtgattgaga gacttaacga gctccaactg aattcaggct ccaagttaac atacttgcac    840
   gtgactcaac ctcgatatac agcgtatggc cagacagagg caggcagaca ggggagtgaa    900
   gaagaggagg cccaactagt gaggaccttg cgaaaagctt atcaaggaac tttcatttcc    960
   agtggtgggt tcaccagaga gctaggagtt gaagcggtag ctcagggtga tgctgatttg   1020
   gtttcctatg gtcgcctttt tatctcaaat ccagacttag ttttacgctt taagctaaat   1080
   gctcctttga ttaggtataa tagatctacc ttctatactc atgatcctgt tgtaggatac   1140
   acagattacc cttttctaag caatggtacc agtggcaatg taccacaatc acgtctgtaa   1200
<210> 28
   <211> 399
   <212> PRT
   <213> Coffea canephora
<400> 28
   Met Ala Glu Thr Lys Ser Asp Gln Gly Ser Pro Ser Leu Phe Ser Pro
   1               5                   10                  15
   Tyr Lys Met Gly Lys Phe Asn Leu Ser His Arg Val Val Leu Ala Pro
               20                  25                  30
   Met Thr Arg Cys Arg Ala Ile Asn Ser Ile Pro Gln Pro Ala Met Ala
           35                  40                  45
   Glu Tyr Tyr Ala Gln Arg Ala Thr Asn Gly Gly Phe Leu Ile Thr Glu
       50                  55                  60
   Gly Thr Met Ile Ser Pro Ser Ala Ala Gly Phe Pro His Val Pro Gly
   65                  70                  75                  80
   Ile Phe Thr Lys Glu Gln Val Glu Ala Trp Lys Gln Val Val Asp Ala
                   85                  90                  95
   Val His Ala Lys Gly Ala Ile Ile Phe Cys Gln Leu Trp His Val Gly
               100                 105                 110
   Arg Ala Ser His Glu Val Tyr Gln Pro Gly Gly Gly Ala Pro Ile Ser
           115                 120                 125
   Ser Thr Gly Lys Pro Ile Ser Lys Arg Trp Arg Ile Leu Met Pro Asp
       130                 135                 140
   Gly Ser His Gly Ile Tyr Pro Lys Pro Arg Pro Leu Thr Thr Ala His
   145                 150                 155                 160
   Glu Ile Ala Gln Val Val Glu Asp Tyr Arg Gln Ser Ala Leu Asn Ala
                   165                 170                 175
   Ile Glu Ala Gly Phe Asp Gly Ile Glu Ile His Gly Ala His Gly Tyr
               180                 185                 190
   Leu Ile Asp Gln Phe Leu Lys Asp Gly Ile Asn Asp Arg Thr Asp Glu
           195                 200                 205
   Tyr Gly Gly Ser Val Ala Asn Arg Cys Lys Phe Ile Val Gln Val Val
       210                 215                 220
   Gln Ala Val Val Ser Ala Ile Gly Ala Asp Arg Val Gly Val Arg Ile
   225                 230                 235                 240
   Ser Pro Ala Ile Asp His Leu Asp Ala Met Asp Ser Asp Pro Leu Ser
                   245                 250                 255
   Leu Gly Leu Ala Val Ile Glu Arg Leu Asn Glu Leu Gln Leu Asn Ser
               260                 265                 270
   Gly Ser Lys Leu Thr Tyr Leu His Val Thr Gln Pro Arg Tyr Thr Ala
           275                 280                 285
   Tyr Gly Gln Thr Glu Ala Gly Arg Gln Gly Ser Glu Glu Glu Glu Ala
       290                 295                 300
   Gln Leu Val Arg Thr Leu Arg Lys Ala Tyr Gln Gly Thr Phe Ile Ser
   305                 310                 315                 320
   Ser Gly Gly Phe Thr Arg Glu Leu Gly Val Glu Ala Val Ala Gln Gly
                   325                 330                 335
   Asp Ala Asp Leu Val Ser Tyr Gly Arg Leu Phe Ile Ser Asn Pro Asp
               340                 345                 350
   Leu Val Leu Arg Phe Lys Leu Asn Ala Pro Leu Ile Arg Tyr Asn Arg
           355                 360                 365
   Ser Thr Phe Tyr Thr His Asp Pro Val Val Gly Tyr Thr Asp Tyr Pro
       370                 375                 380
   Phe Leu Ser Asn Gly Thr Ser Gly Asn Val Pro Gln Ser Arg Leu
   385                 390                 395
<210> 29
   <211> 1414
   <212> DNA
   <213> Glycine max
<220>
   <221> CDS
   <222> (1)..(1167)
<220>
   <221> 3'UTR
   <222> (1168)..(1414)
<400> 29
   atg gca gat aac tca att agc tta ttt tct cca tac aac aag atg ggc      48
   Met Ala Asp Asn Ser Ile Ser Leu Phe Ser Pro Tyr Asn Lys Met Gly
   1               5                   10                  15
   aaa ttc aac ctc tct cat agg gtg gta ttg gct ccc atg acc aga tgc      96
   Lys Phe Asn Leu Ser His Arg Val Val Leu Ala Pro Met Thr Arg Cys
               20                  25                  30
   aga gcg ctc aat ggg act cca ctg gca gca cat gct gaa tac tac gct     144
   Arg Ala Leu Asn Gly Thr Pro Leu Ala Ala His Ala Glu Tyr Tyr Ala
           35                  40                  45
   cag aga tca aca ccg ggt gga ttt ctc atc act gaa ggc acc ttg atc     192
   Gln Arg Ser Thr Pro Gly Gly Phe Leu Ile Thr Glu Gly Thr Leu Ile
       50                  55                  60
   tct cca act tct tct ggg ttt cct cat gtt cct gga ata tac tca gat     240
   Ser Pro Thr Ser Ser Gly Phe Pro His Val Pro Gly Ile Tyr Ser Asp
   65                  70                  75                  80
   gaa cag gta gag gca tgg aga aat gta gtg gac gcc gtg cat gcc aac     288
   Glu Gln Val Glu Ala Trp Arg Asn Val Val Asp Ala Val His Ala Asn
                   85                  90                  95
   ggc agc ttt atc ttc tgt caa ctc tgg cat gtt ggc cgt gca tca cat     336
   Gly Ser Phe Ile Phe Cys Gln Leu Trp His Val Gly Arg Ala Ser His
               100                 105                 110
   cca gtg tat cag cct ggt ggg gct cta ccc tct tcg tcc acc agc aaa     384
   Pro Val Tyr Gln Pro Gly Gly Ala Leu Pro Ser Ser Ser Thr Ser Lys
           115                 120                 125
   ccc ata tca gac aag tgg aaa att ctc atg ccc gat ggc tcc cat ggc     432
   Pro Ile Ser Asp Lys Trp Lys Ile Leu Met Pro Asp Gly Ser His Gly
       130                 135                 140
   atc tat cca gag cct cgt gca ctt acc act tct gag ata tct gaa ata     480
   Ile Tyr Pro Glu Pro Arg Ala Leu Thr Thr Ser Glu Ile Ser Glu Ile
   145                 150                 155                 160
   gtg cat cat tat cgc caa gca gct att aat gca att cga gca ggt ttt     528
   Val His His Tyr Arg Gln Ala Ala Ile Asn Ala Ile Arg Ala Gly Phe
                   165                 170                 175
   gat gga atc gag att cat gga gca cat ggg tat ctc att gat caa ttc     576
   Asp Gly Ile Glu Ile His Gly Ala His Gly Tyr Leu Ile Asp Gln Phe
               180                 185                 190
   tta aag gat gca atc aat gat aga aca gat gaa tac ggt gga cca cta     624
   Leu Lys Asp Ala Ile Asn Asp Arg Thr Asp Glu Tyr Gly Gly Pro Leu
           195                 200                 205
   gaa aac cgg tgc agg ttc tta atg gag gta gtt gaa gct gtt gtc tct       672
   Glu Asn Arg Cys Arg Phe Leu Met Glu Val Val Glu Ala Val Val Ser
       210                 215                 220
   gcc att gga gcg gaa aga gtt gct atc aga att tca cca gca att gat       720
   Ala Ile Gly Ala Glu Arg Val Ala Ile Arg Ile Ser Pro Ala Ile Asp
   225                 230                 235                 240
   ttc aat gac gcc ttt gac tct gac cca ctt ggg cta ggc tta gca gtg       768
   Phe Asn Asp Ala Phe Asp Ser Asp Pro Leu Gly Leu Gly Leu Ala Val
                   245                 250                 255
   att gaa aga ctc aac aat ttg cag aaa caa gtg ggc aca aaa ctc gct       816
   Ile Glu Arg Leu Asn Asn Leu Gln Lys Gln Val Gly Thr Lys Leu Ala
               260                 265                 270
   tat ctt cat gtt act cag cct cga ttc aca ctt ttg gcg caa acc gag       864
   Tyr Leu His Val Thr Gln Pro Arg Phe Thr Leu Leu Ala Gln Thr Glu
           275                 280                 285
   tca gtg agt gaa aag gag gaa gct cat ttc atg cag aaa tgg aga gag       912
   Ser Val Ser Glu Lys Glu Glu Ala His Phe Met Gln Lys Trp Arg Glu
       290                 295                 300
   gct tat gag gga aca ttc atg tgt agt gga gct ttt act agg gac tca       960
   Ala Tyr Glu Gly Thr Phe Met Cys Ser Gly Ala Phe Thr Arg Asp Ser
   305                 310                 315                 320
   gga atg gaa gct gta gct gaa ggc cat gct gat ttg gta tcc tat ggt      1008
   Gly Met Glu Ala Val Ala Glu Gly His Ala Asp Leu Val Ser Tyr Gly
                   325                 330                 335
   cgt ctt ttc atc tcc aat cca gac ttg gtt tta agg ctt aag ctc aat      1056
   Arg Leu Phe Ile Ser Asn Pro Asp Leu Val Leu Arg Leu Lys Leu Asn
               340                 345                 350
   gca cct ctt acc aag tat aac agg aac aca ttt tac acc caa gat cct      1104
   Ala Pro Leu Thr Lys Tyr Asn Arg Asn Thr Phe Tyr Thr Gln Asp Pro
           355                 360                 365
   gtt ata ggc tac aca gat tat cct ttc ttt aat gga aca act gag aca      1152
   Val Ile Gly Tyr Thr Asp Tyr Pro Phe Phe Asn Gly Thr Thr Glu Thr
       370                 375                 380
   aaa tta agt aac tag ctaaggccat gcatgccctt taattttaat ctccatatgg      1207
   Lys Leu Ser Asn
   385
   ctttttgaat aataatgttc ataacattca aaactcttca gttgagttta tcctcagaca      1267
   aacaaattaa gtggttcatt cacttgttag ggtatttaga tcttaggtta attagtctcc      1327
   ggcattttga tttcatttca atttgtattc agtctttcat tttgaataaa ataatattaa      1387
   gttttttgcc ttaaaaaaaa aaaaaaa                                         1414
<210> 30
   <211> 388
   <212> PRT
   <213> Glycine max
<400> 30
   Met Ala Asp Asn Ser Ile Ser Leu Phe Ser Pro Tyr Asn Lys Met Gly
   1               5                   10                  15
   Lys Phe Asn Leu Ser His Arg Val Val Leu Ala Pro Met Thr Arg Cys
   20 25 30
   Arg Ala Leu Asn Gly Thr Pro Leu Ala Ala His Ala Glu Tyr Tyr Ala
   35 40 45
   Gln Arg Ser Thr Pro Gly Gly Phe Leu Ile Thr Glu Gly Thr Leu Ile
   50 55 60
   Ser Pro Thr Ser Ser Gly Phe Pro His Val Pro Gly Ile Tyr Ser Asp
   65 70 75 80
   Glu Gln Val Glu Ala Trp Arg Asn Val Val Asp Ala Val His Ala Asn
   85 90 95
   Gly Ser Phe Ile Phe Cys Gln Leu Trp His Val Gly Arg Ala Ser His
   100 105 110
   Pro Val Tyr Gln Pro Gly Gly Ala Leu Pro Ser Ser Ser Thr Ser Lys
   115 120 125
   Pro Ile Ser Asp Lys Trp Lys Ile Leu Met Pro Asp Gly Ser His Gly
   130 135 140
   Ile Tyr Pro Glu Pro Arg Ala Leu Thr Thr Ser Glu Ile Ser Glu Ile
   145 150 155 160
   Val His His Tyr Arg Gln Ala Ala Ile Asn Ala Ile Arg Ala Gly Phe
   165 170 175
   Asp Gly Ile Glu Ile His Gly Ala His Gly Tyr Leu Ile Asp Gln Phe
   180 185 190
   Leu Lys Asp Ala Ile Asn Asp Arg Thr Asp Glu Tyr Gly Gly Pro Leu
   195 200 205
   Glu Asn Arg Cys Arg Phe Leu Met Glu Val Val Glu Ala Val Val Ser
   210 215 220
   Ala Ile Gly Ala Glu Arg Val Ala Ile Arg Ile Ser Pro Ala Ile Asp
   225 230 235 240
   Phe Asn Asp Ala Phe Asp Ser Asp Pro Leu Gly Leu Gly Leu Ala Val
   245 250 255
   Ile Glu Arg Leu Asn Asn Leu Gln Lys Gln Val Gly Thr Lys Leu Ala
   260 265 270
   Tyr Leu His Val Thr Gln Pro Arg Phe Thr Leu Leu Ala Gln Thr Glu
   275 280 285
   Ser Val Ser Glu Lys Glu Glu Ala His Phe Met Gln Lys Trp Arg Glu
   290 295 300
   Ala Tyr Glu Gly Thr Phe Met Cys Ser Gly Ala Phe Thr Arg Asp Ser
   305                 310                 315                 320
   Gly Met Glu Ala Val Ala Glu Gly His Ala Asp Leu Val Ser Tyr Gly
                   325                 330                 335
   Arg Leu Phe Ile Ser Asn Pro Asp Leu Val Leu Arg Leu Lys Leu Asn
               340                 345                 350
   Ala Pro Leu Thr Lys Tyr Asn Arg Asn Thr Phe Tyr Thr Gln Asp Pro
           355                 360                 365
   Val Ile Gly Tyr Thr Asp Tyr Pro Phe Phe Asn Gly Thr Thr Glu Thr
       370                 375                 380
   Lys Leu Ser Asn
   385